(19) Europäisches Patentamt
European Patent Office
Office européen des brevets

(11) Veröffentlichungsnummer: **0 374 098**
**A2**

(12) **EUROPÄISCHE PATENTANMELDUNG**

(21) Anmeldenummer: **89810923.6**

(22) Anmeldetag: **06.12.89**

(51) Int. Cl.5: **C07K 5/02, C07K 5/06, C07K 7/02, A61K 37/64**

(30) Priorität: **15.12.88 CH 4640/88**

(43) Veröffentlichungstag der Anmeldung:
**20.06.90 Patentblatt 90/25**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Fässler, Alexander, Dr.**
**Hallenstrasse 10**
**CH-4104 Oberwil(CH)**
Erfinder: **Hungerbühler, Ernst, Dr.**
**Zeisigweg 4**
**CH-4310 Rheinfelden(CH)**
Erfinder: **Rüeger, Heinrich, Dr.**
**Alemannenweg 6**
**CH-4112 Flüh(CH)**
Erfinder: **Schneider, Peter, Dr.**
**Bäumliackerstrasse 8**
**CH-4103 Bottmingen(CH)**

(54) **Retrovirale Proteasehemmer.**

(57) Die Erfindung betrifft Verbindungen der Formel

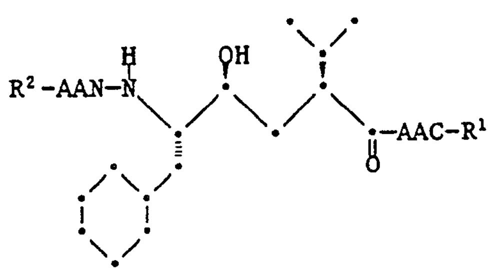

(I),

worin AAN ein bivalentes Radikal bestehend aus einem bis fünf bivalenten α-Aminosäureresten bedeutet, welches N-terminal mit dem Rest $R^2$ und C-terminal mit der Gruppe NH- verbunden ist, AAC ein bivalentes Radikal bestehend aus einem oder zwei bivalenten α-Aminosäureresten bedeutet, welches N-terminal mit der Gruppe -C=O und C-terminal mit dem Rest $R^1$ verbunden ist, $R^1$ für Hydroxy, verethertes Hydroxy, Amino oder substituiertes Amino mit Ausnahme eines von einer α-Aminosäure abgeleiteten Aminorestes bedeutet, und $R^2$ Wasserstoff oder einen Acylrest mit Ausnahme eines gegebenenfalls N-substituierten Acylrestes einer natürlichen Aminosäure bedeutet, ferner Salze von solchen Verbindungen mit salzbildenden Gruppen. Die Verbindungen sind geeignet als gag-Protease-Inhibitoren.

EP 0 374 098 A2

## Retrovirale Proteasehemmer

HIV (human immunodeficiency virus) ist ein Retrovirus, der die im Menschen im allgemeinen tödlich verlaufende Krankheit AIDS verursacht. Während es heute möglich ist, die Auswirkungen von AIDS zu lindern und das Leben der Patienten zu verlängern, fehlen bisher pharmazeutische Präparate, die die Ursache von AIDS wirksam bekämpfen. Eine mögliche Zielrichtung bei der Therapie von AIDS besteht darin, eine Vermehrung von HIV zu verhindern, ohne gleichzeitig die noch intakten Zellverbände des Patienten zu schädigen.

Das Genom der bislang bekannten beiden Arten von HIV, HIV-1 und HIV-2, weist jeweils einen Bereich auf, der eine "gag-Protease" kodiert. Des weiteren enthalten beide Viren einen Bereich, der für die Gruppen-spezifischen Antigene, englisch "group specific antigen" (gag), kodiert. Die entsprechenden Gene werden als Vorläuferprotein exprimiert und daraus proteolytisch von der oben erwähnten gag-Protease die eigentlichen gag-Proteine freigesetzt. Auch die gag-Protease selbst wird aus einem Vorläuferprotein herausgeschnitten, aus dem noch weitere virale Proteine, wie die Reverse Transcriptase und die Integrase, entstehen. Dieser Prozess verläuft vermutlich autoproteolytisch. Es ist ferner bekannt, dass die gag-Protease das "major core protein" (p24) von HIV-1 bzw. HIV-2 vorzugsweise N-terminal bei Prolin spaltet, z.B. bei Phe-Pro, Leu-Pro oder Tyr-Pro. Ein auf diese Weise verkürztes, Pro-terminales P24 dient dann zusammen mit weiteren viralen Strukturproteinen zum Aufbau des Nucleocapsids and der Virushülle von HIV-1 und HIV-2. Des weiteren ist bekannt, dass die HIV-1 gag-Protease Leu-Phe-, Leu-Ala-, Met-Met- und Phe-Leu-Sequenzen mit unterschiedlicher Effizienz spaltet. Es gilt als sicher, dass zusätzlich zur Aminosäuresequenz die Konformation zur Spezifität der gag-Protease beiträgt.

Wenn die Wirkung der gag-Protease unterbunden werden könnte, stünden dem Virus weder funktionelle Strukturproteine noch die notwendigen viralen Enzyme zur Verfügung, und dessen Vermehrung wäre behindert, wenn nicht gar unterbrochen. Es besteht daher ein Bedürfnis für Inhibitoren oder zumindest Hemmer der gag-Protease. Derartigen Verbindungen kommt eine wichtige Rolle als antivirale Mittel gegen AIDS oder andere retrovirale Erkrankungen zu.

Es wurde nun überraschenderweise gefunden, dass die erfindungsgemässen Verbindungen als gag-Protease-Inhibitoren geeignet sind, da sie imstande sind, gag-Proteasen bereits im nanomolaren Konzentrationsbereich zu hemmen.

Bei den erfindungsgemässen Verbindungen handelt es sich um die Verbindungen der Formel

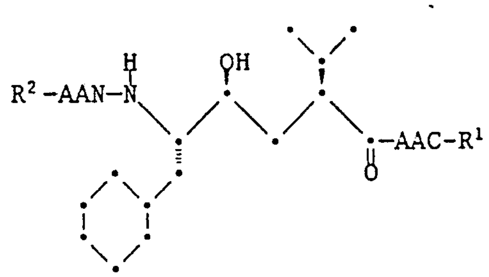

(I),

worin AAN ein bivalentes Radikal bestehend aus einem bis fünf bivalenten α-Aminosäureresten bedeutet, welches N-terminal mit dem Rest $R^2$ und C-terminal mit der Gruppe NH- verbunden ist, AAC ein bivalentes Radikal bestehend aus einem oder zwei bivalenten α-Aminosäureresten bedeutet, welches N-terminal mit der Gruppe -C=O und C-terminal mit dem Rest $R^1$ verbunden ist, $R^1$ für Hydroxy, verethertes Hydroxy, Amino oder substituiertes Amino mit Ausnahme eines von einer α-Aminosäure abgeleiteten Aminorestes steht, und $R^2$ Wasserstoff oder einen Acylrest mit Ausnahme eines gegebenenfalls N-substituierten Acylrestes einer natürlichen Aminosäure bedeutet. Ausser den vorstehend definierten Verbindungen der Formel I betrifft die Erfindung ferner Salze von solchen Verbindungen mit salzbildenden Gruppen, Verfahren zu ihrer Herstellung, pharmazeutische Präparate mit diesen Verbindungen und die Verwendung dieser Verbindungen als Arzneimittel oder zur Herstellung von pharmazeutischen Präparaten, sowie Zwischenprodukte zur Herstellung von Verbindungen der Formel I.

Die Abkürzung AAN steht für den Aminosäureteil, der mit dem N-Terminus des zentralen (2S,4S,5S)-5-Amino-6-cyclohexyl-4-hydroxy-2-isopropylhexanoyl-Restes verknüpft ist (<u>a</u>mino <u>a</u>cid(s) <u>N</u>-terminal). Die Abkürzung AAC steht für den Aminosäureteil, der mit dem C-Terminus des gleichen zentralen Restes verknüpft ist (<u>a</u>mino <u>a</u>cid(s) <u>C</u>-terminal).

In der Beschreibung der vorliegenden Erfindung bedeutet der bei der Definition von Gruppen oder Resten, z.B. Niederalkyl, Niederalkoxy, Niederalkanoyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Die substituierten α-C-Atome der Aminosäurereste, aus denen AAN und AAC bestehen, können die R-, S- oder R,S-Konfiguration haben. Bevorzugt sind Verbindungen der Formel I, worin diese C-Atome die S-Konfiguration aufweisen. Die α-Aminosäurereste des Radikals AAN können N-terminal zur Erhöhung der Stabilität der Verbindung der Formel I gegen enzymatischen Abbau durch Niederalkyl, z.B. Methyl oder Ethyl, substituiert sein.

Ein bivalenter α-Aminosäurerest, aus denen AAN und AAC bestehen, ist beispielsweise ein Rest einer natürlichen α-Aminosäure mit der L-Konfiguration, wie sie normalerweise in Proteinen vorkommen, eines Homologen einer solchen Aminosäure, z.B. worin die Aminosäureseitenkette um eine oder zwei Methylengruppen verlängert oder verkürzt ist und/oder eine Methylgruppe durch Wasserstoff ersetzt ist, einer substituierten aromatischen α-Aminosäure, z.B. eines substituierten Phenylalanins oder Phenylglycins, worin der Substituent Niederalkyl, z.B. Methyl, Halogen, z.B. Fluor, Chlor, Brom oder Iod, Hydroxy, Niederalkoxy, z.B. Methoxy, Niederalkanoyloxy, z.B. Acetoxy, Amino, Niederalkylamino, z.B. Methylamino, Diniederalkylamino, z.B. Dimethylamino, Niederalkanoylamino, z.B. Acetylamino oder Pivaloylamino, Niederalkoxycarbonylamino, z.B. tert-Butoxycarbonylamino, Arylmethoxycarbonylamino, z.B. Benzyloxycarbonylamino, und/oder Nitro sein kann und ein- oder mehrfach vorkommt, eines benzanellierten Phenylalanins oder Phenylglycins, wie α-Naphthylalanins, oder eines hydrierten Phenylalanins oder Phenylglycins, wie Cyclohexylalanins oder Cyclohexylglycins, einer fünf- oder sechs-gliedrigen cyclischen, benzanellierten α-Aminosäure, z.B. Indolin-2-carbonsäure oder 1,2,3,4-Tetrahydroisochinolin-3-carbonsäure, einer natürlichen oder homologen α-Aminosäure, in der eine Carboxygruppe der Seitenkette in veresterter oder amidierter Form vorliegt, z.B. als Niederalkylestergruppe, wie Methoxycarbonyl oder tert-Butoxycarbonyl, oder als Carbamoyl-, als Niederalkylcarbamoyl-, wie Methylcarbamoyl, oder als Diniederalkylcarbamoylgruppe, wie Dimethylcarbamoyl, in der eine Aminogruppe der Seitenkette in acylierter Form vorliegt, z.B. als Niederalkanoylamino-, wie Acetylamino oder Pivaloylamino, als Niederalkoxycarbonylamino-, wie tert-Butoxycarbonylamino, oder als Arylmethoxycarbonylaminogruppe, wie Benzyloxycarbonylamino, oder in der eine Hydroxygruppe der Seitenkette in veretherter oder veresterter Form vorliegt, z.B. als Niederalkoxygruppe, wie Methoxy, als Arylniederalkoxygruppe, wie Benzyloxy, oder als Niederalkanoyloxygruppe, wie Acetoxy, oder eines Epimeren einer solchen Aminosäure, d.h. mit der unnatürlichen D-Konfiguration.

Solche Aminosäuren sind beispielsweise Glycin (H-Gly-OH), Alanin (H-Ala-OH), Valin (H-Val-OH), Norvalin (α-Aminovaleriansäure), Leucin, (H-Leu-OH), Isoleucin (H-Ile-OH), Norleucin (α-Aminohexansäure, H-Nle-OH), Serin (H-Ser-OH), Homoserin (α-Amino-γ-hydroxybuttersäure), Threonin (H-Thr-OH), Methionin (H-Met-OH), Cystein (H-Cys-OH), Prolin (H-Pro-OH), trans-3- und trans-4-Hydroxyprolin, Phenylalanin (H-Phe-OH), Tyrosin (H-Tyr-OH), 4-Nitrophenylalanin, 4-Aminophenylalanin, 4-Chlorphenylalanin, β-Phenylserin (β-Hydroxyphenylalanin), Phenylglycin, α-Naphthylalanin (H-Nal-OH), Cyclohexylalanin (H-Cha-OH), Cyclohexylglycin, Tryptophan (H-Trp-OH), Indolin-2-carbonsäure, 1,2,3,4-Tetrahydroisochinolin-3-carbonsäure, Asparaginsäure (H-Asp-OH), Asparagin (H-Asn-OH), Aminomalonsäure, Aminomalonsäure-monoamid, Glutaminsäure (H-Glu-OH), Glutaminsäure-mono-tert-butylester, Glutamin (H-Gln-OH), $N^{\delta}$-Dimethylglutamin, Histidin (H-His-OH), Arginin (H-Arg-OH), Lysin (H-Lys-OH), $N^{\varepsilon}$-tert-Butoxycarbonyl-lysin, δ-Hydroxylysin, Ornithin (α,δ-Diaminovaleriansäure, $N^{\delta}$-Pivaloyl-ornithin, α,γ-Diaminobuttersäure oder α,β-Diaminopropionsäure.

Die in der Beschreibung der vorliegenden Erfindung verwendeten allgemeinen Ausdrücke und Bezeichnungen haben vorzugsweise die folgenden Bedeutungen:

Eine veretherte Hydroxygruppe $R^1$ ist vorzugsweise durch solche organische Reste verethert, die unter physiologischen Bedingungen abspaltbar sind und nach der Abspaltung in der betreffenden Konzentration pharmakologisch unbedenkliche Spaltprodukte liefern.

Verethertes Hydroxy $R^1$ ist z.B. Acyloxyniederalkoxy, worin Acyl die Acylgruppe einer gegebenenfalls verzweigten Niederalkancarbonsäure oder der durch gegebenenfalls verzweigtes Niederalkyl monoveresterten Kohlensäure darstellt, z.B. Niederalkanoyloxyniederalkoxy, wie Acetoxymethoxy, 1-Acetoxyethoxy, Pivaloyloxymethoxy oder 1-Pivaloyloxyethoxy, oder Niederalkoxycarbonyloxyniederalkoxy, wie Ethoxycarbonyloxymethoxy, 1-Ethoxycarbonyloxyethoxy, tert-Butoxycarbonyloxymethoxy oder 1-tert-Butoxycarbonyloxyethoxy.

Verethertes Hydroxy $R^1$ ist ferner vorzugsweise Niederalkoxy, z.B. Methoxy oder Ethoxy, Aryloxy, z.B. Phenoxy, oder Arylniederalkoxy, z.B. Benzyloxy.

Substituiertes Amino $R^1$ ist beispielsweise eine Aminogruppe, welche durch einen oder gegebenenfalls zwei organische Reste, z.B. unsubstituierte oder substituierte, gesättigte oder ungesättigte, aliphatische

Kohlenwasserstoffreste mit bis einschliesslich 18, bevorzugt bis einschliesslich 10, C-Atomen oder unsubstituierte oder substituierte, aromatische, heteroaromatische, aromatisch-aliphatische oder heteroaromatischaliphatische Reste mit bis zu 18, bevorzugt bis einschliesslich 10, C-Atomen, substituiert ist.

Ausgeschlossen als substituiertes Amino $R^1$ sind der Rest einer $\alpha$-Aminosäure oder deren N-substituierte, veresterte oder amidierte Derivate.

Ein unsubstituierter oder substituierter, gesättigter oder ungesättigter, aliphatischer Kohlenwasserstoffrest, welcher die Aminogruppe $R^1$ substituiert, ist beispielsweise gegebenenfalls substituiertes Alkyl mit bis zu 10 C-Atomen, Niederalkenyl oder Niederalkinyl mit bis einschliesslich 7 C-Atomen, oder Cycloalkyl oder Cycloalkylniederalkyl mit 4-10 C-Atomen. Diese Reste können durch eine oder mehrere der im Zusammenhang mit nachstehend definiertem Niederalkyl $R^a$ genannten funktionellen Gruppen, sowie durch Sulfo, substituiert sein.

Als Substituenten sind Hydroxy, Niederalkoxy, z.B. Methoxy, Niederalkanoyloxy, z.B. Acetoxy, substituiertes oder unsubstituiertes Phenyloxy, z.B. Carbamoylphenyloxy oder Carbamoyl-hydroxy-phenyloxy, Carboxy, verestertes Carboxy, z.B. Niederalkoxycarbonyl, wie Methoxycarbonyl oder tert-Butoxycarbonyl, oder ein physiologisch spaltbares verestertes Carboxy, z.B. 1-(Niederalkanoyloxy)-niederalkoxycarbonyl, wie Acetoxymethoxycarbonyl, Pivaloyloxymethoxycarbonyl oder 1-Propionyloxyethoxycarbonyl, 1-(Niederalkoxycarbonyloxy)-niederalkoxycarbonyl, wie 1-(Ethoxycarbonyloxy)-ethoxycarbonyl, oder $\alpha$-Amino-niederalkanoyloxymethoxycarbonyl, wie $\alpha$-Aminoacetoxymethoxycarbonyl oder (S)-$\alpha$-Amino-$\beta$-methylbutyryloxymethoxycarbonyl, Carbamoyl, substituiertes oder unsubstituiertes Niederalkylcarbamoyl, z.B. Hydroxyniederalkylcarbamoyl, wie 2-Hydroxyethylcarbamoyl oder Tris-(hydroxymethyl)-methylcarbamoyl, Amino, Niederalkylamino, z.B. Methylamino, Diniederalkylamino, z.B. Dimethylamino, Niederalkoxycarbonylamino, z.B. tert-Butoxycarbonylamino, Guanidino, über ein Stickstoff-Atom gebundenes, gesättigtes fünf- oder sechsgliedriges, gewünschtenfalls durch Oxo substituiertes Heterocyclyl, z.B. 1-Piperidinyl, 4-Morpholinyl oder 2-Oxo-1-pyrrolidinyl, oder Sulfo bevorzugt.

Ein aromatischer oder aromatisch-aliphatischer Rest in einer Gruppe $R^1$ hat vorzugsweise die gleichen wie nachstehend unter Aryl $R^a$ oder $R^b$ oder Arylniederalkyl $R^a$ genannten Bedeutungen und ist beispielsweise Phenyl oder Phenylniederalkyl.

Diese Reste können im Aromaten z.B. durch Niederalkyl, z.B. Methyl oder Ethyl, Hydroxy, verethertes Hydroxy, z.B. Niederalkoxy, wie Methoxy oder tert-Butoxy, verestertes Hydroxy, z.B. Niederalkanoyloxy, wie Acetoxy, oder Halogen, z.B. Fluor oder Chlor, Carboxy, verestertes Carboxy, z.B. Niederalkoxycarbonyl, wie tert-Butoxycarbonyl, Carbamoyl, Amino, Niederalkylamino, z.B. Methylamino, Diniederalkylamino, z.B. Dimethylamino, acyliertes Amino, z.B. Niederalkoxycarbonylamino, wie tert-Butoxycarbonylamino, sowie durch Nitro substituiert sein.

Niederalkyl in einem Rest Phenylniederalkyl kann durch die gleichen Substituenten wie Alkyl in einem Rest $R^1$ substituiert sein.

Ein heteroaromatischer oder heteroaromatisch-aliphatischer Rest in einer Gruppe $R^1$ hat vorzugsweise die gleichen wie nachstehend unter Heteroaryl $R^a$ und $R^b$ oder Heteroarylniederalkyl $R^a$ genannten Bedeutungen und ist beispielsweise Pyridylniederalkyl, z.B. 2-, 3- oder 4-Pyridylmethyl, Imidazolylniederalkyl, z.B. 2-(4-Imidazolyl)-ethyl oder auch 2-(2-[4-Imidazolyl]-ethylamino)-ethyl, oder Indolylniederalkyl, z.B. 3-Indolylmethyl oder 2-(3-Indolyl)-ethyl.

Substituiertes Amino $R^1$ ist vorzugsweise Alkylamino, z.B. Methyl-, Ethyl-, n-Propyl-, Isopropyl-, n-Butyl-, Isobutyl-, tert-Butyl-, n-Pentyl, Isopentyl-, n-Hexyl-, n-Octyl- oder n-Decylamino, Diniederalkylamino, z.B. Dimethylamino oder Diethylamino, Hydroxyniederalkylamino, z.B. 2-Hydroxyethylamino, 1-Hydroxybut-2-ylamino, 5-Hydroxypentylamino oder Tris(hydroxymethyl)-methylamino, Di-(hydroxyniederalkyl)-amino, z.B. Di-(2-hydroxyethyl)-amino, Niederalkoxyniederalkylamino, z.B. 2-Methoxyethylamino, Niederalkanoylniederalkylamino, z.B. 2-Acetoxyethylamino, Phenoxyniederalkylamino oder Phenoxy-hydroxy-niederalkylamino, worin Phenoxy gegebenenfalls durch Niederalkyl, Niederalkoxy, Hydroxy, Carboxy, Niederalkoxycarbonyl oder Carbamoyl substituiert ist, z.B. 2-Phenoxyethylamino, 2-(3-Carbamoyl-4-hydroxyphenoxy)-ethylamino oder 3-(3-Carbamoylphenoxy)-2-hydroxy-propylamino, Carboxyalkylamino oder Amino-carboxy-alkylamino, worin der Carboxyrest nicht in 1-Stellung des Alkylrests steht, z.B. 4-Carboxy-n-butylamino, 5-Carboxy-n-pentylamino, 5-Amino-5-carboxy-n-pentylamino, 6-Carboxy-n-hexylamino, 7-Carboxy-n-heptylamino oder 8-Carboxy-n-octylamino, ferner Dicarboxymethylamino, Niederalkoxycarbonylalkylamino oder Acylamino-niederalkoxycarbonyl-alkylamino, worin der Carbonylrest nicht in 1-Stellung des Alkylrests steht, z.B. 4-tert-Butoxycarbonyl-n-butylamino, 5-tert-Butoxycarbonylamino-5-methoxycarbonyl-n-pentylamino, 7-tert-Butoxycarbonyl-n-heptylamino oder 8-tert-Butoxycarbonyl-n-octylamino, ferner Di-niederalkoxycarbonyl-methylamino, z.B. Di-methoxycarbonyl-methylamino, physiologisch spaltbares verestertes Carboxyalkylamino, worin die Esterfunktion nicht in 1-Stellung des Alkylrests steht, z.B. 4-Pivaloyloxymethoxycarbonyl-n-butylamino, 7-(1-Ethoxycarbonyloxyethoxycarbonyl)-n-heptylamino oder 7-Pivaloyloxymethoxycarbonyl-n-heptylamino, Carbamoylalkylamino oder Hydroxyniederalkylcarbamoylalkylamino, worin der Carbamoylrest nicht in 1-Stellung des Alkylrests steht, z.B. 4-Carbamoyl-n-butylamino, 7-Carbamoyl-n-heptylamino oder 4-(Tris[hydroxymethyl]-methyl)-carbamoyl-n-butylamino, ferner Dicarbamoyl-methylamino, Di-(niederalkylcarbamoyl)-methylamino, z.B. Di-(methylcarbamoyl)-methylamino, Di-(hydroxyniederalkylcarbamoyl)-methylamino, z.B. Di-(2-hydroxyethylcarbamoyl)-methylamino, oder Bis-(diniederalkylcarbamoyl)-methylamino, z.B. Bis-(dimethylcarbamoyl)-methylamino, Aminoniederalkylamino, z.B. 2-Aminoethylamino oder 4-Aminobutylamino, Niederalkylaminoniederalkylamino, z.B. 2-Methylaminoethylamino, Diniederalkylaminoniederalkylamino, z.B. 2-Dimethylaminoethylamino oder 3-Dimethylaminopropylamino, Niederalkoxycarbonylaminoniederalkylamino, z.B. 2-(tert-Butoxycarbonylamino)-ethylamino, Guanidinoniederalkylamino, z.B. 2-Guanidinoethylamino, über ein Stickstoff-Atom gebundenes, gesättigtes fünf- oder sechsgliedriges Heterocyclylniederalkylamino, z.B. 2-(4-Morpholinyl)-ethylamino, 3-(4-Morpholinyl)-propylamino oder 3-(2-Oxo-1-pyrrolidinyl)-propylamino, Niederalkenylamino, z.B. Allylamino oder 2- oder 3-Butenylamino, Niederalkinylamino, z.B. Propargylamino, Cycloalkylamino, z.B. Cyclohexylamino oder Decahydronaphthylamino, Cycloalkylniederalkylamino, z.B. Cyclopropylmethylamino oder Cyclohexylmethylamino, Naphthylamino, Phenylamino oder Phenylniederalkylamino, worin Phenyl oder Naphthyl gegebenenfalls durch Niederalkyl, z.B. Methyl, Phenyl, Hydroxy, Niederalkoxy, z.B. Methoxy oder tert-Butoxy, Niederalkanoyloxy, z.B. Acetoxy, Halogen, z.B. Fluor oder Chlor, Carboxy, Niederalkoxycarbonyl, z.B. tert-Butoxycarbonyl, Carbamoyl, Amino, Niederalkylamino, z.B. Methylamino, Diniederalkylamino, z.B. Dimethylamino, Acylamino, z.B. tert-Butoxycarbonylamino und/oder durch Nitro mono- oder mehrfach substituiert ist, z.B. Phenylamino, 2-, 3- oder 4-Methylphenylamino, 4-Biphenylylamino, 4-Hydroxyphenylamino, 4-Methoxyphenylamino, 2,3-, 2,4- oder 2,5-Dimethoxyphenylamino, 4-Chlorphenylamino, 2-, 3- oder 4-Carboxyphenylamino, 2-, 3- oder 4-Methoxy- oder tert-Butoxy-carbonylphenylamino, 2-, 3- oder 4-Carbamoylphenylamino, 4-Aminophenylamino, 4-tert-Butoxycarbonylaminophenylamino oder 4-Nitrophenylamino, ferner z.B. Benzylamino, 4-Methylbenzylamino, 4-Biphenylylmethylamino, 4-Methoxybenzylamino, 2-, 3- oder 4-Carboxybenzylamino, 2-, 3- oder 4-tert-Butoxycarbonylbenzylamino, 2-, 3- oder 4-Carbamoylbenzylamino, 2-Phenylethylamino, 3-Phenylpropylamino oder 5-Phenylpentylamino, Pyridylniederalkylamino, z.B. 2-, 3- oder 4-Pyridylmethylamino, 2-(2-, 3- oder 4-Pyridyl)-ethylamino oder 3-(2-, 3- oder 4-Pyridyl)-propylamino, Imidazolylniederalkylamino, z.B. 4-Imidazolylmethylamino, 2-(4-Imidazolyl)-ethylamino oder 2-(2-[4-Imidazolyl]-ethylamino)-ethylamino, Indolylniederalkylamino, z.B. 3-Indolylmethylamino oder 2-(3-Indolyl)-ethylamino, oder Sulfoniederalkylamino, z.B. 2-Sulfoethylamino.

Acyl $R^2$ hat z.B. bis zu 25, bevorzugt bis zu 19 C-Atome und ist in erster Linie die Acylgruppe einer Carbonsäure, eines Halbesters der Kohlensäure, einer gegebenenfalls N-substituierten Carbamin- oder Thiocarbaminsäure, eines gegebenenfalls N-substituierten Oxalamids, einer Phosphorsäure, einer Sulfonsäure oder einer gegebenenfalls N-substituierten Amidosulfonsäure.

Bevorzugte Acylgruppen $R^2$ sind beispielsweise Alkanoyl, z.B. n-Decanoyl oder Niederalkanoyl, z.B. Formyl, Acetyl, Propionyl oder Pivaloyl, Hydroxyniederalkanoyl, z.B. β-Hydroxypropionyl, Niederalkoxyniederalkanoyl, z.B. Niederalkoxyacetyl oder Niederalkoxypropionyl, wie Methoxyacetyl oder β-Methoxypropionyl, Phenoxyniederalkanoyl, z.B. Phenoxyacetyl, Naphthoxyniederalkanoyl, z.B. α- oder β-Naphthoxyacetyl, Niederalkanoyloxyniederalkanoyl, z.B. Niederalkanoyloxyacetyl oder Niederalkanoyloxypropionyl, wie Acetoxyacetyl oder β-Acetoxypropionyl, Halogenniederalkanoyl, z.B. α-Halogenacetyl, wie α-Chlor-, α-Brom-, α-Iod- oder α,α,α-Trichloracetyl, oder Halogenpropionyl, wie β-Chlor-oder β-Brompropionyl, Carboxyniederalkanoyl, z.B. Carboxyacetyl oder β-Carboxypropionyl, Niederalkoxycarbonylniederalkanoyl, z.B. Niederalkoxycarbonylacetyl oder Niederalkoxycarbonylpropionyl, wie Methoxy carbonylacetyl, β-Methoxycarbonylpropionyl, Ethoxycarbonylacetyl oder β-Ethoxycarbonylpropionyl, Carbamoylniederalkanoyl, z.B. Carbamoylacetyl oder β-Carbamoylpropionyl, Niederalkylcarbamoylniederalkanoyl, z.B. Methylcarbamoylacetyl, Diniederalkylcarbamoylniederalkanoyl, z.B. Dimethylcarbamoylacetyl, Oxoniederalkanoyl, z.B. Acetoacetyl oder Propionylacetyl, Hydroxy-carboxy-niederalkanoyl, z.B. α-Hydroxy-α-carboxy-acetyl oder α-Hydroxy-β-carboxypropionyl, Hydroxy-niederalkoxycarbonyl-niederalkanoyl, z.B. α-Hydroxy-α-ethoxy- oder -methoxycarbonyl-acetyl oder α-Hydroxy-β-ethoxy- oder -methoxycarbonyl-propionyl, verestertes Hydroxyniederalkoxycarbonyl-niederalkanoyl, z.B. α-Acetoxy-α-methoxycarbonyl-acetyl, Dihydroxy-carboxy-niederalkanoyl, z.B. α,β-Dihydroxy-β-carboxy-propionyl, Dihydroxy-niederalkoxycarbonyl-niederalkanoyl, z.B. α,β-Dihydroxy-β-ethoxy- oder -methoxycarbonyl-propionyl, verestertes Dihydroxy-niederalkoxycarbonyl-niederalkanoyl, z.B. α,β-Diacetoxy-β-methoxycarbonyl-propionyl, α-Naphthoxy-carboxy-niederalkanoyl, z.B. 2-α-Naphthoxy-4-carboxy-butyryl, α-Naphthoxy-niederalkoxycarbonyl-niederalkanoyl, z.B. α-Naphthoxy-ethoxycarbonyl-acetyl, 2-α-Naphthoxy-3-ethoxycarbonyl-propionyl oder 2-α-Naphthoxy-4-tert-butoxycarbonyl-butyryl, α-Naphthoxy-benzyloxycarbonyl-niederalkanoyl, z.B. 2-α-Naphthoxy-3-benzyloxycarbonyl-propionyl, α-Naphthoxy-carbamoyl-niederalkanoyl, z.B. 2-α-Naphthoxy-4-carbamoyl-butyryl, α-Naphthoxy-cyano-niederalkanoyl, z.B. α-Naphthoxy-cyano-acetyl oder 2-α-Naphthoxy-4-cyano-buty-

ryl, α-Naphthoxy-diniederalkylamino-niederalkanoyl, z.B. 2-α-Naphthoxy-5-dimethylamino-pentanoyl, α-Naphthoxy-oxo-niederalkanoyl, z.B. 2-α-Naphthoxy-4-oxo-pentanoyl, Heterocyclylniederalkanoyl, z.B. Indolylacetyl oder Benzofuranylacetyl, Niederalkenoyl, z.B. Acryloyl, Vinylacetyl, Crotonoyl oder 3- oder 4-Pentenoyl, Niederalkinoyl, z.B. Propioloyl oder 2- oder 3-Butinoyl, Cycloalkylcarbonyl, z.B. Cyclopropyl-, Cyclobutyl-, Cyclopentyl- oder Cyclohexylcarbonyl, Bicycloalkylcarbonyl, z.B. Decahydronaphthyl-2-carbonyl, endo- oder exo-Norbornyl-2-carbonyl, Bicyclo[2.2.2]oct-2-ylcarbonyl oder Bicyclo[3.3.1]non-9-ylcarbonyl, Tricycloalkylcarbonyl, z.B. 1- oder 2-Adamantylcarbonyl, Cycloalkenylcarbonyl, z.B. 1-Cyclohexenylcarbonyl oder 1,4-Cyclohexadienylcarbonyl, Bicycloalkenylcarbonyl, z.B. 5-Norbornen-2-ylcarbonyl oder Bicyclo[2.2.2]octen-2-ylcarbonyl, Cycloalkylniederalkanoyl, z.B. Cyclopropylacetyl, Cyclopentylacetyl, Cyclohexylacetyl oder 3-Cyclohexylpropionyl, Cycloalkylniederalkenoyl, z.B. Cyclohexylacryloyl, Cycloalkenylniederalkanoyl, z.B. 1-Cyclohexenylacetyl oder 1,4-Cyclohexadienylacetyl, Benzoyl, unsubstituiert, mono-oder mehrfachsubstituiert durch Niederalkyl, z.B. Methyl, Phenyl, Halogen, z.B. Chlor, Hydroxy, Niederalkoxy, z.B. Methoxy, und/oder Nitro, z.B. 4-Chlor-, 4-Methoxy- oder 4-Nitrobenzoyl, ferner Phenyl-, α-Naphthyl-oder β-Naphthyl-niederalkanoyl, worin Phenyl unsubstituiert, mono- oder mehrfachsubstituiert durch Niederalkyl, z.B. Methyl, Phenyl, Halogen, z.B. Chlor, Hydroxy, Niederalkoxy, z.B. Methoxy, und/oder Nitro und Niederalkanoyl .unsubstituiert oder substituiert z.B. durch Hydroxy, Niederalkoxy, Acyloxy, Carboxy, verestertes Carboxy, Carbamoyl, substituiertes Carbamoyl, Cyano, Phosphono, verestertes Phosphono, Benzofuranyl und/oder Oxo sein kann und gegebenenfalls verzweigt ist, z.B. Phenylacetyl, α-Naphthylacetyl, β-Naphthylacetyl, Niederalkylphenylacetyl, wie 4-Methylphenylacetyl, Niederalkoxyphenylacetyl, wie 4-Methoxyphenylacetyl, 3-Phenylpropionyl, 3-(p-Hydroxyphenyl)-propionyl, Diphenylacetyl, Di-(4-methoxyphenyl)-acetyl, Triphenylacetyl, substituiertes Anilinophenylacetyl, wie 2-(o,o-Dichloranilino)-phenylacetyl oder 2-(o,o-Dichloro-N-benzylanilino)-phenylacetyl, 3-α- oder β-Naphthylpropionyl, 3-Phenyl- oder 3-α-Naphthyl-2-hydroxy-propionyl, 3-Phenyl-oder 3-α-Naphthyl-2-niederalkoxy-propionyl, wie 3-Phenyl- oder 3-α-Naphthyl-2-neopentyloxy-propionyl, 3-Phenyl- oder 3-α-Naphthyl-2-acyloxy-propionyl, wie 3-Phenyl-2-pivaloyloxy- oder 2-acetoxy-propionyl, 3-α-Naphthyl-2-pivaloyloxy- oder 2-acetoxy-propionyl, 3-α-Naphthyl-2-acetoacetoxy-propionyl, 3-α-Naphthyl-2-ethylaminocarbonyloxy-propionyl oder 3-α-Naphthyl-2-(2-amino-oder 2-benzyloxycarbonylamino-2-methylpropionyloxy)-propionyl, 3-Phenyl- oder 3-α-Naphthyl-2-carboxymethylpropionyl, 3-Phenyl- oder 3-α-Naphthyl-2-niederalkoxycarbonyl-propionyl, wie 3-α-Naphthyl-2-ethoxycarbonyl-propionyl, 3-Phenyl- oder 3-α-Naphthyl-2-benzyloxycarbonylmethyl-propionyl, 3-Phenyl-oder 3-α-Naphthyl-2-carbamoyl-propionyl, 3-Phenyl- oder 3-α-Naphthyl-2-tert-butylcarbamoyl-propionyl, 3-Phenyl- oder 3-α-Naphthyl-2-(2-dimethylaminoethyl)-carbamoyl-propionyl, 3-α-Naphthyl-2-(carboxy- oder tert-butoxycarbonyl)-methylcarbamoyl-propionyl, 3-Phenyl- oder 3-α-Naphthyl-2-(3-hydroxy-2-propyl)-carbamoyl-propionyl, 3-Phenyl- oder 3-α-Naphthyl-2-(2,2-dimethoxyethyl)-carbamoyl-propionyl, 3-Phenyl-oder 3-α-Naphthyl-2-(5- amino-5-carboxypentyl)-carbamoyl-propionyl, 3-Phenyl- oder 3-α-Naphthyl-2-cyano-propionyl, 3-Phenyl- oder 3-α-Naphthyl-2-cyanomethyl-propionyl, 3-Phenyl-2-phosphono- oder phosphonomethyl-propionyl, 3-Phenyl-2-dimethoxyphosphoryl- oder dimethoxyphosphorylmethyl-propionyl, 3-Phenyl-2-diethoxyphosphoryl- oder diethoxyphosphorylmethyl-propionyl, 3-Phenyl-2-ethoxy- oder methoxy-hydroxyphosphoryl-propionyl, 3-Phenyl- oder 3-α-Naphthyl-2-acetonyl-propionyl, 3-Phenyl- oder 3-α-Naphthyl-2-dimethylaminomethyl-propionyl, 2-Benzyl- oder 2-α-Naphthylmethyl-4-cyano-butyryl, 4-Phenyl- oder 4-α-Naphthyl-3-carboxy-butyryl, 4-Phenyl- oder 4-α-Naphthyl-3-benzyloxycarbonyl-butyryl, 2-Benzyl-4-(2-benzofuranyl)-4-oxo-butyryl, 2-Benzyl- oder 2-α-Naphthylmethyl-4-oxo-pentanoyl, 2-Benzyl-oder 2-α-Naphthylmethyl-4,4-dimethyl-3-oxo-pentanoyl, 2-Benzyl- oder 2-α-Naphthylmethyl-5-dimethylamino-pentanoyl, 2-Benzyl- oder 2-α-Naphthylmethyl-5-dimethylamino-4-oxo-pentanoyl, 2-Benzyl- oder 2-α-Naphthylmethyl-5,5-dimethyl-4-oxo-hexanoyl, α,p-Diamino-phenylacetyl, α,p-Diacylamino-phenylacetyl, wie α,p-Dibenzyloxycarbonylamino-phenylacetyl oder α-Pivaloylamino-p-benzyloxycarbonylamino-phenylacetyl, Phenylniederalkenoyl, z.B. β-Phenylacryloyl oder β-Phenylvinylacetyl, Naphthylcarbonyl, z.B. α- oder β-Naphthylcarbonyl oder 1,8-Naphthalindicarbonyl, Indenylcarbonyl, z.B. 1-, 2- oder 3-Indenylcarbonyl, Indanylcarbonyl, z.B. 1- oder 2-Indanylcarbonyl, Phenanthrenylcarbonyl, z.B. 9-Phenanthrenylcarbonyl, gegebenenfalls substituiertes Pyrrolylcarbonyl, z.B. 2- oder 3-Pyrrolylcarbonyl oder 4- oder 5-Phenylpyrrolyl-2-carbonyl, Furylcarbonyl, z.B. 2-Furylcarbonyl, Thienylcarbonyl, z.B. 2-Thienylcarbonyl, Pyridylcarbonyl, z.B. 2-, 3- oder 4-Pyridylcarbonyl, gegebenenfalls substituiertes Indolylcarbonyl, z.B. 2-, 3- oder 5-Indolylcarbonyl, 1-Methyl-, 5-Methyl-, 5-Methoxy-, 5-Benzyloxy-, 5-Chlor- oder 4,5-Dimethylindolyl-2-carbonyl, 1-Benzylindolyl-2- oder 3-carbonyl, 4,5,6,7-Tetrahydroindolyl-2-carbonyl, Cyclohepta[b]pyrrolyl-5-carbonyl, gegebenenfalls substituiertes Chinolylcarbonyl, z.B. 2-, 3- oder 4-Chinolylcarbonyl oder 4-Hydroxychinolyl-2-carbonyl, gegebenenfalls substituiertes Isochinolylcarbonyl, z.B. 1-, 3- oder 4-Isochinolylcarbonyl oder 1-Oxo-1,2-dihydroisochinolyl-3-carbonyl, 2-Chinoxalinylcarbonyl, 2-Benzofuranylcarbonyl, 3-Chromancarbonyl, 3-Thiochromancarbonyl, Benz[e]indolyl-2-carbonyl, β-Carbolinyl-3-carbonyl, Pyrrolidinyl-3-carbonyl, Hydroxypyrrolidinylcarbonyl, z.B. 3- oder 4-Hydroxy pyrrolidinyl-2-carbonyl, Oxopyrrolidinylcarbonyl, z.B. 5-Oxopyrrolidinyl-2-carbonyl, Piperidinylcarbonyl, z.B. 2-, 3- oder 4-Piperidinylcarbonyl, Indolinylcarbonyl, z.B. 2- oder 3-Indolinylcarbonyl, 1,2,3,4-Tetrahydrochinolylcarbonyl, z.B. 1,2,3,4-Tetrahydrochinolyl-2-, -3- oder -4-carbonyl, 1,2,3,4-Tetrahydroisochinolylcarbonyl, z.B. 1,2,3,4-Tetrahydroisochinolyl-1-, -3- oder 4-carbonyl oder 1-Oxo-1,2,3,4-tetrahydroisochinolyl-3-carbonyl, Niederalkoxycarbonyl, z.B. Methoxy-, Ethoxy- oder tert-Niederalkoxycarbonyl, wie tert-Butoxycarbonyl, 2-Halogenniederalkoxycarbonyl, z.B. 2-Chlor-, 2-Brom-, 2-Iod- oder 2,2,2-Trichlorethoxycarbonyl, Arylniederalkoxycarbonyl, z.B. Arylmethoxycarbonyl, worin Aryl Phenyl, Biphenylyl, 1- oder 2-Naphthyl, Fluorenyl oder durch Niederalkyl, z.B. Methyl oder tert-Butyl, Niederalkoxy, z.B. Methoxy, Ethoxy oder tert-Butoxy, Hydroxy, Halogen, z.B. Chlor oder Brom, und/oder Nitro mono- oder mehrfachsubstituiertes Phenyl ist, z.B. Phenylniederalkoxycarbonyl, wie Benzyloxycarbonyl, 4-Methoxybenzyloxycarbonyl, 4-Nitrobenzyloxycarbonyl, Diphenylniederalkoxycarbonyl, wie Diphenylmethoxycarbonyl, Di-(4-methoxyphenyl)-methoxycarbonyl oder Trityloxycarbonyl, Fluorenylniederalkoxycarbonyl, wie 9-Fluorenylmethoxycarbonyl, ferner Oxamoyl oder Niederalkyloxamoyl, z.B. Methyl-oder Ethyloxamoyl.

Acyl $R^2$ ist ferner in erster Linie eine durch eine Thio-, Sulfinyl- oder Sulfonylgruppe und gegebenenfalls durch weitere, Heteroatome enthaltende Gruppen substituierte Acylgruppe einer gesättigten oder ungesättigten, aliphatischen, cycloaliphatischen, cycloaliphatisch-aliphatischen, aromatischen, aromatisch-aliphatischen, heteroaromatischen oder heteroaromatisch-aliphatischen Carbonsäure mit Ausnahme der gegebenenfalls N-substituierten natürlichen Aminosäure Methionin.

Bevorzugte Substituenten $R^2$ sind Acylgruppen der Formel

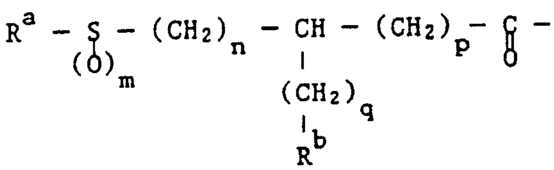

(Ia),

worin $R^a$ unsubstituiertes oder substituiertes Niederalkyl, Niederalkenyl, Niederalkinyl, Mono-, Bi- oder Tricycloalkyl, Cycloalkylniederalkyl, unsubstituiertes oder substituiertes Aryl, Arylniederalkyl, Arylniederalkenyl, unsubstituiertes oder substituiertes Heteroaryl, Heteroarylniederalkyl, unsubstituiertes oder substituiertes Hydroxy oder unsubstituiertes oder substituiertes Amino, $R^b$ Wasserstoff, Mono-, Bi-oder Tricycloalkyl, unsubstituiertes oder substituiertes Aryl oder unsubstituiertes oder substituiertes Heteroaryl, m 0, 1 oder 2, n 0, 1 oder 2, p 0, 1 oder 2 und q 0, 1, 2, 3 oder 4 darstellt.

Das Methin-Kohlenstoffatom in der Teilformel Ia und, falls m 1 ist, auch das Schwefelatom können in der R-, S- oder R,S-Konfiguration vorliegen.

Niederalkyl $R^a$ hat vorzugsweise 1 - 7 C-Atome und ist z.B. Methyl, Ethyl, n-Propyl, Isopropyl, n-Butyl oder tert-Butyl, welche durch eine oder mehrere funktionelle Gruppen, beispielsweise Hydroxy, verethertes Hydroxy, z.B. Niederalkoxy, wie Methoxy oder Ethoxy, oder Phenyloxy, verestertes Hydroxy, z.B. Niederalkanoyloxy, wie Acetoxy, Halogen, z.B. Chlor oder Brom, Hydroxysulfonyloxy, Carboxy, verestertes Carboxy, z.B. Niederalkoxycarbonyl, wie Methoxy- oder Ethoxycarbonyl, amidiertes Carboxy, z.B. Carbamoyl oder Mono- oder Diniederalkylcarbamoyl, wie Methyl- oder Dimethylcarbamoyl, Cyano, Amino, substituiertes Amino, z.B. Mononiederalkylamino, Diniederalkylamino, Acylamino oder substituiertes Amino, worin die Aminogruppe Teil eines fünf- oder sechsgliedrigen Heterocyclus enthaltend ein bis zwei Stickstoffatome und gewünschtenfalls ein Sauerstoff- oder Schwefelatom ist, oder durch Oxo substituiert sein können.

Substituiertes Niederalkyl $R^a$ ist beispielsweise Hydroxyniederalkyl, z.B. 2-Hydroxyethyl, Niederalkoxyniederalkyl, z.B. Niederalkoxyethyl, wie 2-Methoxyethyl, Phenoxyniederalkyl, z.B. 2-Phenoxyethyl, Niederalkanoyloxyniederalkyl, z.B. Niederalkanoyloxyethyl, wie 2-Acetoxyethyl, Halogenniederalkyl, z.B. Halogenethyl, wie 2-Chlor- oder 2-Bromethyl, Hydroxysulfonyloxyniederalkyl, z.B. 2-Hydroxysulfonyloxyethyl, Carboxyniederalkyl, z.B. Carboxymethyl oder 2-Carboxyethyl, Niederalkoxycarbonylniederalkyl, z.B. Niederalkoxycarbonylmethyl oder Niederalkoxy carbonylethyl, wie Methoxycarbonylmethyl, 2-Methoxycarbonylethyl, Ethoxycarbonylmethyl oder 2-Ethoxycarbonylethyl, Carbamoylniederalkyl, z.B. Carbamoylmethyl oder 2-Carbamoylethyl, Niederalkylcarbamoylniederalkyl, z.B. Methylcarbamoylmethyl, Diniederalkylcarbamoylniederalkyl, z.B. Dimethylcarbamoylmethyl, Cyanoniederalkyl, z.B. 2-Cyanoethyl, Aminoniederalkyl, z.B. 2-Aminoethyl, Niederalkylaminoniederalkyl, z.B. 2-Methylaminoethyl, Diniederalkylaminoniederalkyl, z.B. 2-Dimethylaminoethyl, Morpholinoniederalkyl, z.B. 2-Morpholinoethyl, Piperidinoniederalkyl, z.B. 2-Piperidinoethyl, Acylaminoniederalkyl, z.B. Niederalkanoylaminoniederalkyl, wie 2-Acetylaminoethyl, Benzyloxycarbonylaminoniederalkyl, wie 2-Benzyloxycarbonylaminoethyl, Niederalkoxycarbonylaminoniederalkyl, wie 2-tert-Butoxycarbonylaminoethyl, oder Oxoniederalkyl, z.B. 2-Oxopropyl oder 2-Oxobutyl.

Niederalkenyl $R^a$ enthält z.B. 2 - 7, insbesondere 2 - 4, C-Atome und ist z.B. Vinyl, Allyl oder 2- oder 3-Butenyl. Niederalkenyl $R^a$ kann durch die gleichen Substituenten substituiert sein wie Niederalkyl, z.B. durch Hydroxy, verethertes Hydroxy, z.B. Methoxy, verestertes Hydroxy, z.B. Acetoxy, Halogen, z.B. Chlor oder Brom, Carboxy, verestertes Carboxy, z.B. Methoxycarbonyl oder Ethoxycarbonyl, oder amidiertes Carboxy, z.B. Carbamoyl.

Niederalkinyl $R^a$ enthält z.B. 2 - 7, insbesondere 2 - 4, C-Atome und ist beispielsweise Ethinyl, 1-Propinyl oder 2-Propinyl.

Cycloalkyl $R^a$ oder $R^b$ enthält z.B. 3 - 8, insbesondere 3 - 6, C-Atome und ist z.B. Cyclopropyl, Cyclobutyl, Cyclopentyl oder Cyclohexyl.

Bicycloalkyl $R^a$ oder $R^b$ enthält z.B. 5 - 10, insbesondere 6 - 9, C-Atome und ist z.B. Bicyclohexyl, -heptyl, -octyl, -nonyl oder -decyl, z.B. Bicyclo[3.1.0]hex-1-, -2- oder -3-yl, Bicyclo[4.1.0]hept-1- oder -7-yl, Bicyclo[2.2.1]hept-2-yl, z.B. endo- oder exo-Norbornyl, Bicyclo[3.2.1]oct-2-yl, Bicyclo[3.3.0]oct-3-yl oder Bicyclo[3.3.1]non-9-yl, ferner α- oder β-Decahydronaphthyl.

Tricycloalkyl $R^a$ oder $R^b$ enthält z.B. 8 - 10 C-Atome und ist z.B. Tricyclo[5.2.1.$0^{2,6}$]dec-8-yl oder Adamantyl, wie 1-Adamantyl.

Cycloalkylniederalkyl $R^a$ enthält z.B. 4 - 10, insbesondere 4 - 7, C-Atome und ist beispielsweise Cyclopropylmethyl, Cyclobutylmethyl, Cyclopentylmethyl oder Cyclohexylmethyl.

Die genannten cycloaliphatischen oder cycloaliphatisch-aliphatischen Reste können durch die gleichen Substituenten wie Niederalkyl $R^a$ substituiert sein.

Aryl $R^a$ oder $R^b$ enthält z.B. 6 bis 14 C-Atome und ist beispielsweise Phenyl, Indenyl, z.B. 2- oder 4-Indenyl, 1- oder 2-Naphthyl, Anthryl, z.B. 1- oder 2-Anthryl, Phenanthryl, z.B. 9-Phenanthryl, oder Acenaphthenyl, z.B. 1-Acenaphthenyl. Aryl $R^a$ oder $R^b$ ist beispielsweise durch Nideralkyl, z.B. Methyl, Phenyl, Hydroxy, Niederalkoxy, z.B. Methoxy, Acyloxy, z.B. Niederalkanoyloxy, wie Acetoxy, Amino, Niederalkylamino, z.B. Methylamino, Diniederalkylamino, z.B. Dimethylamino, Acrylamino, z.B. tert-Butoxycarbonylamino, oder Halo, z.B. Chlor, Brom oder lod, substituiert, wobei der Substituent in irgendeiner Stellung des Arylrestes, z.B. in o-, m- oder p-Stellung des Phenylrestes, stehen kann und der Arylrest auch mehrfach mit gleichen oder verschiedenen Substituenten substituiert sein kann.

Arylniederalkyl $R^a$ hat z.B. 7 bis 15 C-Atome und enthält beispielsweise einen unter Niederalkyl $R^a$ genannten unsubstituierten oder substituierten, gegebenenfalls verzweigten Rest und einen unter Aryl $R^a$ oder $R^b$ genannten unsubstituierten oder substituierten Rest. Ein solches Arylniederalkyl ist beispielsweise Benzyl, Niederalkylbenzyl, wie 4-Methylbenzyl, Biphenylylmethyl, wie 4-Biphenylylmethyl, Niederalkoxybenzyl, wie 4-Methoxybenzyl, 2-Phenylethyl, 2(p-Hydroxyphenyl)-ethyl, Diphenylmethyl, Di-(4-methoxyphenyl)-methyl, Trityl oder α- oder β-Naphthylmethyl.

Arylniederalkenyl $R^a$ hat z.B. 8 bis 16 C-Atome und enthält beispielsweise einen unter Niederalkenyl $R^a$ genannten unsubstituierten oder substituierten Rest und einen unter Aryl $R^a$ oder $R^b$ genannten unsubstituierten oder substituierten Rest. Ein solches Arylniederalkenyl ist beispielsweise Styryl, 3-Phenylallyl, 2-(α-Naphthyl)-vinyl oder 2-(β-Naphthyl)-vinyl.

Unsubstituiertes oder substituiertes Heteroaryl $R^a$ oder $R^b$ ist mono-, bi- oder tricyclisch und enthält ein bis zwei Stickstoffatome und/oder ein Sauerstoff- oder Schwefelatom. $R^a$ oder $R^b$ ist beispielsweise Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzanelliertes, cyclopenta-, cyclohexa- oder cycloheptaanelliertes Derivat dieser Reste. Dieser Heterocyclus kann an einem Stickstoffatom durch Oxido, Niederalkyl, z.B. Methyl oder Ethyl, Phenyl oder Phenylniederalkyl, z.B. Benzyl, und/oder an einem oder mehreren Kohlenstoff-Atomen durch Niederalkyl, z.B. Methyl, Phenyl, Phenylniederalkyl, z.B. Benzyl, Halogen, z.B. Chlor, Hydroxy, Niederalkoxy, z.B. Methoxy, Phenylniederalkoxy, z.B. Benzyloxy, oder Oxo substituiert und teilweise gesättigt sein und ist beispielsweise 2- oder 3-Pyrrolyl, Phenyl-pyrrolyl, z.B. 4- oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-Pyridyl, 1-Oxido-2-, 3- oder 4-Pyridinio, 2-Pyrazinyl, 2-, 4- oder 5-Pyrimidinyl, 2-, 3- oder 5-Indolyl, substituiertes 2-Indolyl, z.B. 1-Methyl-, 5-Methyl-, 5-Methoxy-, 5-Benzyloxy-, 5-Chlor- oder 4,5-Dimethyl-2-indolyl, 1-Benzyl-2- oder 3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl oder β-Carbolin-3-yl.

Heteroarylniederalkyl $R^a$ enthält z.B. einen unter Niederalkyl $R^a$ genannten unsubstituierten oder substituierten Rest und einen unter Heteroaryl $R^a$ oder $R^b$ genannten unsubstituierten oder substituierten Rest und ist beispielsweise 2- oder 3-Pyrrolylmethyl, 2-, 3- oder 4-Pyridyl methyl, 2-(2-, 3- oder 4-Pyridyl)-ethyl, 4-Imidazolylmethyl, 2-(4-Imidazolyl)-ethyl, 2- oder 3-Indolylmethyl 2-(3-Indolyl)-ethyl oder 2-Chinolylmethyl.

Hydroxy $R^a$ ist unsubstituiert oder beispielsweise durch Niederalkyl oder Aryl substituiert und ist z.B.

Hydroxy, Methoxy, Ethoxy n-Butoxy, Phenoxy, 4-Hydroxyphenoxy oder 3,4-Methylendioxyphenoxy.

Amino $R^a$ ist unsubstituiert, durch eine oder zwei Niederalkylgruppen oder durch Arylniederalkyl, Niederalkanoyl, Niederalkoxycarbonyl oder Arylmethoxycarbonyl substituiert oder Teil eines fünf- oder sechsgliedrigen Heterocyclus enthaltend ein bis zwei Stickstoffatome und gewünschtenfalls ein Sauerstoff- oder Schwefelatom und ist z.B. Amino, Methylamino, Ethylamino, Isopropylamino, n-Butylamino, Dimethylamino, Diethylamino, Benzylamino, Acetylamino, Pivaloylamino, Methoxy-, Ethoxy- oder tert-Butoxycarbonylamino, Benzyloxycarbonylamino, 1-Pyrrolidinyl, 1-Piperidinyl, 1-Methyl-4-pyridazinyl, 4-Morpholinyl oder 4-Thiomorpholinyl.

Salze sind in erster Linie die pharmazeutisch verwendbaren, nichttoxischen Salze von Verbindungen der Formel I.

Solche Salze werden beispielsweise von Verbindungen der Formel I mit einer sauren Gruppe, z.B. einer Carboxygruppe, gebildet und sind in erster Linie geeignete Alkalimetall-, z.B. Natrium- oder Kalium-, oder Erdalkalimetallsalze, z.B. Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, gebildet werden, z.B. mit Diethylamin, Di-(2-hydroxyethyl)-amin, Triethylamin, N,N-Dimethyl-N-(2-hydroxyethyl)-amin, Tri-(2-hydroxyethyl)-amin oder N-Methyl-D-glucamin. Der Verbindungen der Formel I mit einer basischen Gruppe, z.B. einer Aminogruppe, können Säureadditionssalze bilden, z.B. mit anorganischen Säuren, z.B. Salzsäure, Schwefelsäure oder Phosphorsäure, oder mit organischen Carbon-, Sulfon- oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner mit Aminosäuren, wie z.B. den weiter vom genannten α-Aminosäuren, sowie mit Methansulfonsäure, Ethansulfonsäure, 2-Hydroxyethansulfonsäure, Ethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Die Verbindungen der vorliegenden Erfindung weisen gag-Protease-hemmende Wirkungen auf. Insbesondere hemmen sie in Konzentrationen bis herunter zum nanomolaren Bereich die Wirkung der gag-Protease von HIV-1 und HIV-2 und sind daher geeignet als Mittel gegen durch diese oder verwandte Retroviren verursachte Krankheiten, wie z.B. gegen AIDS.

Die Fähigkeit der Verbindungen der Formel I, die proteolytische Aktivität von z.B. HIV-1 Protease zu inhibieren, lässt sich beispielsweise gemäss dem von J. Hansen et al., The EMBO Journal 7, 1785-1791 (1988), beschriebenen Verfahren demonstrieren. Dabei wird die Hemmung der Wirkung der gag-Protease auf ein Substrat gemessen, das ein in E. coli exprimiertes Fusionsprotein aus dem gag-Vorläuferprotein und MS-2 ist. Das Substrat und seine Spaltprodukte werden durch Polyacrylamid-Gelektrophorese getrennt und durch Immunoblotting mit monoklonalen Antikörpern gegen MS-2 sichtbar gemacht.

In einem noch einfacher zu handhabenden Test, der genaue quantitative Aussagen ermöglicht, wird als Substrat für die gag-Protease ein synthetisches Icosapeptid eingesetzt, das der Spaltstelle des gag-Vorläuferproteins entspricht. Dieses Substrat und seine Spaltprodukte können durch Hochdruckflüssig-Chromatographie (HPLC) gemessen werden. Verbindungen der vorliegenden Erfindung zeigen in diesem Test Hemmwirkungen in Konzentrationen von $10^{-6}$ Mol/l.

In einem weiteren Test kann gezeigt werden, dass die Verbindungen der vorliegenden Erfindung Zellen, die normalerweise von HIV infiziert werden, vor einer solchen Infektion schützt oder zumindest eine solche Infektion verlangsamt. Dabei wird die menschliche T-Zell-Leukämie Zellinie MT-2 (Science 229, 563 (1985)), die extrem empfindlich für den zytopathogenen Effekt von HIV ist, mit HIV allein oder mit HIV in Gegenwart der erfindungsgemässen Verbindungen inkubiert und nach einigen Tagen die Lebensfähigkeit der so behandelten Zellen beurteilt. Erfindungsgemässe Verbindungen zeigen Infektions-hemmende Wirkungen in Konzentrationen von $10^{-5}$ Mol/l.

Bevorzugte Verbindungen der Formel I sind dadurch gekennzeichnet, dass sie im Radikal AAN zwei oder mehr α-Aminosäurereste aufweisen oder dass das Radikal AAN zus nur einem α-Aminosäurerest besteht und gleichzeitig der Rest $R^2$ ein Analogon zu Phenylalanyl (H-Phe) ist und/oder dass sie im Radikal AAC zwei α-Aminosäurereste aufweisen oder dass das Radikal AAC aus nur einem α-Aminosäurerest besteht und gleichzeitig der Rest $R^1$ ein Analogon zum über Stickstoff gebundenen Rest der Aminosäure Tyrosin (-Tyr-OH) ist.

Ein Rest $R^2$, der als Analogon zu Phenylalanyl gelten kann, weist das Strukturelement der Formel

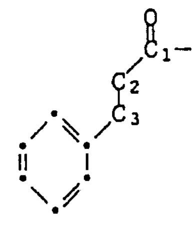

(Ib)

auf, worin der carbocyclische Ring auch ganz oder teilweise gesättigt sein kann, eines der Kohlenstoffatome des carbocyclischen Ringes mit $C_2$ oder $C_3$ zu einem vorzugsweise fünf- oder sechsgliedrigen Ring verknüpft sein kann, eines der Kohlenstoffatome $C_2$ oder $C_3$ durch ein Heteroatom, wie NH, O oder S, ersetzt sein kann, die Carbonylgruppe eine Heterocarbonylgruppe wie P=O sein kann und freie Valenzen Wasserstoff oder Substituenten tragen, beispielsweise Niederalkyl, Niederalkylthioniederalkyl, Niederalkylsulfinylniederalkyl, Niederalkylsulfonylniederalkyl, Phenylniederalkyl, Hydroxy, Niederalkoxy, Phenylniederalkoxy, Amino niederalkyl, Niederalkylaminoniederalkyl, Niederalkanoylaminoniederalkyl, Niederalkanoyl, Niederalkanolyniederalkyl oder Niederalkylcarbamoyl, wobei diese Substituenten bevorzugt an $C_2$ vorkommen.

Ein ganz oder teilweise gesättigter carbocyclischer Ring ist beispielsweise in diesem Zusammenhang Cyclohexyl oder Cyclohexenyl. Fünf- oder sechsgliedrige Ringe im Sinne des vorstehenden Absatzes sind beispielsweise Phenyl, Cyclohexyl, Pyrrolyl, Pyranyl, Dihydropyranyl oder Thioanaloga der genannten Heterocyclylreste. Diese Definitionen gelten auch für die nachfolgend beschriebenen zu Tyrosin analogen Reste $R^1$.

Derartige Analoga $R^2$ sind beispielsweise 3-Phenyl- oder 3-Cyclohexylpropionyl, die insbesondere in 2-Stellung durch Substituenten der Art Niederalkyl, wie Methyl, Ethyl oder tert-Butyl, Niederalkylthioniederalkyl, wie tert-Butylthiomethyl, Niederalkylsulfinylniederalkyl, wie tert-Butylsulfinylmethyl, Niederalkylsulfonylniederalkyl, wie tert-Butylsulfonylmethyl, Phenylniederalkyl, wie Benzyl, Hydroxy, Niederalkoxy, wie Methoxy, Phenylniederalkoxy, wie Benzyloxy, Aminoniederalkyl, wie Aminomethyl oder 2-Amino-2-propyl, Niederalkylaminoniederalkyl, wie Methylaminomethyl, Niederalkanoylaminoniederalkyl, wie Acetylaminomethyl, Niederalkanoyl, wie Acetyl, Niederalkanoylniederalkyl, wie Acetylmethyl, Isobutyrylmethyl oder Pivaloylmethyl, oder Niederalkylcarbamoyl, wie Methylcarbamoyl, substituiert sein können, 2-Naphthylcarbonyl oder hydrierte Formen davon, wie z.B. 2-Decahydronaphthylcarbonyl, 2-(3-Indolyl)-acetyl oder 2-(3-Benzofuranyl)-acetyl, 3-Chroman- oder 3-Thiochromancarbonyl, oder Dibenzyloxyphosphoryl.

Ein Rest $R^1$, der als Analogon zum über Stickstoff gebundenen Rest der Aminosäure Tyrosin gelten kann, weist das Strukturelement der Formel

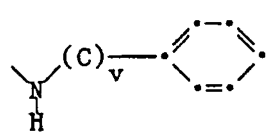

(Ic)

auf, worin der carbocyclische Ring ein Heteroatom, wie Stickstoff, enthalten kann und auch ganz oder teilweise gesättigt und/oder substituiert, beispielsweise durch Hydroxy oder durch Phenyl, sein kann, der Index v für null bis fünf, beispielsweise null bis drei, steht, eines der Kohlenstoffatome des carbocyclischen Ringes mit einem der Atome $-(C)_v-$ zu einem vorzugsweise fünf- oder sechsgliedrigen Ring verbunden sein kann und freie Valenzen Wasserstoff oder Substituenten tragen, beispielsweise Niederalkyl oder Hydroxyniederalkyl. Ferner kann ein solcher Rest $R^1$, der als Analogon zum Rest der Aminosäure Tyrosin gelten kann, auch Niederalkylamino sein.

Derartige Analoga $R^1$ sind beispielsweise Butylamino, Cyclohexylamino, 2-Decahydronaphthylamino, 6-Hydroxy-2-tetrahydronaphthylamino oder 2-Tetrahydronaphthylamino, 6-Hydroxy-2-naphthylamino oder 2-Naphthylamino, Pyridylmethylamino, Phenyl- oder Hydroxyphenylniederalkylamino, wie 2-Phenyl- oder Hydroxyphenylethylamino oder 5-Phenyl- oder Hydroxyphenylpentylamino, 1-Hydroxymethyl-2-hydroxyphenylethylamino oder Biphenylylniederalkylamino, wie 4-Biphenylylmethylamino.

Bevorzugte erfindungsgemässe Verbindungen sind Verbindungen der Formel II

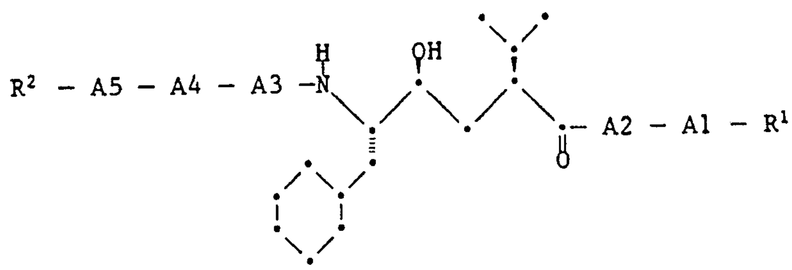

(II)

worin die Radikale A1, A2, A3 und A4 je einen bivalenten α-Aminosäurerest bedeuten, der N-terminal mit dem in Formel II jeweils links davon stehenden Radikal und C-terminal mit dem jeweils rechts davon stehenden Radikal bzw. mit dem Rest $R^1$ verbunden ist, A5 für eine einfache Bindung steht oder einen bivalenten Rest bestehend aus bis zu drei peptidisch verknüpften α-Aminosäuren bedeutet, welcher N-terminal mit $R^2$ und C-terminal mit A4 verbunden ist, und die anderen Symbole die für Formel I angegebenen Bedeutungen aufweisen.

Bevorzugt sind Verbindungen der Formel II, worin A1 ausgewählt ist unter den bivalenten Resten von Tyrosin (Tyr), Phenylalanin (Phe), Naphthylalanin (Nal), Tryptophan (Trp), Lysin (Lys) und Asparaginsäure (Asp), A2 ausgewählt ist unter den bivalenten Resten von Valin (Val), Isoleucin (Ile), Leucin (Leu), Norleucin (Nle), Phenylalanin (Phe), Alanin (Ala) und Glycin (Gly), A3 ausgewählt ist unter den bivalenten Resten von Valin (Val), Alanin (Ala), Leucin (Leu), Isoleucin (Ile), Asparagin (Asn), Glutamin (Gln), Norleucin (Nle), Phenylalanin (Phe), Serin (Ser) und Histidin (His), A4 ausgewählt ist unter den bivalenten Resten von Phenylalanin (Phe), Tyrosin (Tyr) (unter Einschluss von durch Niederalkyl verethertem Tyrosin), Tryptophan (Trp), Cyclohexylalanin (Cha), Leucin (Leu), Naphthylalanin (Nal), Histidin (His), Asparaginsäure (Asp) und Lysin (Lys), und die anderen Symbole die für Formel II angegebenen Bedeutungen aufweisen.

Bevorzugt sind ferner Verbindungen der Formel II, worin A1, A2, A3 und A4 die unmittelbar vorstehend angegebenen Bedeutungen aufweisen, A5 für eine einfache Bindung steht oder einen bivalenten Rest bestehend aus bis zu drei gleichen oder verschiedenen, peptidisch verknüpften α-Aminosäuren ausgewählt unter Arginin, Prolin, Histidin, Lysin, Ornithin oder Tryptophan bedeutet, $R^1$ für Hydroxy, Niederalkoxy, Amino oder Mono- oder Diniederalkylamino steht, und $R^2$ Wasserstoff, Niederalkanoyl, Niederalkoxycarbonyl, Arylniederalkoxycarbonyl oder einen Rest der Formel

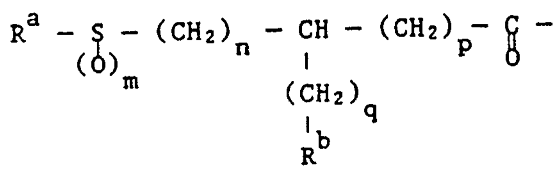

(Ia),

worin $R^a$ unsubstituiertes oder durch Hydroxy oder Niederalkoxy substituiertes Niederalkyl, z.B. Methyl, Ethyl, Isopropyl, tert-Butyl, 2-Hydroxyethyl oder 2-Methoxyethyl, Phenyl, Benzyl oder unsubstituiertes oder durch Oxido oder Niederalkyl substituiertes Heteroaryl mit 1 oder 2 Stickstoffatomen, z.B. 2- oder 4-Imidazolyl, 1-Methyl-2-imidazolyl, 2-, 3- oder 4-Pyridyl, 1-Oxido-2-, 3- oder 4-Pyridinio oder 2-Pyrimidinyl, $R^b$ Cyclohexyl oder Phenyl, m 0, 1 oder 2, n 1, p 0 und q 1 oder 2 darstellen, bedeutet, sowie Salze davon.

Hierunter sind diejenigen Verbindungen bevorzugt, in denen A5 für eine einfache Bindung oder den bivalenten Rest Arg-Arg-Pro steht.

Besonders bevorzugt sind Verbindungen der Formel II, worin A1 ausgewählt ist unter Tyr, Phe, Trp, Nal, Lys und Asp, A2 und A3 ausgewählt sind unter Val, Ile, Leu, Nle und Ala, A4 Phe, Tyr oder Nal bedeutet, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Niederalkoxy substituiertes Phenylniederalkoxycarbonyl, Diphenylniederalkoxycarbonyl oder Fluorenylniederalkoxycarbonyl bedeutet, sowie Salze davon.

Besonders bevorzugt sind ebenfalls Verbindungen der Formel II, worin A1 Tyr bedeutet, A2 ausgewählt ist unter Val, Ile, Nle, Leu, Phe, Ala und Gly, A3 Val bedeutet, A4 Phe, Tyr oder Nal bedeutet, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Niederalkoxy substituiertes Phenylniederalkoxycarbonyl, Diphenylniederalkoxycarbonyl oder Fluorenylniederalkoxycarbonyl bedeutet, sowie Salze davon.

Besonders bevorzugt sind ebenfalls Verbindungen der Formel II, worin A1 Tyr bedeutet, A2 für Val oder Leu steht, A3 ausgewählt ist unter Val, Leu, Ile, Nle, Ala, Asn, Gln, Phe, Ser und His, A4 Phe bedeutet, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Niederalkoxy substituiertes Phenylniederalkoxycarbonyl, Diphenylniederalkoxycarbonyl oder Fluorenylniederalkoxycarbonyl bedeutet, sowie Salze davon.

Besonders bevorzugt sind ebenfalls Verbindungen der Formel II, worin A1 Tyr bedeutet, A2 für Val oder Leu steht, A3 für Val steht, A4 ausgewählt ist unter Phe, Tyr, mit Methyl verethertem Tyr, Trp, Cha, Leu, Nal und Lys, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Niederalkoxy substituiertes Phenylniederalkoxycarbonyl, Diphenylniederalkoxycarbonyl oder Fluorenylniederalkoxycarbonyl bedeutet, sowie Salze davon.

Bevorzugt sind ferner die unter die Verbindungen der Formel I fallenden Verbindungen der Formel III

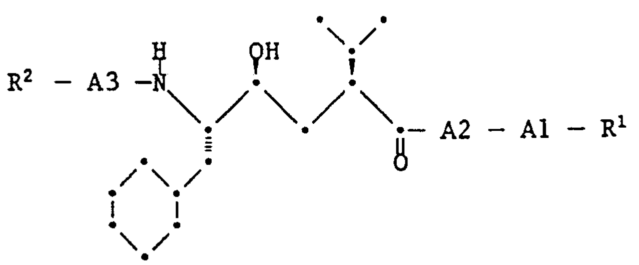

(III)

worin alle Symbole die für Formel II angegebenen Bedeutungen aufweisen.

Bevorzugt sind Verbindungen der Formel III, worin A1 ausgewählt ist unter den bivalenten Resten von Tyrosin (Tyr), Phenylalanin (Phe), Tryptophan (Trp), α-Naphthylalanin (Nal), Lysin (Lys) und Asparaginsäure (Asp), A2 ausgewählt ist unter den bivalenten Resten von Valin (Val), Isoleucin (Ile), Leucin (Leu), Norleucin (Nle), Phenylalanin (Phe), Alanin (Ala) und Glycin (Gly), A3 ausgewählt ist unter den bivalenten Resten von Valin (Val), Alanin (Ala), Leucin (Leu), Isoleucin (Ile), Asparagin (Asn), Glutamin (Gln), Norleucin (Nle), Phenylalanin (Phe), Serin (Ser) und Histidin (His), und die anderen Symbole die für Formel I angegebenen Bedeutungen aufweisen.

Gleichermassen bevorzugt sind Verbindungen der Formel III, worin A1, A2, $R^1$ und $R^2$ die genannten Bedeutungen haben und A3 der bivalente Rest von Tyrosin (Tyr) oder von mit Niederalkyl verethertem Tyrosin ist.

Bevorzugt sind ferner Verbindungen der Formel III, worin A1, A2 und A3 die unmittelbar vorstehend angegebenen Bedeutungen aufweisen, $R^1$ für Hydroxy, Niederalkoxy, Amino oder Mono- oder Diniederalkylamino steht, und $R^2$ einen Rest der Formel

$$R^a - \underset{(O)_m}{S} - (CH_2)_n - \underset{\underset{R^b}{|}}{\underset{(CH_2)_q}{\underset{|}{CH}}} - (CH_2)_p - \underset{O}{\overset{}{\underset{\|}{C}}} - \qquad (Ia)$$

bedeutet, worin $R^a$ unsubstituiertes oder durch Hydroxy oder Niederalkoxy substituiertes Niederalkyl, z.B. Methyl, Ethyl, Isopropyl, tert-Butyl, 2-Hydroxyethyl oder 2-Methoxyethyl, Phenyl, Benzyl oder unsubstituiertes oder durch Oxido oder Niederalkyl substituiertes Heteroaryl mit 1 oder 2 Stickstoffatomen, z.B. 2- oder 4-Imidazolyl, 1-Methyl-2-imidazolyl, 2-, 3- oder 4-Pyridyl, 1-Oxido-2-, 3- oder 4-pyridinio oder 2-Pyrimidinyl, $R^b$ Cyclohexyl oder Phenyl, m 0, 1 oder 2, n 1, p 0 und q 1 oder 2 darstellen.

Gleichermassen bevorzugt sind Verbindungen der Formel III, worin A1, A2, A3 und $R^1$ die unmittelbar vorstehend angegebenen Bedeutungen aufweisen und $R^2$ einen Rest mit dem Strukturelement Ib, der als Analogon zu Phenylalanyl gelten kann, darstellt, wie er weiter vorn definiert ist.

Besonders bevorzugt sind Verbindungen der Formel III, worin A1 ausgewählt ist unter Tyr, Phe, Trp, Nal, Lys und Asp, A2 und A3 Val oder Leu bedeuten, $R^1$ für Hydroxy, Niederalkoxy, Niederalkylamino oder Amino steht, und $R^2$ einen Rest der Formel Ia bedeutet, worin $R^a$ für Niederalkyl steht, $R^b$ Phenyl bedeutet und m zwei, n eins, p null und q eine bedeuten, sowie Salze davon.

Besonders bevorzugt sind ebenfalls Verbindungen der Formel III, worin A1 Tyr bedeutet, A2 ausgewählt ist unter Val, Ile, Leu, Nle, Phe, Ala und Gly, A3 Val oder Leu bedeutet, $R^1$ für Hydroxy, Niederalkoxy, Niederalkylamino oder Amino steht, und $R^2$ einen Rest der Formel Ia bedeutet, worin $R^a$ für Niederalkyl steht, $R^b$ Phenyl bedeutet und m zwei, n eins, p null und q eins bedeuten, sowie Salze davon.

Besonders bevorzugt sind ebenfalls Verbindungen der Formel III, worin A1 Tyr bedeutet, A2 für Val oder Leu steht, A3 ausgewählt ist unter Val, Ala, Leu, Ile, Asn, Gln, Nle, Phe, Ser und His, $R^1$ für Hydroxy, Niederalkoxy, Niederalkylamino oder Amino steht, und $R^2$ einen Rest der Formel Ia bedeutet, worin $R^a$ für Niederalkyl steht, $R^b$ Phenyl bedeutet und m zwei, n eins, p null und q eins bedeuten, sowie Salze davon.

Besonders bevorzugt sind ebenfalls Verbindungen der Formel III, worin A1 Tyr bedeutet, A2 Val bedeutet, A3 ausgewählt ist unter Val, Leu, Nle, Phe, Ala, Ser, Tyr und mit Niederalkyl verethertem Tyr, $R^1$ für Hydroxy, Niederalkoxy, Niederalkylamino oder Amino steht, und $R^2$ 3-Phenyl- oder 3-Cyclohexyl-propionyl bedeutet, das in 2-Stellung durch Niederalkylsulfonylniederalkyl oder durch Niederalkanoylniederalkyl substituiert ist, sowie Salze davon.

Bevorzugt sind ebenfalls die unter die Verbindungen der Formel I fallenden Verbindungen der Formel

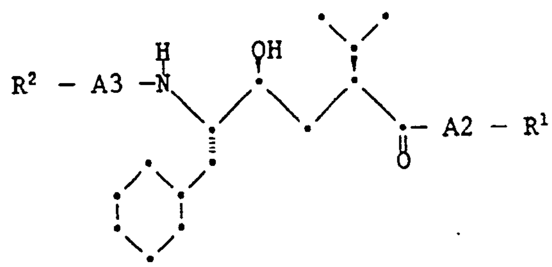 (IV),

worin A2 und A3 die unter Formel I angegebenen Bedeutungen aufweisen, $R^1$ für einen Rest steht, der als Analogon zum über Stickstoff gebundenen Rest der Aminosäure Tyrosin gelten kann und $R^2$ für einen Rest steht, der als Analogon zu Phenylalanyl gelten kann, sowie Salze davon.

Unter den Verbindungen der Formel IV sind jene bevorzugt, bei denen A2 ausgewählt ist unter den bivalenten Resten von Valin (Val), Isoleucin (Ile), Norleucin (Nle), Leucin (Leu), Phenylalanin (Phe), Alanin (Ala) und Glycin (Gly), und A3 ausgewählt ist unter den bivalenten Resten von Valin (Val), Alanin (Ala), Leucin (Leu), Asparagin (Asn), Glutamin (Gln), Norleucin (Nle), Phenylalanin (Phe), Serin (Ser) und Histidin (His), insbesondere jene, in denen A2 und A3 jeweils für Val oder Leu stehen.

Besonders bevorzugt sind Verbindungen der Formel IV, worin A2 Valin bedeutet, A3 ausgewählt ist unter Val, Leu, Nle, Phe, Ala, Ser, Tyr und mit Niederalkyl verethertem Tyr, $R^1$ Niederalkylamino oder ein Rest mit dem Strukturelement der Formel Ic ist, worin der carbocyclische Ring unsubstituiert oder durch Hydroxy oder durch Phenyl substituiert sein kann, der Index v für null bis fünf steht und freie Valenzen Wasserstoff tragen, und $R^2$ 3-Phenyl- oder 3-Cyclohexyl-propionyl bedeutet, das in 2-Stellung durch Niederalkylsulfonylniederalkyl oder durch Niederalkanoylniederalkyl substituiert ist, sowie Salze davon.

Die Erfindung betrifft zuallererst die in den Beispielen genannten Verbindungen und ihre Salze.

Die erfindungsgemässen Verbindungen der Formel I und Salze von solchen Verbindungen mit mindestens einer salzbildenden Gruppe werden nach an sich bekannten Verfahren erhalten, z.B. indem man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene funktionelle Gruppen mit Ausnahme der an der Reaktion teilnehmenden Gruppen gegebenenfalls in geschützter Form vorliegen, unter Bildung einer Amidbindung kondensiert, oder

b) die Ketogruppe in einer Verbindung der Formel

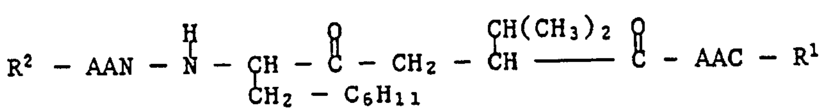 (V),

worin die Symbole die genannten Bedeutungen haben und funktionelle Gruppen mit Ausnahme der an der Reaktion teilnehmenden Ketogruppe gegebenenfalls in geschützter Form vorliegen, durch Umsetzung mit

einem geeigneten Reduktionsmittel zu einer Hydroxygruppe reduziert, oder

c) eine Aldehyd-Verbindung der Formel

$$R^2 - AAN - \overset{H}{\overset{|}{N}} - \underset{\underset{CH_2 - C_6H_{11}}{|}}{CH} - \overset{O}{\overset{\|}{C}} - H \qquad (VI),$$

worin die Symbole die genannten Bedeutungen haben und funktionelle Gruppen mit Ausnahme der Aldehydgruppe gegebenenfalls in geschützter Form vorliegen, mit einer Organometallverbindung der Formel

$$M - CH_2 - \overset{CH(CH_3)_2}{\overset{|}{CH}} \text{———} \overset{O}{\overset{\|}{C}} - AAC - R^1 \qquad (VII),$$

worin die Symbole die genannten Bedeutungen haben und M ein Metallradikal bedeutet, umsetzt und das gebildete Additionsprodukt hydrolysiert, oder

d) in einer Verbindung der Formel

$$R^2 - AAN - \overset{H}{\overset{|}{N}} - \underset{\underset{CH_2 - C_6H_{11}}{|}}{CH} - \overset{X}{\overset{|}{CH}} - CH_2 - \overset{CH(CH_3)_2}{\overset{|}{CH}} \text{———} \overset{O}{\overset{\|}{C}} - AAC - R^1 \qquad (VIII),$$

worin X eine nukleofuge Abgangsgruppe ist, die übrigen Symbole die oben genannten Bedeutungen haben und funktionelle Gruppen gegebenenfalls in geschützter Form vorliegen, den Substituenten X durch eine Hydroxygruppe austauscht, oder

e) in einer Verbindung der Formel

$$R^2 - AAN - \overset{H}{\overset{|}{N}} - \underset{\underset{CH_2 - C_6H_{11}}{|}}{CH} - \overset{OH}{\overset{|}{CH}} - CH_2 - \overset{CH(CH_3)_2}{\overset{|}{CH}} \text{———} \overset{O}{\overset{\|}{C}} - AAC' - CN \qquad (IX),$$

worin die Symbole die genannten Bedeutungen haben und AAC′ die Bedeutung von AAC abzüglich der terminalen Carbonylgruppe hat und vorhandene funktionelle Gruppen gegebenenfalls in geschützter Form vorliegen, die Gruppe AAC′-CN in eine Gruppe AAC-$R^1$ überführt, oder

f) in einer Verbindung der Formel

$$R^2 - AAN - \overset{H}{\overset{|}{N}} - \underset{\underset{CH_2 - C_6H_{11}}{|}}{CH} - \overset{O}{CH\text{-}CH} - \overset{CH(CH_3)_2}{\overset{|}{CH}} \text{———} \overset{O}{\overset{\|}{C}} - AAC - R^1 \qquad (X),$$

worin die Symbole die genannten Bedeutungen haben und funktionelle Gruppen gegebenenfalls in geschützter Form vorliegen, mit einem regioselectiven Reduktionsmittel die Epoxygruppe zu einer Hydroxygruppe reduziert, oder

g) zur Herstellung einer Verbindung der Formel I, worin $R^2$ einen Rest der Formel

$$R^a - \underset{(O)_m}{S} - (CH_2)_n - \underset{\underset{R^b}{|}}{\underset{(CH_2)_q}{\underset{|}{CH}}} - (CH_2)_p - \underset{O}{\overset{}{\underset{\|}{C}}} - \qquad (Ia)$$

und m 0 oder 2, n 1 und p 0 bedeuten, eine Verbindung der Formel $R^a$-$S(O)_mH$ oder ein Salz davon an eine Verbindung der Formel

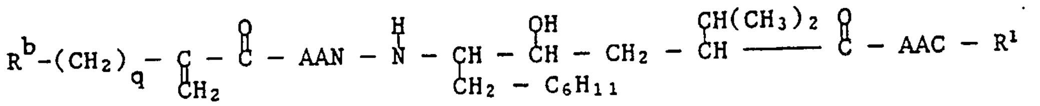 (XI),

worin die Symbole die genannten Bedeutungen haben und funktionelle Gruppen gegebenenfalls in geschützter Form vorliegen, addiert, oder

h) zur Herstellung einer Verbindung der Formel I, worin $R^2$ einen Rest der Formel

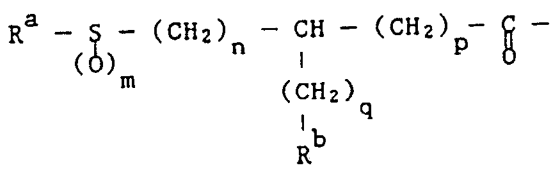 (Ia)

und p O bedeutet, eine Verbindung der Formel

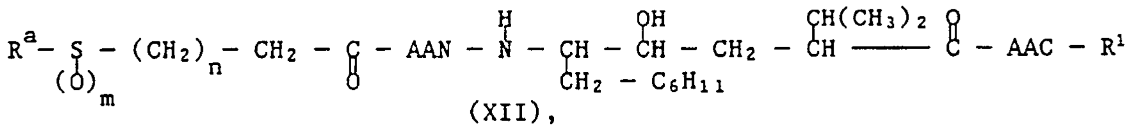

(XII),

worin die Symbole die genannten Bedeutungen haben und funktionelle Gruppen gegebenenfalls in geschützter Form vorliegen, mit einer den Rest $R^b$-$(CH_2)q$- einführenden Verbindung alkyliert, und gewünschtenfalls

i) in einer erhältlichen Verbindung vorhandene Schutzgruppen abspaltet und/oder gewünschtenfalls nach Ausführung eines der vorstehend genannten Verfahren a) - h) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

Die Erfindung betrifft auch die nach irgendeinem der oben genannten Verfahren erhältlichen anderen Verbindungen als Verbindungen der Formel I (Nebenprodukt), sowie Verbindungen der Formel I und ihre Salze, welche nach einem anderen Verfahren als einem der weiter vorn genannten hergestellt werden.

<u>Verfahren a) (Herstellung einer Amidbindung):</u>

Fragmente einer Verbindung der Formel I mit einer endständigen Carboxygruppe, welche mit einem zur Verbindung der Formel I komplementären Fragment unter Bildung einer Amidbindung kondensiert werden können, sind z.B. Verbindungen der Formeln $R^2$-OH, $R^2$-A5-OH, $R^2$-A5-A4-OH, $R^2$-AAN-OH,

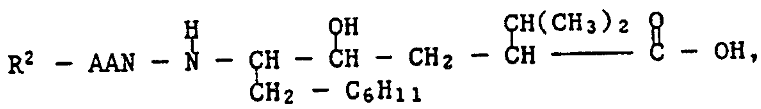

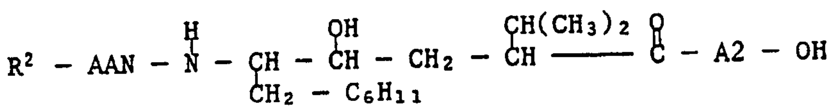 oder

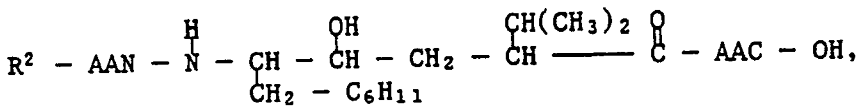

die von diesen Verbindungen abgeleiteten aktivierten Ester oder reaktionsfähigen Anhydride, ferner reaktionsfähige cyclische Amide. Die reaktionsfähigen Säurederivate können auch in situ gebildet werden.

Aktivierte Ester sind insbesondere am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylvinylester (erhältlich z.B. durch Behandeln der entsprechenden Säure mit einem Isoxazoliumreagens; 1,2-Oxazolium- oder Woodward-Methode) oder 1-Niederalkoxyvinylester (erhältlich z.B. durch Behandeln der ent sprechenden Säure mit einem Niederalkoxyacetylen; Ethoxyacetylen-Methode), oder Ester vom Amidinotyp, wie N,N′-disubstituierte Amidinoester (erhältlich z.B. durch Behandeln der entsprechenden Säure mit einem geeigneten N,N′-disubstituierten Carbodiimid, z.B. N,N′-Dicyclohexylcarbodiimid; Carbodiimid-Methode) oder N,N-disubstituierte Amidinoester (erhältlich z.B. durch Behandeln der entsprechenden Säure mit einem N,N-disubstituierten Cyanamid; Cyanamid-Methode), geeignete Arylester, insbesondere durch elektronenanziehende Substituenten substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N′-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u.a. mit Hilfe der Anhydrid- oder Carbodiimide-Methode; Methode der aktivierten Thiolester) oder insbesondere Amino- oder Amidoester (erhältlich z.B. durch Behandeln der entsprechenden Säure mit einer N-Hydroxyamino- bzw. N-Hydroxyamido-Verbindung und deren aktivierten Derivaten, z.B. N-Hydroxysuccinimid, N-Hydroxypiperidin, N-Hydroxyphthalimid, N-Hydroxy-5-norbornen- oder norbornan-2,3-dicarbonsäureimid, 1-Hydroxybenztriazol bzw. Benzotriazol-1-yloxy-phosphoniumsalzen oder 0-Benzotriazol-1-yluroniumsalzen, oder 3-Hydroxy-3,4-dihydro-1,2,3-benzotriazin-4-on, z.B. nach der Anhydrid- oder Carbodiimid-Methode; Methode der aktivierten N-Hydroxyester).

Anhydride von Säuren können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z.B. durch Behandeln der entsprechenden Säure mit Chlorameisensäureniederalkylestern oder mit einem 1-Niederalkoxycarbonyl-2-niederalk oxy-1,2-dihydrochinolin, z.B. 1-Ethoxycarbonyl-2-ethoxy-1,2-dihydrochinolin; Methode der gemischten O-Alkylkohlensäureanhydride), Anhydride mit dihalogenierter, insbesondere dichlorierter Phosphorsäure (erhältlich z.B. durch Behandeln der entsprechenden Säure mit Phosphoroxychlorid; Phosphoroxychloridmethode), Anhydride mit anderen Phosphorsäurederivaten (z.B. solchen, die man mit Phenyl-N-phenylphosphoramidochloridat erhalten kann) oder mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimids oder von 1-Diethylaminopropin; Methode der symmetrischen Anhyride).

Geeignete cyclische Amide sind insbesondere Amide mit fünfgliedrigen Diazacyclen aromatischen Charakters, wie Amide mit Imidazolen, z.B. Imidazol (erhältlich z.B. durch Behandeln der entsprechenden Säure mit N,N′-Carbonyldiimidazol; Imidazol-Methode), oder Pyrazolen, z.B. 3,5-Dimethylpyrazol (erhältlich z.B. über das Säurehydrazid durch Behandeln mit Acetylaceton; Pyrazolid-Methode).

Zur Verbindung der Formel I komplementäre Fragmente mit einer freien Aminogruppe sind z.B. je nach Bedeutung von $R^1$ ein primäres oder sekundäres Amin, ferner Verbindungen der Formeln

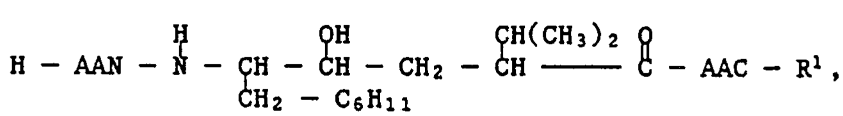

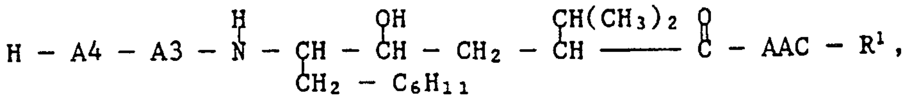

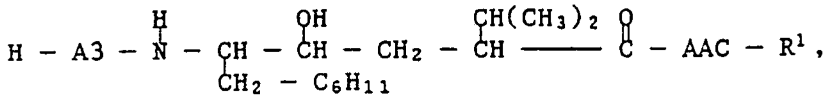

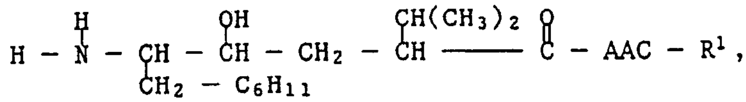

H - AAC - $R^1$ oder H - Al - $R^1$.

Die an der Reaktion teilnehmende Aminogruppe in einem zu einer Verbindung der Formel I komplementären Fragment liegt bevorzugt in freier Form vor, insbesondere wenn die damit reagierende Carboxygruppe in reaktionsfähiger Form vorliegt; sie kann aber auch selbst derivatisiert sein, z.B. durch Reaktion mit einem Phosphit, wie Diethylchlorphosphit, 1,2-Phenylenchlorphosphit, Ethyldichlorphosphit, Ethylenchlorphosphit oder Tetraethylpyrophosphit. Ein Derivat eines solchen komplementären Bruchstücks mit einer Aminogruppe ist z.B. auch ein Carbaminsäurehalogenid oder ein Isocyanat, wobei die an der Reaktion teilnehmende Aminogruppe durch Halogencarbonyl, z.B. Chlorcarbonyl, substituiert bzw. als Isocyanatgruppe abgewandelt ist, wobei im letzteren Falle nur Verbindungen der Formel I zugänglich sind, die am Stickstoffatom der durch die Reaktion gebildeten Amidgruppe ein Wasserstoffatom tragen.

Ist das komplementäre Fragment mit einer Aminogruppe ein durch Niederalkyl oder Arylniederalkyl mono- oder disubstituiertes Amin, so stellt auch eine entsprechende Harnstoff-Verbindung ein reaktionsfähiges Derivat dar. Beispielsweise erhält man beim Erhitzen äquimolarer Mengen dieser Harnstoffverbindung und der Komponente mit freier Carboxygruppe entsprechende Verbindungen der Formel I. Ist das komplementäre Fragment Dimethylamin, so ist auch Dimethylformamid ein reaktionsfähiges Derivat.

Funktionelle Gruppen in Ausgangsmaterialien, deren Umsetzung vermieden werden soll, insbesondere Carboxy-, Amino-, Hydroxy-, Mercapto- und Sulfogruppen, können durch geeignete Schutzgruppen geschützt sein, wie sie üblicherweise bei der Synthese von Peptid-Verbindungen, aber auch von Cephalosporinen und Penicillinen verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. Schutzgruppen können aber auch in den Endstoffen vorhanden sein. Verbindungen der Formel I mit geschützten funktionellen Gruppen können eine höhere metabolische Stabilität aufweisen als die entsprechenden Verbindungen mit freien funktionellen Gruppen.

Der Schutz von funktionellen Gruppen durch solche Schutzgruppen, die Schutzgruppen selbst, sowie ihre Abspaltungsreaktionen, sind beispielsweise in Standardwerken wie in J.F.W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (Herausg. E. Gross und J. Meienhofer), Academic Press, London und New York 1981, sowie in "Methoden der organischen Chemie", Houben-Weyl, 4. Auflage, Bd. 15/I, Georg Thieme Verlag, Stuttgart 1974, beschrieben.

Eine Carboxygruppe ist z.B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z.B. tert-Butoxycarbonyl, oder Arylmethoxycarbonyl mit einem oder zwei Arylresten, worin Aryl unsubstituiertes oder z.B. durch Niederalkyl, z.B. tert-Niederalkyl, wie tert-Butyl, Niederalkoxy, z.B. Methoxy, Hydroxy, Halogen, z.B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z.B. 4-Nitrobenzyloxycarbonyl oder 4-Methoxybenzyl oxycarbonyl, Diphenylmethoxycarbonyl oder durch die genannten Substituenten substituiertes Diphenylmethoxycarbonyl, z.B. Di-(4-methoxyphenyl)-methoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise 1-Niederalkoxyniederalkoxycarbonyl, z.B. Methoxymethoxycarbonyl, 1-Methoxyethoxycarbonyl oder 1-Ethoxyethoxycarbonyl, 1-Niederalkylthioniederalkoxycarbonyl, z.B. 1-Methylthiomethoxycarbonyl oder 1-Ethylthioethoxycarbonyl, Aroylmethoxycarbonyl, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, sowie 2-Triniederalkylsilylniederalkoxycarbonyl, z.B. 2-Trimethylsilylethoxycarbonyl.

Eine Carboxygruppe kann auch als organische Silyloxycarbonylgruppe geschützt sein. Eine organische Silyloxycarbonylgruppe ist beispielsweise eine Triniederalkylsilyloxycarbonylgruppe, z.B. Trimethylsilyloxycarbonyl. Das Siliciumatom der Silyloxycarbonylgruppe kann auch durch zwei Niederalkylgruppen, z.B. Methylgruppen, und die Aminogruppe oder die Carboxygruppe eines zweiten Moleküls der Formel I substituiert sein. Verbindungen mit solchen Schutzgruppen lassen sich z.B. mit Dimethylchlorsilan als Silylierungsmittel herstellen.

Eine geschützte Carboxygruppe ist bevorzugt tert-Niederalkoxycarbonyl, z.B. tert-Butoxycarbonyl, Benzyloxycarbonyl, 4-Nitrobenzyloxycarbonyl oder Diphenylmethoxycarbonyl.

Eine Aminogruppe kann z.B. in Form einer Acylamino-, Arylmethylamino-, veretherten Mercaptoamino- oder Silylaminogruppe oder als Azidogruppe geschützt sein.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Niederalkancarbonsäure oder gegebenenfalls, z.B. durch Halogen, Niederalkoxy oder Nitro, substituierten Benzoesäure, oder bevorzugt eines Kohlensäurehalbesters. Solche Acylgruppen sind beispielsweise Niederalkanoyl, wie Formyl, Acetyl, Propionyl oder Pivaloyl, Halogenniederalkanoyl, z.B. 2-Halogenacetyl, wie 2-Chlor-, 2-Brom-, 2-Iod-, 2,2,2-Trifluor- oder 2,2,2-Trichloracetyl, gegebenenfalls z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, z.B. tert-Niederalkoxycarbonyl, wie tert-Butoxycarbonyl, Arylmethoxycarbonyl mit einem oder zwei Arylresten, die gegebenenfalls, z.B. durch Niederalkyl, z.B. tert-Niederalkyl, wie tert-Butyl, Niederalkoxy, wie Methoxy, Hydroxy, Halogen, wie Chlor, und/oder Nitro, mono- oder polysubstituiertes Phenyl darstellen, z.B. Benzyloxycarbonyl, 4-Nitrobenzyloxycarbonyl, Diphenylmethoxycarbonyl oder Di-(4-methoxyphenyl)-methoxycarbonyl, Aroylmethoxycarbonyl, z.B. Phenacyloxycarbonyl, 2-Halogen-niederalkoxycarbonyl, z.B. 2-Chlorethoxycarbonyl, 2,2,2-Trichlorethoxycarbonyl, 2-Bromethoxycarbonyl oder 2-Iodethoxycarbonyl, 2-Triniederalkylsilylniederalkoxycarbonyl, z.B. 2-Trimethylsilylethoxycarbonyl, oder 2-Triarylsilylniederalkoxycarbonyl, z.B. 2-Triphenylsilylethoxycarbonyl.

Eine Arylmethylaminogruppe ist z.B. Mono-, Di- oder insbesondere Triphenylmethylamino, z.B. Benzyl-, Diphenylmethyl- oder Tritylamino.

In einer veretherten Mercaptoaminogruppe ist die veretherte Mercaptogruppe in erster Linie substituiertes Arylthio, z.B. 4- Nitrophenylthio.

Eine Silylaminogruppe ist beispielsweise eine Triniederalkylsilylaminogruppe, z.B. Trimethylsilylamino. Das Siliciumatom der Silylaminogruppe kann auch nur durch zwei Niederalkylgruppen, z.B. Methylgruppen, und die Aminogruppe oder Carboxygruppe eines zweiten Moleküls der Formel I substituiert sein. Verbindungen mit solchen Schutzgruppen lassen sich z.B. mit Dimethylchlorsilan als Silylierungsmittel herstellen.

Bevorzugte Aminoschutzgruppen sind Acylreste von Kohlensäurehalbestern, insbesondere tert-Butoxycarbonyl, gegebenenfalls substituiertes Benzyloxycarbonyl, z.B. 4-Nitrobenzyloxycarbonyl, Diphenylmethoxycarbonyl, 2-Halogen-niederalkoxycarbonyl, z.B. 2,2,2-Trichlorethoxycarbonyl, ferner Trityl und Formyl.

Eine Hydroxygruppe kann beispielsweise durch eine durch Halogen, z.B. Chlor, substituierte Niederalkanoylgruppe, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2-Chlorethoxycarbonyl, 2,2,2-Trichlorethoxycarbonyl, 4-Nitrobenzyloxycarbonyl oder Diphenylmethoxycarbonyl. Eine Hydroxygruppe kann ferner durch Triniederalkylsilyl, z.B. Trimethylsilyl oder bevorzugt Dimethyl-tert-butylsilyl, eine leicht abspaltbare Alkylgruppe, wie tert-Niederalkyl, z.B. tert-Butyl, einen oxa- oder einen thiaaliphatischen oder -cycloaliphatischen Kohlenwasserstoffrest, beispielsweise 1-Niederalkoxyniederalkyl oder 1-Niederalkylthioniederalkyl, z.B. Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5 - 7 Ringatomen, z.B. 2-Tetrahydrofuryl oder 2-Tetrahydropyranyl, oder ein entsprechendes Thiaanaloges, sowie durch 1-Phenylniederalkyl, z.B. Benzyl, Diphenylmethyl oder Trityl, wobei die Phenylreste beispielsweise durch Halogen, z.B. Chlor, Niederalkoxy, z.B. Methoxy, und/oder Nitro substituiert sein können, geschützt sein.

Zwei benachbarte Hydroxylgruppen können beispielsweise durch eine vorzugsweise substituierte Methylengruppe geschützt sein, z.B. durch Niederalkyliden, z.B. Isopropyliden, Cycloalkyliden, z.B. Cyclohexyliden, oder Benzyliden.

Eine Mercaptogruppe, wie z.B. in Cystein, kann insbesondere durch S-Alkylierung mit gegebenenfalls substituierten Alkylresten, Silylierung, Thioacetalbildung, S-Acylierung oder durch die Bildung asymmetrischer Disulfid-Gruppierungen geschützt sein. Bevorzugte Mercaptoschutzgruppen sind z.B. gegebenenfalls im Phenylrest, z.B. durch Methoxy oder Nitro, substituiertes Benzyl, wie 4-Methoxybenzyl, gegebenenfalls am Phenyl rest, z.B. durch Methoxy, substituiertes Diphenylmethyl, wie Di-(4-methoxyphenyl)-methyl, Triphenylmethyl, Trimethylsilyl, Benzylthiomethyl, 2-Tetrahydropyranyl, Acylaminomethyl, Benzoyl, Benzyloxycarbonyl oder Niederalkylaminocarbonyl, wie Ethylaminocarbonyl, ferner Niederalkylthio, z.B. Methylthio.

Eine Sulfogruppe kann beispielsweise durch Niederalkyl, z.B. Methyl oder Ethyl, durch Phenyl oder als Sulfonamid, beispielsweise als Imidazolid, geschützt sein.

Die Kondensation zur Herstellung der Amidbindung kann in an sich bekannter Weise durchgeführt werden, beispielsweise wie in Standardwerken, wie "Houben-Weyl, Methoden der organischen Chemie", 4. Auflage, Band 15/II, Georg Thieme Verlag, Stuttgart 1974, "The Peptides" (Herausg. E. Gross und J. Meienhofer), Band 1 und 2, Academic Press, London und New York, 1979/1980, oder M. Bodanszky, "Principles of Peptide Synthesis", Springer-Verlag, Berlin 1984, beschrieben.

Die Kondensation kann in Gegenwart eines der üblichen Kondensationsmittel durchgeführt werden. Uebliche Kondensationsmittel sind z.B. Carbodiimide, beispielsweise Diethyl-, Dipropyl-, N-Ethyl-N′-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Ethyl-5-phenyl-1,2-oxazolium-3′-sulfonat und 2-tert-Butyl-5-methylisoxazoliumperchlorat, oder eine geeignete Acylaminoverbindung, z.B. 2-Ethoxy-1-ethoxycarbonyl-1,2-dihydrochinolin, ferner aktivierte Phosphorsäurederivate, z.B. Diphenylphosphorylazid, Diethylphosphorylcyanid, Phenyl-N-phenylphosphoramidochloridat, Bis-(2-oxo-3-oxazolidinyl)-phosphinsäurechlorid oder 1-Benzotriazolyloxy-tris-(dimethylamino)-phosphonium-hexafluorophosphat.

Gewünschtenfalls wird eine organische Base zugegeben, z.B. ein Triniederalkylamin mit voluminösen Resten, z.B. Ethyldiisopropylamin, oder eine heterocyclische Base, z.B. Pyridin, 4-Dimethylaminopyridin oder bevorzugt N-Methylmorpholin.

Die Kondensation von Säureanhydriden mit Aminen kann z.B. in Gegenwart von anorganischen Carbonaten, z.B. Alkalimetallcarbonaten oder -hydrogencarbonaten, wie Natrium- oder Kaliumcarbonat oder -hydrogencarbonat (üblicherweise zusammen mit einem Sulfat), erfolgen.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, cyclischen Ether, z.B. Tetrahydrofuran, einem Ester, z.B. Essigsäureethylester, oder einem Nitril, z.B. Acetonitril, oder in Mischungen davon, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre.

Reaktionsfähige Säurederivate können auch in situ gebildet werden. So kann man z.B. N,N′-disubstituierte Amidinoester in situ bilden, indem man das Gemisch des Fragments mit freier Carboxygruppe und des komplementären Fragments mit einer Aminogruppe in Gegenwart eines geeigneten disubstituierten Carbodiimids, z.B. Dicyclohexylcarbodiimid, umsetzt. Ferner kann man Amino- oder Amidoester von solchen Säuren in Gegenwart der zu acylierenden Aminokomponente bilden, indem man das Gemisch der entsprechenden Säure- und Amino-Ausgangsstoffe in Gegenwart eines disubstituierten Carbodiimids, z.B. Dicyclohexylcarbodiimid, und eines N-Hydroxylamins oder N-Hydroxyamids, z.B. N-Hydroxybenztriazol, N-Hydroxysuccinimid oder N-Hydroxy-norbornan-2,3-dicarbonsäureimid, gegebenenfalls in Anwesenheit einer geeigneten Base, z.B. 4-Dimethylaminopyridin, N-Methylmorpholin oder Ethyldiisopropylamin, umsetzt.

Die Kondensation einer Carbonsäure mit dem entsprechenden, zur Verbindung der Formel I komplementären Fragment mit einer freien Aminogruppe kann auch auf an sich bekannte Weise mit Hilfe von Enzymen erreicht werden, z.B. wie von H.-D. Jakubke et al. in Angewandte Chemie 97, 79 (1985) beschrieben. Als Enzyme sind beispielsweise Thermolysin, Carboxypepti dase Y, Papain, Chymotrypsin, Trypsin oder Pepsin geeignet. Die Reaktion wird vorzugsweise in Wasser oder in Gemischen von Wasser mit organischen Lösungsmitteln, z.B. mit Niederalkanolen, wie Ethanol, Dimethylformamid, Dimethylsulfoxid, Ethern, wie Tetrahydrofuran, Dioxan oder 1,2-Dimethoxyethan, Aceton, Acetonitril oder Polyalkoholen, z.B. Ethylenglykol, Di-, Tri- oder Poly-ethylenglykol, aber auch mit nicht mischbaren organischen Lösungsmitteln, z.B. Methylenchlorid oder Essigsäureethylester, bei einem pH von 5 bis 8, bevorzugt um den Neutralpunkt, bei Temperaturen zwischen 0°C und 50°C durchgeführt. Die Lösungsmittel und die Reaktionsbedingungen werden vorzugsweise so gewählt, dass die gewünschte Verbindung ausfällt oder in die nicht-mischbare organische Phase extrahiert wird und so dem Reaktionsgleichgewicht entzogen wird. Es ist auch möglich, die Kondensation mit auf einem geeigneten Träger immobilisierten Enzymen, wie sie oben

genannt sind, in den genannten organischen Lösungsmitteln im Gemisch mit wenig Wasser durchzuführen.

Das Verfahren a) kann auf an sich bekannte Weise auch in automatisierter Form durchgeführt werden, z.B. gemäss der als Festphasen-synthese bekannten Technik, die auf R. Merrifield zurückgeht und beispielsweise in Angew. Chem. 97, 801-812 (1985), Naturwissenschaften 71, 252-258 (1984) oder in R.A. Houghten, Proc. Natl. Acad. Sci., 82, 5131-5135 (1985) beschrieben ist.

Die Fragmente der Verbindung der Formel I, die gemäss Verfahren a) miteinander kondensiert werden, können auch als Racemate oder als Diastereomerengemische eingesetzt werden. Dabei entstehen Diastereomerengemische, die Verbindungen der Formel I enthalten und noch einer Trennung der Diastereomeren gemäss Verfahren i) unterworfen werden müssen.

Verfahren b) (Reduktion einer Ketogruppe):

In einem Ausgangsmaterial der Formel V sind funktionelle Gruppen mit Ausnahme der zu reduzierenden Ketogruppe gegebenenfalls durch eine der unter Verfahren a) genannten Schutzgruppen geschützt.

Zur Reduktion der Ketogruppe in einer Verbindung der Formel V eignen sich solche Reduktionsmittel, die unter den Reaktionsbedingungen des Verfahrens eine isolierte Ketogruppe selektiv oder schneller als die in Verbindungen der Formel I vorhandenen Amidgruppen reduzieren.

In erster Linie zu nennen sind geeignete Borhydride, wie Alkalimetallborhydride, insbesondere Natriumborhydrid, Lithiumborhydrid oder Natriumcyanborhydrid, ferner Zinkborhydrid, oder geeignete Aluminiumhydride, wie Alkalimetallniederalkoxyaluminiumhydride mit voluminösen Resten, z.B. Lithium-tris-tert-butoxyaluminiumhydrid.

Die Reduktion kann auch mit Wasserstoff in Gegenwart geeigneter Schwermetallkatalysatoren, z.B. Raney-Nickel oder Platin- oder Palladiumkatalysatoren, z.B. Platin- oder Palladium-Aktivkohle, oder nach Meerwein-Ponndorf-Verley mit Hilfe von Aluminiumalkanolaten, bevorzugt Aluminium-2-propanolat oder -ethanolat, durchgeführt werden.

Die Reduktion kann vorzugsweise mit stöchiometrischen Mengen oder einem sinnvoll bemessenen Ueberschuss des Reduktionsmittels in einem inerten Lösungsmittel bei Temperaturen zwischen -80 °C und dem Siedepunkt des Lösungsmittels, vorzugsweise zwischen -20 °C und +100 °C, wenn nötig unter Schutzgas, z.B. Stickstoff oder Argon, durchgeführt werden. Ein Ueberschuss des Reduktionsmittels ist insbesondere dann nötig, wenn dieses auch mit dem Lösungsmittel, z.B. den Protonen eines protischen Lösungsmittels, reagiert.

Bei Verwendung von Natriumborhydrid eignen sich polare, protische Lösungsmittel, z.B. Methanol, Ethanol oder Isopropanol; bei Verwendung der anderen Reduktionsmittel die unter Verfahren a) genannten polaren, aprotischen Lösungsmittel, z.B. Tetrahydrofuran.

Verfahren c) (Addition einer metallorganischen Verbindung):

In einem Ausgangsmaterial der Formel VI sind funktionelle Gruppen mit Ausnahme der Aldehydgruppe gegebenenfalls durch die unter Verfahren a) genannten Schutzgruppen geschützt. Ebenso sind vorhandene funktionelle Gruppen in einer Verbindung der Formel VII geschützt.

In einer Verbindung der Formel VII ist ein Metallradikal -M beispielsweise -Li oder -MgHal, z.B. -MgCl, -MgBr oder -MgI.

Die Umsetzung einer Verbindung der Formel VI mit einer Verbindung der Formel VII erfolgt in üblicher Weise in einem wasserfreien, inerten, aprotischen Lösungsmittel, z.B. in einem Ether, wie Diethylether oder Tetrahydrofuran, oder einem Kohlenwasserstoff, wie Benzol oder Toluol, oder Gemischen davon, gegebenenfalls unter Kühlen, insbesondere nach Beginn der Reaktion, z.B. bis etwa -30 °C, oder unter Erwärmen, z.B. bis zur Siedetemperatur des Reaktionsgemisches, gegebenenfalls in einer Inertgas-, z.B. Stickstoffatmosphäre. Eine bevorzugte Ausführungsform des Verfahrens ist die Umsetzung des Aldehyds der Formel VI mit einem Ueberschuss der Lithiumverbindung der Formel VII.

Die Hydrolyse des Additionsprodukts erfolgt mit $H^+$-Ionen liefernden Lösungsmitteln, z.B. Wasser (Eis-Wasser-Gemisch) oder verdünnten, wässrigen Säuren, z.B. verdünnten Mineralsäuren, wie verdünnter, wässriger Schwefelsäure, oder verdünnten organischen Säuren, z.B. verdünnter, wässriger Essigsäure.

Die Umsetzung einer Verbindung der Formel VI kann auch mit einer in situ hergestellten Verbindung der Formel VII erfolgen, welche man z.B. aus dem entsprechenden Halogenid, z.B. Chlorid, durch Umsetzung mit einem Metallierungsmittel, z.B. Magnesium, Lithium oder tert-Butyllithium, erhält.

Verfahren d) (nukleophile Substitution):

In einem Ausgangsmaterial der Formel VIII sind funktionelle Gruppen gegebenenfalls durch die unter Verfahren a) genannten Schutzgruppen geschützt.

In einer Verbindung der Formel VIII ist die nukleofuge Abgangsgruppe X insbesondere mit einer starken anorganischen oder organischen Säure verestertes Hydroxy, wie mit einer Mineralsäure, z.B. Halogenwasserstoffsäure, wie Chlor-, Brom- oder Iodwasserstoffsäure, Schwefelsäure oder Halogenschwefelsäure, z.B. Fluorschwefelsäure, mit einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom und/oder Nitro substituierten Benzolsulfonsäure, z.B. einer Methansulfon-, Trifluormethansulfon- oder p-Toluolsulfonsäure, oder mit Stickstoffwasserstoffsäure verestertes Hydroxy.

Ein eine Hydroxygruppe einführendes Reagens ist z.B. eine Hydroxidhaltige Base, z.B. Natrium- oder Kaliumhydroxid.

Vorzugsweise werden die Reaktionsbedingungen so gewählt, dass die Reaktion im wesentlichen als nukleophile Substitution zweiter Ordnung ($S_N2$) abläuft. Die Reaktion mit einer Hydroxid-haltigen Base wird vorzugsweise in Wasser, dem gegebenenfalls als Lösungsvermittler ein organisches Lösungsmittel, z.B. Ethanol, Tetrahydrofuran oder Aceton, beigemischt ist, durchgeführt. Die Substitutionsreaktion wird gegebenenfalls bei erniedrigter oder erhöhter Temperatur, z.B. in einem Temperaturbereich von etwa -40 °C bis etwa +100 °C, bevorzugt von etwa -10 °C bis etwa +50 °C, und gegebenenfalls unter Inertgas-, z.B. Stickstoffatmosphäre, durchgeführt.

Verfahren e) (Ueberführung einer Cyanogruppe in eine Gruppe $COR^1$):

In einem Ausgangsmaterial der Formel IX sind funktionelle Gruppen gegebenenfalls durch die unter a) genannten Schutzgruppen geschützt. Die Ueberführung einer Verbindung der Formel IX in eine Verbindung der Formel I kann z.B. durch eine Ritter-Reaktion oder über Carbonsäureesterimid-Salze erfolgen.

Bei der Ritter-Reaktion setzt man die Nitrile in Gegenwart einer starken Säure, beispielsweise 85-90%iger Schwefelsäure, oder auch Polyphosphorsäure, Fluorwasserstoff, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen, wie Propylen, oder Alkoholen, wie Benzylalkohol, meist ohne Lösungsmittel oder beispielsweise in Eisessig.

In einer Variante der Ritter-Reaktion wird ein Nitril der Formel IX mit einem Olefin und Quecksilber(II)-nitrat umgesetzt und die Organoquecksilberverbindung anschliessend mit Natriumborhydrid zu einer N-substituierten Verbindung der Formel I reduziert.

Durch säurekatalysierte, bevorzugt Chlorwasserstoff-katalysierte, Anlagerung von Alkoholen an die Nitrile der Formel IX erhält man Carbonsäureesterimide, die durch thermische Umlagerung bei Temperaturen oberhalb von etwa 80 °C Amide der Formel I ergeben.

Für die Umwandlung eines Nitrils in eine freie Säure wird die Hydrolyse mit Wasser vorteilhaft in einem inerten organischen Lösungsmittel, das zumindest teilweise mit Wasser mischbar ist, wie Ether (z.B. Diethyl- und Diisopropylether, 1,2-Dimethoxyethan oder insbesondere Dioxan oder Tetrahydrofuran), oder Niederalkanolen (z.B. Methanol, Ethanol, Isopropylalkohol, Butylalkohole, insbesondere tert-Butylalkohol) durchgeführt, wobei eine grössere Menge Wasser in den letztgenannten Fällen erforderlich ist, um eine Alkoholyse zu verhindern. Die Hydrolyse kann sowohl durch starke Säuren, insbesondere anorganische Säuren, wie Schwefelsäure, oder vorzugsweise Halogenwasserstoffsäuren (z.B. Bromwasserstoffsäure oder als erste Wahl Chlorwasserstoffsäure), als auch durch Basen, insbesondere anorganische Basen, wie Hydroxide und Carbonate der Alkalimetalle, z.B. Natrium- und Kaliumhydroxid, katalysiert werden.

Die Basen werden gewöhnlich in zumindest stöchiometrischen Anteilen verwendet, die Carbonsäuresalze als primäre Produkte entstehen lassen. Die sauren Katalysatoren werden vorteilhaft in Form einer verdünnten wässrigen Lösung zur Erzielung des besten Resultats angewandt.

Endprodukte der Formel I, worin $R^1$ verethertes Hydroxy bedeutet, können erhalten werden, indem man die Solvolyse des Nitrils mit dem entsprechenden Alkohol (Alkoholyse) in Gegenwart einer katalytischen Menge einer wasserfreien starken Säure, vorteilhaft von gasförmigen Chlorwasserstoff, durchführt. Gewöhnlich wird ein Ueberschuss an Alkohol als Lösungsmittel verwendet, jedoch können inerte organische Lösungsmittel, wie acyclische und cyclische Ether (insbesondere die vorstehend erwähnten), und/oder halogenierte Niederalkane (insbesondere Chloroform und Dichlormethan) zugegeben werden. Wird die Alkoholyse unter streng wasserfreien Bedingungen durchgeführt, muss das primäre Produkt (Imidoester)

vorteilhaft durch Zugabe von Wasser zu der Reaktionsmischung hydrolysiert werden. Andererseits wird, wenn man die Alkoholyse in Gegenwart eines annähernd stöchiometrischen Aequivalents von Wasser durchführt, der gewünschte Ester direkt erhalten.

Verfahren f) (Reduktion des Epoxids):

In einem Ausgangsmaterial der Formel X sind funktionelle Gruppen gegebenenfalls durch die unter Verfahren a) genannten Schutzgruppen geschützt.

Es können solche Reduktionsmittel verwendet werden, die unter den Reaktionsbedingungen des Verfahrens die Epoxygruppe selektiv oder schneller als die vorhandenen Amidgruppen reduzieren und das Epoxid so öffnen, dass ein genügend und möglichst grosser Anteil der Reaktionsprodukte die neugebildete Hydroxygruppe in der der Formel I entsprechenden Position trägt. Beispiele für solche selektiven Reduktionsmittel sind Lithiumborhydrid oder Natriumcyanborhydrid/Bortrifluoridetherat. Mit dem letztgenannten Reagens lässt sich die Reaktion z.B. so durchführen, dass man zu 1 Mol der Verbindung der Formel X und einem Ueberschuss, z.B. 1,4-3 Mol, Natriumcyanborhydrid in Tetrahydrofuran bei erhöhter Temperatur, z.B. unter Rückfluss, eine Lösung von Bortri fluoridetherat, $BF_3 \cdot O(C_2H_5)_2$, in Tetrahydrofuran so zugibt, dass der pH-Wert der Reaktionslösung in der Nähe des Umschlagpunktes des ebenfalls zugegebenen Indikators Bromkresolgrün gehalten wird. Die Reduktion mit Lithiumborhydrid wird vorzugsweise in einem Ether, z.B. Tetrahydrofuran, 1,2-Dimethoxyethan oder Diethylenglykoldimethylether, bei Temperaturen zwischen Raumtemperatur und Rückflusstemperatur durchgeführt.

Verfahren g) (Addition an ein Acrylamid)

Eine Verbindung der Formel $R^a$-$S(O)_mH$ ist entweder ein Thiol der Formel $R^a$-SH oder eine Sulfinsäure der Formel $R^a$-$SO_2H$.

In einem Ausgangsmaterial der Formel XI sind funktionelle Gruppen gegebenenfalls durch die unter Verfahren a) genannten Schutzgruppen geschützt. Ebenso sind vorhandene funktionelle Gruppen in der Verbindung der Formel $R^a$-$S(O)_mH$ gegebenenfalls geschützt.

Geeignete Salze der Verbindung der Formel $R^a$-$S(O)_mH$ sind beispielsweise Alkalimetallsalze, z.B. Natrium- oder Kaliumsalze.

Die Addition einer Verbindung der Formel $R^a$-$S(O)_mH$ oder eines geeigneten Salzes davon an eine Verbindung der Formel XI erfolgt in üblicher Weise in einem inerten, polaren Lösungsmittel, z.B. in einem polaren Ether, z.B. Tetrahydrofuran, Dioxan oder Dimethoxyethan, einem Niederalkanol, z.B. Methanol, Ethanol oder Isopropanol, oder einem dipolar aprotischen Lösungsmittel, z.B. Dimethylformamid, Dimethylacetamid, Dimethylsulfoxid, Hexamethylphosphorsäuretriamid, N-Methylpyrrolidon oder Acetonitril, gegebenenfalls auch in Gemischen der genannten Lösungsmittel untereinander oder mit Wasser, bei Temperaturen zwischen ca. -30° C und dem Siedepunkt des jeweiligen Lösungsmittel, z.B. zwischen 0° und +80° C, beispielsweise um 50° C.

Anstelle einer Sulfinsäure $R^a$-$SO_2H$ werden vorzugsweise deren Salze eingesetzt, beispielsweise das Natrium- oder Kaliumsalz.

Ein Salz eines Thiols der Formel $R^a$-SH kann auch in situ gebildet werden, beispielsweise durch Zugabe einer geeigneten Base, z.B. Alkalimetallhydroxid, wie Natrium- oder Kaliumhydroxid, oder Alkalimetallhydrid, z.B. Natriumhydrid, wobei allerdings nur wasserfreie Lösungsmittel verwendet werden können. Es ist auch möglich, die Additionsreaktion mit einem freien Thiol der Formel $R^a$-SH in Gegenwart einer organischen Base, z.B. eines tertiären Amins, z.B. Triethylamin, N-Methylmorpholin, Dimethylanilin, Diazabicyclo[5.4.0]undec-7-en oder Diazabicyclo[4.3.0]-non-5-en, zu führen.

Verfahren h) (Alkylierung im Acylrest der Teilformel Ia)

In einem Ausgangsmaterial der Formel XII sind funktionelle Gruppen gegebenenfalls durch die unter Verfahren a) genannten Schutzgruppen geschützt.

Eine den Rest $R_b$-$(CH_2)q$- einführende Verbindung ist beispielsweise das entsprechende Halogenid, z.B. Chlorid, Bromid oder Iodid, oder ein reaktiver Ester des entsprechenden Alkohols, z.B. ein Sulfonsäureester, wie der Methansulfonsäureester oder p-Toluolsulfonsäureester.

Zur Alkylierung wird die Verbindung der Formel XII vorzugsweise nach den üblichen Methoden mit

einer starken, nicht-nukleophilen Base in das entsprechende Anion übergeführt, beispielsweise mit dem Lithium- oder Kaliumsalz eines sterisch gehinderten sekundären Amins, z.B. mit Lithiumdiisopropylamid, Lithiumcyclohexylisopropylamid, Lithium-2,2,6,6-tetramethylpiperidid, Lithium- oder Kalium-bis-(trimethylsilyl)-amid oder dergleichen. Die Deprotonierung mit der Base wird vorzugsweise bei tiefen Temperaturen, z.B. zwischen -100°C und -50°C in einem inerten polaren Lösungsmittel, z.B. in einem polaren Ether, z.B. Tetrahydrofuran, Dioxan oder Dimethoxyethan, gegebenenfalls gemischt mit einem Kohlenwasserstoff, z.B. Hexan oder Toluol, und/oder Hexamethylphosphorsäuretriamid oder N,N′-Dimethyl-N,N′-propylenharnstoff, durchgeführt. Enthält die Verbindung der Formel XII noch weitere, leichter deprotonierbare Methylen- oder Methingruppen, z.B. solche neben der Sulfinyl- oder Sulfonylgruppe, so werden vorzugsweise zwei oder mehr Aequivalente der Base eingesetzt, um das entsprechende Di- oder Polyanion zu erhalten.

Die dermassen deprotonierte Verbindung der Formel XII wird mit dem den Rest $R^b$-$(CH_2)q$- einführenden Alkylierungsmittel bevorzugt in situ bei tiefen Temperaturen, z.B. bei -78° bis -30°C, und nachfolgendem Aufwärmen auf Raumtemperatur oder leicht erhöhter Temperatur, z.B. auf 50°C, im gleichen Lösungsmittel oder Lösungsmittelgemisch umgesetzt.

Ist in einer Verbindung der Formel XII n = 0, so kann die Alkylierung unter wesentlich milderen Bedingungen durchgeführt werden, beispielsweise in einem der oben genannten Lösungsmittel oder anderen polaren Lösungsmitteln, z.B. Dimethylsulfoxid, Dimethylformamid oder Acetonitril, mit dem den Rest $R^b$-$(CH_2)q$- einführenden Alkylierungsmittel bei Temperaturen zwischen -30°C und etwa Raumtemperatur und einem tertiären Amin, z.B. Triethylamin, N-Methylmorpholin, Diazabicyclo[5.4.0]undec-7-en oder Diazabicyclo[4.3.0]non-5-en, oder einer unlöslichen anorganischen Base, z.B. Kaliumcarbonat oder Natriumhydrid, oder einem Alkoholat, z.B. Kalium-tert-butanolat. Geeignet ist auch die Alkylierung unter Phasentransfer-Bedingungen, d.h. in einem zweiphasigen Gemisch aus wässriger Base, z.B. Natronlauge, und einem nicht mischbaren organischen Lösungsmittel, z.B. Methylenchlorid oder Toluol, und einem Phasentransfer-Katalysator, z.B. einem Ammonium- oder Phosphoniumsalz.

Verfahren i) (Nachoperationen):

Die Gruppe $R^1$ bildet zusammen mit der terminalen Carbonylgruppe des Radikals AAC der Verbindungen der Formel I eine Carboxygruppe, eine veresterte Carboxygruppe, eine Carboxamidgruppe oder eine substituierte Carboxamidgruppe. Wenn im Zusammenhang mit Nachoperationen derartige Gruppen genannt werden, sind damit neben entsprechenden Gruppen, die als Substituenten vorhanden sein können, z.B. in den Aminosäureresten A1, A2, A3, A4 und A5, auch jeweils die C-terminalen Gruppen umfasst, die durch $R^1$ und die daran unmittelbar gebundene Carbonylgruppe gebildet werden.

In einer erhältlichen Verbindung der Formel I kann man eine Carboxamidgruppe substituieren, eine in freier oder in reaktionsfähiger Form vorliegende Carboxygruppe verestern bzw. eine veresterte Carboxygruppe in eine freie Carboxy- oder eine Carboxamidgruppe überführen.

Die Substitution einer Carboxamidgruppe oder einer anderen Aminogruppe erfolgt z.B. durch Alkylierung.

Geeignete Mittel zur Alkylierung einer Carboxamidgruppe in einer Verbindung der Formel I sind z.B. Diazoverbindungen, z.B. Diazomethan. Man kann Diazomethan in einem inerten Lösungsmittel zersetzen, wobei das gebildete freie Methylen mit der Carboxamidgruppe in der Verbindung der Formel I reagiert. Die Zersetzung von Diazomethan erfolgt vorzugsweise katalytisch, z.B. in Gegenwart eines Edelmetalls in fein verteilter Form, z.B. Kupfer, oder eines Edelmetallsalzes, z.B. Kupfer(I)-chlorid oder Kupfer(II)-sulfat.

Weitere Alkylierungsmittel sind die in der Deutschen Offenlegungsschrift 2 331 133 genannten Alkylierungsmittel, z.B. Alkylhalogenide, Sulfonsäureester, Meerweinsalze oder 1-substituierte 3-Aryltriazene, welche man unter den dort genannten Reaktionsbedingungen mit einer Verbindung der Formel I mit einer Carboxamidgruppe umsetzen kann.

Zur Veresterung einer Carboxygruppe in einer Verbindung der Formel I kann man die freie Säure verwenden oder die freie Säure in eines der unter Verfahren a) genannten reaktionsfähigen Derivate überführen und mit einem Alkohol umsetzen, oder man kann die freie Säure oder ein reaktionsfähiges Salz, z.B. das Cäsiumsalz, mit einem reaktionsfähigen Derivat eines Alkohol umsetzen. Beispielsweise kann man das Cäsiumsalz einer Carbonsäure mit dem dem Alkohol entsprechenden Halogenid umsetzen.

Die Veresterung einer Carboxygruppe kann mit den für die Substitution der Carboxamidgruppe oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

Zur Ueberführung einer veresterten Carboxygruppe in einer Verbindung der Formel I in eine freie

Carboxygruppe kann eine der unter Verfahren a), Abspaltung der Carboxyschutzgruppen, beschriebenen Methoden oder gewünschtenfalls eine alkalische Verseifung nach den im Organikum, 15. Auflage, VEB Deutscher Verlag der Wissenschaften, Berlin (Ost) 1976, genannten Reaktionsbedingungen angewendet werden.

In einer Verbindung der Formel I kann man eine veresterte Carboxygruppe durch Aminolyse mit Ammoniak oder einem primären oder sekundären Amin in eine gegebenenfalls substituierte Carboxamidgruppe überführen. Die Aminolyse kann nach den im Organikum, 15. Auflage, VEB Deutscher Verlag der Wissenschaften, Berlin (Ost) 1976, für solche Umsetzungen genannten Reaktionsbedingungen erfolgen.

In einer erhältlichen Verbindung der Formel I, worin die Substituenten die genannten Bedeutungen haben und mindestens eine freie Hydroxygruppe vorhanden ist und die übrigen funktionellen Gruppen gegebenenfalls in geschützter Form vorliegen, kann man die freie Hydroxygruppe verethern oder verestern.

Die Veretherung dieser Hydroxygruppe kann mit den oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

Die Veresterung der freien Hydroxygruppe kann mit den üblichen Acylierungsmitteln und den üblichen im Organikum angegebenen Reaktionsbedingungen erfolgen, z.B. mit Essigsäureanhydrid.

Die genannten Alkylierungsreaktionen, Veretherungen, Veresterungen etc. können statt im Endstoff auch in einem Ausgangsmaterial entsprechend durchgeführt werden.

In einer erhältlichen Verbindung der Formel I kann man eine Thiogruppe zu einer Sulfinyl- oder Sulfonylgruppe oder eine Sulfinylgruppe zu einer Sulfonylgruppe oxidieren.

Die Oxidation zur Sulfonylgruppe kann mit den meisten der üblichen Oxidationsmittel durchgeführt werden. Bevorzugt verwendet man solche Oxidationsmittel, die die Thiogruppe oder Sulfinylgruppe selektiv in Gegenwart anderer funktioneller Gruppen der Verbindung der Formel I, z.B. der Amidfunktion und der Hydroxygruppe, oxidieren, beispielswiese aromatische oder aliphatische Peroxycarbonsäuren, z.B. Perbenzoesäure, Monoperphthalsäure, m-Chlorperbenzoesäure, Peressigsäure, Perameisensäure oder Trifluorperessigsäure. Die Oxidation mit Peroxycarbonsäure erfolgt in den üblichen dafür geeigneten Lösungsmitteln, beispielsweise Chlorkohlenwasserstoffen, z.B. Methylenchlorid oder Chloroform, Ether, Essigester oder dergleichen, bei Temperaturen zwischen -78° C und Raumtemperatur, z.B. zwischen -20° C und +10° C, bevorzugt um 0° C. Die Peroxycarbonsäure kann auch in situ gebildet werden, z.B. mit Wasserstoffperoxid in Essigsäure oder Ameisensäure, die gegebenenfalls Essigsäureanhydrid enthält, z.B. mit 30 % oder 90 % Wasserstoffperoxid in Essigsäure/Essigsäureanhydrid. Geeignet sind auch andere Peroxoverbindungen, beispielsweise Kaliumperoxomonosulfat in Niederalkanol/Wasser-Mischungen, z.B. Methanol-Wasser oder Ethanol-Wasser, oder in wässriger Essigsäure bei Temperaturen zwischen -70° C und +30° C, z.B. zwischen -20° C und Raumtemperatur, ferner Natriummetaperiodat in Methanol oder Methanol-Wasser-Gemischen bei Temperaturen zwischen 0° C und 50° C, z.B. um Raumtemperatur.

Für die Oxidation der Thiogruppe zur Sulfinylgruppe werden selektive Oxidationsmittel in äquimolaren Mengen oder nur geringem Ueberschuss unter kontrollierten Reaktionsbedingungen verwendet, um eine Ueberoxidation zur Sulfonylgruppe zu vermeiden. Geeignet sind beispielsweise Natriummetaperiodat in Methanol oder Methanol-Wasser-Gemischen bei Temperaturen zwischen -15° C und Raumtemperatur, z.B. um 0° C, m-Chlorperbenzoesäure in Methylenchlorid, Chloroform oder Essigester bei Temperaturen zwischen -78° C und 10° C, bevorzugt zwischen -30° C und 0° C, ferner tert-Butylhypochlorit in Niederalkanolen, z.B. Methanol, oder Wasserstoffperoxid in Aceton oder Essigsäure bei Temperaturen um 0° C, oder das oben genannte Kaliumperoxomonosulfat bei tiefen Temperaturen.

In einer erhältlichen Verbindung der Formel I mit einer Sulfinylgruppe kann man diese Gruppe zu einer Thiogruppe reduzieren. Bevorzugt sind selektive Reduktionsmittel, die andere funktionelle Gruppe der Verbindung der Formel I, z.B. die Amidfunktion, unverändert lassen. Beispiele für solche selektiven Reduktionsmittel sind Dichlorboran, das vorzugsweise in Tetrahydrofuran oder Dimethoxyethan bei Temperaturen zwischen -30° C und +10° C eingesetzt wird, Triphenylphosphin in siedendem Tetrachlorkohlenstoff, Trichlorsilan oder Hexachlordisilan, Eisenpentacarbonyl, ferner Natriumhydrogensulfit in wässrigalkoholischen Lösungsmitteln, z.B. Wasser-Methanol, Wasser-Ethanol oder auch Wasser-Tetrahydrofuran, bei Temperaturen zwischen -10° C und +50° C, ferner Natriumborhydrid in Gegenwart von Kobalt(II)chlorid oder auch Wasserstoff in Gegenwart von katalytischen Mengen Palladium, z.B. Palladium/Kohle in siedendem Ethanol.

Gewünschtenfalls kann in einer erhältlichen Verbindung der Formel I eine Sulfonylgruppe zu einer Thiogruppe reduziert werden, beispielsweise mit Diisobutylaluminiumhydrid in Ether oder Tetrahydrofuran.

In einer erhältlichen Verbindung der Formel I mit einer Sulfonamidgruppe kann diese in der für Carboxamidgruppen beschriebenen Art und Weise alkyliert oder mit Säure oder Alkali zu einer Sulfogruppe hydrolysiert werden. Eine Sulfenamidgruppe kann mit einem der unter der Oxidation von Thiogruppen zu

Sulfonylgruppen genannten Reagentien, z.B. Kaliumperoxomonosulfat, zum Sulfonamid oxidiert und gleich in situ hydrolysiert werden. Eine Sulfonsäureestergruppe kann ebenfalls durch Säure oder Base, beispielsweise wie oben für die Hydrolyse einer Carbonsäureestergruppe beschrieben, in eine Sulfogruppe übergeführt werden.

In einer erhältlichen Verbindung der Formel I mit einer Sulfogruppe kann man diese auf bekannte Art und Weise in eine Sulfonsäureester- oder Sulfonamidgruppe überführen, beispielsweise durch Umwandlung in eine Sulfonsäurehalogenidgruppe und Reaktion mit einem Alkohol, Phenol oder Amin. Eine Sulfonsäureestergruppe wird analog der Carbonsäureestergruppe mit einem Amin in die entsprechende Sulfonamidgruppe umgewandelt.

In einer erhältlichen Verbindung der Formel I, worin eine oder mehrere funktionelle Gruppen geschützt sind, können diese Gruppen, z.B. Carboxy-, Amino-, Hydroxy-, Mercapto- und/oder Sulfogruppen, in an sich bekannter Weise, mittels Solvolyse, insbesondere Hydrolyse, gegebenenfalls enzymatischer Hydrolyse, Alkoholyse oder Acidolyse, oder mittels Reduktion, insbesondere Hydrogenolyse, oder chemischer Reduktion, gegebenenfalls stufenweise oder gleichzeitig, freigesetzt werden. Die Abspaltung der Schutzgruppen ist den weiter vorn im Abschnitt "Schutzgruppen" genannten Standardwerken beschrieben.

Beispielsweise kann man geschütztes Carboxy, z.B. tert-Niederalkoxycarbonyl, in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl, durch Behandeln mit einer geeigneten Säure, z.B. Ameisensäure oder Trifluoressigsäure, gegebenenfalls unter Zugabe einer nukleophilen Verbindung, z.B. Phenol oder Anisol, in freies Carboxy überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch durch Reduktion, z.B. durch Behandeln mit einem Alkalimetall-dithionit, z.B. Natrium-dithionit, mit einem reduzierenden Metall, z.B. Zink, oder einem reduzierenden Metallsalz, wie einem Chrom(II)-salz, z.B. Chrom(II)-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy über führen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umwandeln. Aroylmethoxycarbonyl kann ebenfalls durch Behandeln mit einem nukleophilen, vorzugsweise salzbildenden Reagens, wie Natriumthiophenolat oder Natriumiodid, gespalten werden. 2-Triniederalkylsilylniederalkoxycarbonyl kann auch durch Behandeln mit einem das Fluoridanion liefernden Salz der Fluorwasserstoffsäure, wie einem Alkalimetallfluorid, z.B. Natrium- oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylniederalkylammoniumfluorid, z.B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy übergeführt werden. Mit einer organischen Silylgruppe, wie Triniederalkylsilyl, z.B. Trimethylsilyl, verestertes Carboxy kann in üblicher Weise solvolytisch, z.B. durch Behandeln mit Wasser, einem Alkohol oder Säure, oder ausserdem einem Fluorid, wie oben beschrieben, freigesetzt werden. Verestertes Carboxy kann auch enzymatisch gespalten werden, z.B. verestertes Arginin oder Lysin, wie Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino kann z.B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nukleophilen, vorzugsweise salzbildenden Reagens, wie Natriumthiophenolat, und 4-Nitrobenzyloxycarbonylamino auch durch Behandeln mit einem Alkalimetall-, z.B. Natrium-dithionit, gespalten werden. Gegebenenfalls substituiertes Diphenylmethoxycarbonylamino, tert-Niederalkoxycarbonyl amino oder 2-Triniederalkylsilylniederalkoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch

Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionsprodukts freigesetzt werden. Eine durch 2-Triniederalkylsilylniederalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 30°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy-bzw. Mercaptogruppe wird z.B. durch basische Hydrolyse, eine durch tert-Niederalkyl oder durch einen 2-oxa- oder 2-thiaaliphatischen oder -cycloaliphatischen Kohlenwasserstoffrest geschützte Gruppe durch Acidolyse, z.B. durch Behandeln mit einer Mineralsäure oder einer starken Carbonsäure, z.B. Trifluoressigsäure, freigesetzt. Eine Silylgruppe, z.B. eine Trimethylsilyl- oder tert-Butyldimethylsilylgruppe, wird ebenfalls durch Acidolyse, z.B. durch Mineralsäure, bevorzugt Fluorwasserstoffsäure, oder eine starke Carbonsäure abgespalten. 2-Halogenniederalkoxycarbonyl wird durch die obengenannten Reduktionsmittel, z.B. reduzierendes Metall, wie Zink, reduzierende Metallsalze, wie Chrom(II)-salze, oder durch Schwefelverbindungen, beispielsweise Natriumdithionit oder bevorzugt Natriumsulfid und Schwefelkohlenstoff, entfernt.

Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Hydrolyse, z.B. in Gegenwart einer Mineralsäure oder einer starken organischen Säure, freigesetzt werden.

Eine als Sulfonsäureester oder Sulfonamid geschützte Sulfogruppe wird beispielsweise durch saure Hydrolyse, z.B. in Gegenwart von Mineralsäure, oder bevorzugt durch basische Hydrolyse, z.B. mit Alkalimetallhydroxid oder Alkalimetallcarbonat, beispielsweise Natriumcarbonat, freigesetzt.

Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der 2-Ethylhexansäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauscherreagens. Innere Salze von Verbindungen der Formel I, welche z.B. eine freie Carboxygruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen übergeführt werden: Metall- und Ammoniumsalze z.B. durch Behandeln mit geeigneten Säuren, Säureadditionssalze z.B. durch Behandeln mit einem geeigneten basischen Mittel.

Stereoisomerengemische, insbesondere Diastereomerengemische, können in an sich bekannter Weise, z.B. durch fraktionierte Kristallisation, Chromatographie etc., in die einzelnen Isomeren aufgetrennt werden.

Racemate können in an sich bekannter Weise, z.B. nach Ueberführung der optischen Antipoden in Diastereomere, beispielsweise durch Umsetzung mit optisch aktiven Säuren oder Basen, gespalten werden.

An einzelnen Chiralitätszentren in einer Verbindung der Formel I, z.B. dem C4-Atom im zentralen 5-Amino-6-cyclohexyl-4-hydroxy-2-isopropyl-hexanoyl-Rest, kann die Konfiguration gezielt umgekehrt werden. Beispielsweise kann man die Konfiguration am C4-Atom durch nukleophile Substitution zweiter Ordnung gemäss Verfahren d) nach Ueberführung der Hydroxygruppe in eine nucleofuge Abgangsgruppe X und

Reaktion mit einem eine Hydroxygruppe einführenden Reagens umkehren.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder man das Verfahren auf irgendeiner Stufe abbricht oder man eine nach dem erfindungsgemässen Verfahren erhältliche Verbindung unter den Verfahrensbedingungen erzeugt und in situ weiterverarbeitet.

Pharmazeutische Präparate:

Die pharmakologisch verwendbaren Verbindungen der vorliegenden Erfindung können z.B. zur Herstellung von pharmazeutischen Präparaten verwendet werden, welche eine wirksame Menge des Wirkstoffs zusammen oder im Gemisch mit einer signifikanten Menge von anorganischen oder organischen, festen oder flüssigen, pharmazeutisch verwendbaren Trägerstoffen enthalten.

Bei den erfindungsgemässen pharmazeutischen Präparaten handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, Alter und dem individuellen Zustand, der zu behandelnden Krankheit sowie von der Applikationsweise ab.

Die Erfindung betrifft auch eine Methode zur Behandlung von durch Retroviren verursachten Krankheiten, z.B. von AIDS, dadurch gekennzeichnet, dass eine therapeutisch wirksame Menge von erfindungsgemässen Verbindungen der Formel I verabreicht wird. Die an Warmblüter, z.B. Menschen von etwa 70 kg Körpergewicht, zu verabreichenden Dosismengen liegen zwischen etwa 3 mg und etwa 3 g, vorzugsweise zwischen etwa 10 mg und etwa 1,5 g, z.B. bei ungefähr 300 mg bis 1000 mg pro Person und Tag, verteilt auf vorzugsweise 1 bis 3 Einzeldosen, die z.B. gleich gross sein können. Ueblicherweise erhalten Kinder die halbe Dosis von Erwachsenen.

Die neuen pharmazeutischen Präparate enthalten von etwa 1 % bis etwa 95 %, vorzugsweise von etwa 20 % bis etwa 90 % des Wirkstoffes. Erfindungsgemässe pharmazeutische Präparate können z.B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z.B. mittels konventioneller Lösungs-, Lyophilisierungs-, Misch-, Granulier- oder Dragierverfahren, hergestellt.

Vorzugsweise verwendet man Lösungen des Wirkstoffs, daneben auch Suspensionen, und zwar insbesondere isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Oel enthalten als ölige Komponente die für Injektionszwecke gebräuchlichen vegetabilen, synthetischen oder halbsynthetischen Oele. Als solche sind insbesondere flüssige Fettsäureester zu nennen, die als Säurekomponente eine langkettige Fettsäure mit 8 - 22, besonders 12 - 22, Kohlenstoffatomen, wie z.B. Laurinsäure, Tridecylsäure, Myristinsäure, Pentadecylsäure, Palmitinsäure, Margarinsäure, Stearinsäure, Arachinsäure, Behensäure oder entsprechende ungesättigte Säuren wie z.B. Oelsäure, Elaidinsäure, Erucasäure, Brasidinsäure oder Linolsäure, enthalten. Die Alkoholkomponente dieser Fettsäureester hat maximal 6 Kohlenstoffatome und ist ein ein- oder mehrwertiger, z.B. ein-, zwei-oder dreiwertiger Alkohol, z.B. Methanol, Ethanol, Propanol, Butanol oder Pentanol oder deren Isomere, vor allem aber Glycol oder Glycerin. Als Fettsäureester sind daher beispielsweise zu nennen: Ethyloleat, Isopropylmyristat, Isopropylpalmitat, "Labrafil M 2735" (Polyoxyethylenglycerintrioleat der Firma Gattefossé, Paris), "Myglyol 812" (Triglycerid gesättigter Fettsäuren der Kettenlänge $C_8$ bis $C_{12}$ der Firma Chemische Werke Witten/Ruhr, Deutschland), besonders aber vegetabile Oele wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder

Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Ethylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Ausgangsmaterialien:

Neue Ausgangsmaterialien und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den als bevorzugt aufgeführten Verbindungen gelangt.

Die Ausgangsmaterialien zur Durchführung des Verfahrens a) können nach an sich bekannten Verfahren hergestellt werden, z.B. aus den betreffenden Aminosäuren durch Kondensation analog dem vorstehend beschriebenen Verfahren a). Beispielsweise kann man eine Verbindung der Formel

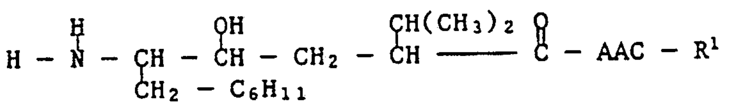

(XIII)

analog dem in den Europäischen Patentanmeldungen EP 143 746 oder EP 258 183 beschriebenen Verfahren herstellen.

Verbindungen der Formel V werden beispielsweise hergestellt, indem man eine Carbonsäure der Formel

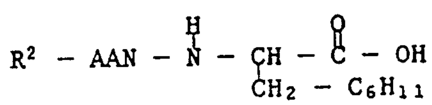

(XIV)

oder ein geeignetes funktionelles Derivat davon, worin die Symbole die genannten Bedeutungen haben und funktionelle Gruppen mit Ausnahme der gegebenenfalls abgewandelten Carboxygruppe gegebenenfalls in geschützter Form vorliegen, mit einer Organometallverbindung der Formel VII, worin M ein Metallradikal, z.B. -Li oder -MgHal, wie -MgCl, -MgBr oder -MgI bedeutet, umsetzt und das gebildete Additionsprodukt solvolysiert. Geeignete funktionelle Derivate einer Carbonsäure der Formel XIV sind beispielsweise das entsprechende Lithiumsalz der Carbonsäure, ein Carbonsäurehalogenid, z.B. Carbonsäurechlorid, ein Anhydrid, z.B. das symmetrische Carbonsäureanhydrid oder ein gemischtes Carbonsäureanhydrid mit einer sterisch gehinderten Carbonsäure, z.B. mit Pivalinsäure, oder ein Thioester, z.B. 2-Pyridylthioester.

Die Umsetzung einer Carbonsäure der Formel XIV oder eines geeigneten funktionellen Derivats davon mit einer Verbindung der Formel VII erfolgt in der üblichen Weise, z.B. nach den in Verfahren c) angegebenen Reaktionsbedingungen, gegebenenfalls jedoch unter Kühlung, z.B. bei Temperaturen von ca. -50° C bis ca. 0° C. In einer bevorzugten Ausführungsform des Verfahrens wird ein 2-Pyridylthioester der Carbonsäure der Formel XIV mit einer Brommagnesium-Verbindung der Formel VII umgesetzt.

Verbindungen der Formel VI können nach an sich bekannten Verfahren hergestellt werden beispielsweise indem man in einer Verbindung der Formel XIV, worin die Symbole die genannten Bedeutungen haben

und funktionelle Gruppen gegebenenfalls in geschützter Form vorliegen, die Carboxygruppe nach an sich bekannten Methoden, biespielsweise über den entsprechenden Methyl- oder Ethylester, über ein Imidazolid oder über ein N-Methoxy-N-methylamid, zur Aldehyd-Funktion reduziert.

Verbindungen der Formel VII können beispielsweise durch Umsetzung eines bekannten oder mit an sich bekannten Methoden herstellbaren Halogenids der Formel

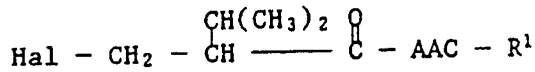
(XV),

z.B. des Chlorids, mit einem Metallierungsmittel, z.B. Magnesium, Lithium oder tert-Butyllithium, hergestellt werden.

Verbindungen der Formel VIII werden beispielsweise hergestellt, indem man einen Aldehyd der Formel VI mit einer Organometallverbindung der Formel VII gemäss Verfahren c) umsetzt und die gebildete Hydroxy-Verbin dung der Formel I, gegebenenfalls nach Trennung der Isomeren, mit einer der Definition von X entsprechenden starken organischen oder anorganischen Säure verestert.

Nitrile der Formel IX werden beispielsweise hergestellt, indem man eine Verbindung der Formel

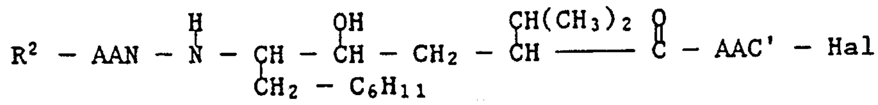
(XVI),

worin die Symbole die gennanten Bedeutungen haben, mit einem Salz der Cyanwasserstoffsäure umsetzt.

Geeignete Salze der Cyanwasserstoffsäure sollten im gewählten inerten Lösungsmittel genügend löslich sein, damit eine Umsetzung erfolgen kann. Solche Salze sind z.B. Ammoniumcyanid, Alkalimetall- oder Erdalkalimetallcyanide, z.B. Natrium- oder Kaliumcyanid, oder Uebergangsmetallcyanide, z.B. Kupfercyanid. Letztere eignen sich aufgrund ihrer im Vergleich zu den Alkalimetallcyaniden geringeren Basizität.

Je nach der Art des eingesetzten Cyanids und des Lösungsmittels stellt sich ein Gleichgewicht zwischen der isomeren Nitril- und der Isonitrilform ein. Die Nitrilform wird bevorzugt gebildet, wenn z.B. die Umsetzung mit solchen Metallcyaniden erfolgt, deren Metallkationen ein niedrigeres Atomgewicht als das von Kupfer besitzen.

Geeignete inerte Lösungsmittel sind vor allem polare, aprotische Lösungsmittel, beispielsweise Carbonsäureamide, z.B. Dimethylformamid oder Dimethylacetamid, Nitrile, z.B. Acetonitril oder Propionitril, oder Diniederalkylsulfoxide, z.B. Dimethylsulfoxid.

Die Umsetzung erfolgt bei Raumtemperatur, bei erniedrigter oder bei erhöhter Temperatur, z.B. in einem Temperaturbereich von etwa -40° C bis etwa +100° C, bevorzugt von etwa -10° C bis etwa +50° C und gewünschtenfalls in einer Inertgas-, z.B. Stickstoffatmosphäre.

Epoxide der Formel X werden z.B. hergestellt, indem man eine Verbindung der Formel

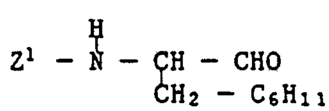
(XVII),

worin $Z^1$ eine Aminoschutzgruppe ist, mit einer Phosphoranylidenverbindung der Formel

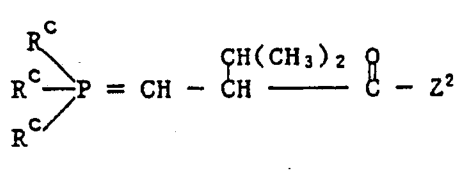
(XVIII),

worin $R^c$ einen gegebenenfalls substituierten Kohlenwasserstoffrest darstellt und $Z^2$ eine Carboxyschutzgruppe ist, umsetzt und eine erhältliche Verbindung der Formel

$$Z^1HN - \underset{CH_2 - C_6H_{11}}{CH} - CH = CH - \overset{CH(CH_3)_2}{CH} \text{———} \overset{O}{\overset{\|}{C}} - Z^2 \qquad (XIX)$$

mit einem die Peroxygruppe enthaltenden Oxidationsmittel in ein Epoxid überführt und in einer erhältlichen Verbindung in beliebiger Reihenfolge der Reaktionsschritte die Schutzgruppen $Z^1$ und $Z^2$ abspaltet und durch die Gruppen $R^2$-AAN- und -AAC-$R^1$ ersetzt.

$R^c$ ist bevorzugt Phenyl. Die Umsetzung einer Verbindung der Formel XVII mit einer Phosphoranylidenverbindung der Formel XVIII erfolgt unter den für Wittig-Reaktionen bekannten und z.B. im Organikum beschriebenen Reaktionsbedingungen. Das dabei erhältliche Olefin der Formel XIX wird gegebenenfalls in situ mit dem Oxidationsmittel, z.B. Peressigsäure oder m-Chlorperbenzoesäure, umgesetzt. Die Abspaltung der Schutzgruppen $Z^1$ und $Z^2$ und die Einführung der Gruppen $R^2$-AAN- bzw. -AAC-$R^1$ ist weiter vorn unter Verfahren a) beschrieben.

Verbindungen der Formel XI werden beispielsweise hergestellt, indem man eine Acrylsäure der Formel

$$R^b - (CH_2)_q - \underset{CH_2}{\underset{\|}{C}} - COOH \qquad (XX)$$

oder ein geeignetes funktionelles Derivat davon mit einer Verbindung der Formel

$$H - AAN - \overset{H}{N} - \underset{CH_2 - C_6H_{11}}{CH} - \overset{OH}{CH} - CH_2 - \overset{CH(CH_3)_2}{CH} \text{———} \overset{O}{\overset{\|}{C}} - AAC - R^1 \qquad (XXI)$$

gemäss Verfahren a) umsetzt. Die Verbindung der Formel XXI wird ebenfalls gemäss Verfahren a), beispielsweise aus einer an der Aminogruppe geschützten Verbindung der Formel $Z^1$-AAN-OH und einer Verbindung der Formel XIII und anschliessender Abspaltung der Schutzgruppe $Z^1$ hergestellt.

Verbindungen der Formel XII werden gleichermassen durch Kondensation einer Verbindung der Formel XXI mit der entsprechenden Carbonsäure oder einem geeigneten funktionellen Derivat davon gemäss Verfahren a) erhalten.

Die folgenden Beispiele dienen zur Illustration der Erfindung, schränken deren Umfang jedoch in keiner Weise ein.

Temperaturen werden in Celsiusgraden angegeben. Die $R_f$-Werte werden auf Kieselgeldünnschichtplatten in folgenden Lösungsmittelsystemen ermittelt:

| | |
|---|---|
| A Essigester-n-Hexan | 1:1 |
| B Essigester-n-Hexan | 1:2 |
| C Essigester-n-Hexan | 1:4 |
| D Essigester-n-Hexan | 1:6 |
| E Essigester-n-Hexan | 1:9 |
| F Methylenchlorid-Methanol | 19:1 |
| G Methylenchlorid-Methanol | 9:1 |
| H Methylenchlorid-Methanol | 4:1 |
| I Methylenchlorid-Methanol-Wasser | 300:10:1 |
| J Methylenchlorid-Ether | 4:1 |
| K Methylenchlorid-Methanol-Ammoniak konz. | 40:10:1 |
| L Methylenchlorid-Methanol-Ammoniak konz. | 350:50:1 |
| M Methylenchlorid-Methanol-Wasser-Eisessig | 150:54:10:1 |
| N Methylenchlorid-Methanol-Wasser | 14: 6:1 |
| O Chloroform-Methanol-Wasser-Eisessig | 150:54:10:1 |
| P Chloroform-Methanol-Wasser-Eisessig | 180:20:2:1 |
| Q Chloroform-Methanol-Wasser-Eisessig | 170:26:3:1 |
| R Essigester | |
| S Methylenchlorid-Methanol-Ammoniak konz. | 800:50:1 |
| T Methylenchlorid-Methanol-Ammoniak konz. | 90:10:1 |
| U Toluol-Essigester | 4:1 |

Beispielsweise bedeutet die Abkürzung "$R_f$(A)", dass der $R_f$-Wert im System A ermittelt wurde. Das Mengenverhältnis der Lösungsmittel zueinander ist in Volumenanteilen angegeben.

Die gleichen Abkürzungen werden für die Bezeichnung der Fliessmittel-Systeme bei der Flash-Chromatographie und der Mitteldruckchromatographie verwendet.

Die Retentionszeiten ($t_{Ret}$) in der Hochdruckflüssig-Chromatographie (HPLC) werden auf einer 250 x 4,6 mm reversed phase $C_{18}$ Nucleosil® 5 $\mu$ Säule bei einer Fliessgeschwindigkeit von 1 ml/Min. ermittelt. Gradient A: 0 % → 100 % Acetonitril in Wasser enthaltend 0,05 % Trifluoressigsäure in 60 Min.; Gradient B: 10 % → 100 % Acetonitril in Wasser enthaltend 0,05 % Trifluoressigsäure in 40 Min. Beispielsweise bedeutet die Abkürzung "$t_{Ret}$(A)", dass die Retentionszeit mit Gradient A ermittelt wurde.

Die Werte für Protonen-Kernresonanzspektroskopie ($^1$H-NMR) werden in ppm (parts per million) bezogen auf Tetramethylsilan als internen Standard angegeben. s = Singulett, d = Dublett, t = Triplett, q = Quartett, m = Multiplett, dd = Doppeldublett, br = breit. Bei der "fast atom bombardment" Massenspektrometrie (FAB-MS) sind die Werte für die protonierte Masse $(M+H)^+$ angegeben.

Der Rest mit der Bezeichnung -Cha $\stackrel{c}{-}$ Val- bedeutet das zweiwertige Radikal von (2S,4S,5S)-5-Amino-6-cyclohexyl-4-hydroxy-2-isopropyl-hexansäure und hat die Formel

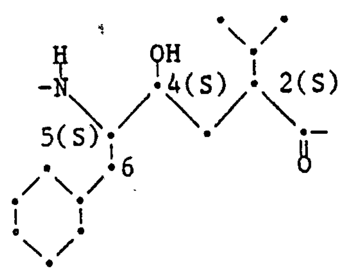

Der Rest mit der Bezeichnung -Cha $\stackrel{cx}{-}$ Val- leitet sich vom Rest -Cha $\stackrel{c}{-}$ Val-durch Ueberbrückung von NH und OH durch eine Isopropyliden-Gruppe ab und hat die Formel

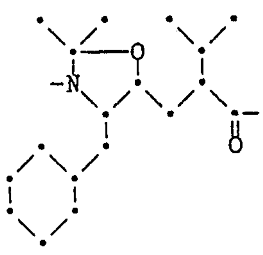

Zur Bezeichnung von zweiwertigen Radikalen von natürlichen α-Aminosäuren werden die in der Peptidchemie üblichen Abkürzungen verwendet. An der phenolischen Hydroxygruppe mit dem Rest R veretherte Tyrosin-Radikale werden mit Tyr(OR) bezeichnet. Nle bedeutet das Radikal von Norleucin, Cha das Radikal von Cyclohexylalanin.

Weitere Abkürzungen:

abs. = absolut (wasserfrei)
BBSP = 2(S)-Benzyl-3-tert-butylsulfonyl-propionyl
BOC = tert-Butoxycarbonyl
BOP = Benzotriazol-1-yloxytris(dimethylamino)phosphonium-hexafluorophosphat
DCCI = Dicyclohexylcarbodiimid
DCH = Dicyclohexylharnstoff
DMF = Dimethylformamid
DMSO = Dimethylsulfoxid
Fmoc = 9-Fluorenylmethoxycarbonyl
ges. = gesättigt
HBTU = O-Benzotriazol-1-yl-N,N,N′,N′-tetramethyluronium-hexafluorophosphat
HOBH = N-Hydroxy-endo-norbornan-2,3-dicarbonsäureimid
HOBt = 1-Hydroxybenzotriazol
konz. = konzentriert
Min. = Minute(n)
Sdp. = Siedepunkt
Smp. = Schmelzpunkt
Std. = Stunde(n)
Tcp = Trichlorphenyl
THF - Tetrahydrofuran
Z = Benzyloxycarbonyl

Beispiel 1: Z-Arg-Arg-Pro-Phe-Val-Cha $\stackrel{c}{-}$ Val-Val-Tyr-OMe

Eine Mischung aus 160 mg H-Phe-Val-Cha $\stackrel{c}{-}$ Val-Val-Tyr-OMe, 180 mg Z-Arg-Arg-Pro-OH•HCl, 42 mg Triethylaminhydrochlorid, 62 mg DCCI, 42 mg HOBt und 10 ml DMF wird 48 Std. bei Raumtemperatur gerührt. Der kristallisierte DCH wird abfiltriert und das Filtrat eingedampft. Der Rückstand wird in Methanol gelöst und mit Diisopropylether gefällt. Das Rohprodukt wird anschliessend durch Flash-Chromatographie an 20 g Kieselgel (Laufmittel M) gereinigt. $R_f(M)$ = 0,25; FAB-MS: $(M+H)^+$ = 1337; $t_{Ret}(A)$ = 35,2 Min.

Die Ausgangsmaterialien werden folgendermassen hergestellt:

a) 2(S)-Benzyloxycarbonylamino-3-cyclohexyl-propionsäureethylester:

243 g 2(S)-Benzyloxycarbonylamino-3-cyclohexyl-propionsäure (Herstellung: Helvetica Chimica Acta 57, 2131(1974)) werden in 600 ml Toluol und 900 ml Ethanol vorgelegt. Das Reaktionsgemisch wird auf 0° gekühlt und 88,3 g Thionylchlorid innerhalb 30 Min. zugetropft. Die Kühlung wird entfernt und die Mischung während 18 Std. gerührt. Das Reaktionsgemisch wird filtriert und das Filtrat eingeengt. Der Rückstand wird mittels Flash-Chromatographie (2 kg Kieselgel 60, 40-63 µm, Laufmittel E) aufgetrennt. Die produkthaltigen Fraktionen werden vereinigt, eingedampft und im Hochvakuum getrocknet. Man erhält die Titelverbindung

als leicht gelbliches Oel. $R_f(E)$ = 0,2; $R_f$ (B) = 0,52.

a) 2(S)-Benzyloxycarbonylamino-3-cyclohexyl-propanal:

116,1 g 2(S)-Benzyloxycarbonylamino-3-cyclohexyl-propionsäureethylester werden in 2,2 l Toluol vorgelegt und auf -65° abgekühlt. 836 ml Diisobutylaluminiumhydrid werden innerhalb 30 Min. tropfenweise bei -65° zugegeben und das Gemisch 20 Min. nachgerührt. Dann werden bei -65° 84,2 ml Methanol innerhalb 10 Min. zugetropft, anschliessend 825 ml wässrige Kalium-natrium-tartrat-Lösung ohne Kühlung. Das Reaktionsgemisch wird auf 3 l Kalium-natrium-tartrat-Lösung/Eis ausgetragen und mit 5 l Ether extrahiert. Die Etherphase wird mit 2 l Wasser gewaschen, dann sofort in eine Lösung bestehend aus 106 g Semicarbazid-Hydrochlorid und 156,5 g Natriumacetat in 620 ml Wasser und 620 ml Ethanol gegossen. Das Reaktionsgemisch wird 1 Std. bei Raumtemperatur nachgerührt, dann im Scheidetrichter abgetrennt und die Wasserphase mit 2 x 1,5 l Ether extrahiert. Die organische Phase wird über Magnesiumsulfat getrocknet und eingedampft. Das Rohprodukt wird mittels Flash-Chromatographie gereinigt (2 kg Kieselgel 60, 40-63 µm, Laufmittel A). Durch Eindampfen der vereinigten, produkthaltigen Fraktionen erhält man das Semicarbazon der Titelverbindung, $R_f$ (G) = 0,51. 130 g dieses Semicarbazons werden in 1 l THF gelöst, mit 282 ml 37 % Formaldehydlösung und dann bei 10° mit 143 ml 0,5N HCl versetzt. Das Reaktionsgemisch wird 2 Std. bei Raumtemperatur gerührt, filtriert und das Filtrat mit 0,5 l Wasser, 0,5 l $NaHCO_3$ und 0,5 l Wasser gewaschen. Die Wasserphasen werden mit 600 ml Ether extrahiert. Die Etherphasen werden über Magnesiumsulfat getrocknet und eingedampft. Der Rückstand wird mit 100 ml Toluol versetzt und eingedampft, wobei die Titelverbindung erhalten wird. Diese wird sofort weiterverarbeitet.

c) (1(S)-Benzyloxycarbonylamino-2-cyclohexyl-ethyl)-oxiran:

18,9 g Natriumhydriddispersion (55 % in Oel) werden in einem trockenen Sulfierkolben unter Argon durch dreimaliges Aufrühren in 50 ml Petrolether (Sdp. 40-60°) und anschliessendes Abdekantieren des Lösungsmittels vom Oel befreit. Nach Trocknen im Hochvakuum wird ein graues Pulver erhalten, das in 500 ml THF vorgelegt und mit 55,6 g Trimethylsulfoxoniumiodid versetzt wird, wobei die Temperatur auf ca. 40° steigt. Die graue Suspension wird am Rückfluss 1 Std. gekocht und anschliessend innerhalb von 50 Min. bei -70° mit einer Lösung von 108,6 g 2(S)-Benzyloxycarbonylamino-3-cyclohexyl-propanal in 250 ml THF versetzt. Die gelbe Suspension wird 2 Std. bei 0° gerührt. Die gelblich-trübe Lösung wird auf 500 g Eis gegossen. Die wässrige Lösung wird mit 2,5 l Ether extrahiert, die organische Phase mit Wasser gewaschen und nach Trocknen über Natriumsulfat eingedampft. Der ölige Rückstand wird mittels Flash-Chromatographie (2,5 kg Kieselgel 60, 40-63 µm, Laufmittel C) aufgetrennt. Die produkthaltigen Fraktionen werden vereinigt, eingedampft und im Hochvakuum getrocknet. Man erhält die Titelverbindung (Diastereomerengemisch, ca. 4:1) als leicht gelbliches Oel. $R_f$(I) = 0,71; $R_f$(C) = 0,16.

d) 3(S)-Benzyloxycarbonylamino-4-cyclohexyl-1-iod-butan-2(R,S)-ol:

42,3 g (1(S)-Benzyloxycarbonylamino-2-cyclohexyl-ethyl)-oxiran werden in 200 ml Acetonitril aufgenommen und die erhaltene Lösung auf 0° abgekühlt. Nach Zugabe von 20,9 g Natriumiodid werden während 30 Min. tropfenweise bei 0° 17,7 ml Trimethylchlorsilan zugegeben. Das Gemisch wird 40 Min. bei 0 - 3° gerührt und anschliessend auf 700 ml eiskaltes Wasser gegossen. Das wässrige Gemisch wird mit Ether extrahiert und die organische Phase mit 750 ml 5 %-iger wässriger Natriumthiosulfatlösung und 750 ml gesättigter, wässriger Natriumchloridlösung gewaschen. Nach Trocknen über Natriumsulfat und Eindampfen erhält man ein öliges Gemisch der Titelverbindung, die direkt weiterverarbeitet wird.

e) 3-Benzyloxycarbonyl-4(S)-cyclohexylmethyl-2,2-dimethyl-5(R)-iod-methyl-1,3-oxazolidin:

49,3 g der Verbindung Beispiel 1d) und 1,07 g p-Toluolsulfonsäuremonohydrat werden in 140 ml 2,2-Dimethoxypropan und 450 ml Methylenchlorid 3 Std. lang bei Raumtemperatur gerührt. Das Gemisch wird zwischen 1 l Methylenchlorid und 500 ml gesättigter, wässriger Natriumhydrogencarbonatlösung ausgeschüttelt. Die organische Phase wird mit Wasser gewaschen, über Natriumsulfat getrocknet und eingedampft. Das Rohprodukt wird mittels Flash-Chromatographie gereinigt (3 kg Kieselgel 60, 40-63 µm,

Laufmittel D). Durch Eindampfen der vereinigten, produkthaltigen Fraktionen erhält man die Titelverbindung als leicht gelbliches Oel. $R_f$ (C) = 0,55; $R_f$ (D) = 0,46.

f) 2(R,S)-(3-Benzyloxycarbonyl-4(S)-cyclohexylmethyl-2,2-dimethyl-1,3-oxazolidinyl-5(S)-methyl)-3-methylbuttersäuremethylester:

14,3 ml Diisopropylamin werden in 200 ml absolutem Tetrahydrofuran unter Argon gelöst und auf 0° abgekühlt. Anschliessend wird bei 0 - 5° das Gemisch 20 Min. lang tropfenweise mit 65,8 ml einer 1,6M Lösung von n-Butyllithium in Hexan versetzt und 20 Min. gerührt. Dann werden bei -70° bis -75° 13,3 ml Isovaleriansäuremethylester zugetropft und die Mischung 1,5 Std. bei -75° gerührt. Bei -60° bis -75° werden unter Rühren 320 ml Hexamethylphosphorsäuretriamid zugetropft. Die entstandene Suspension wird 10 Min. lang gerührt und schliesslich bei -70° bis -75° in 5 Min. tropfenweise mit einer Lösung von 43,4 g der Verbindung Beispiel 1e) in 110 ml Tetrahydrofuran versetzt. Das Reaktionsgemisch wird bei Raumtemperatur während 2,5 Std. gerührt und schliesslich auf ein Gemisch von 1 l gesättigter, wässriger Ammoniumchloridlösung und 500 g Eis gegossen. Die wässrige Phase wird mit 2 l Essigester extrahiert, die organische Phase mit Wasser gewaschen und über Natriumsulfat getrocknet. Nach Eindampfen erhält man das Diastereomerengemisch der Titelverbindung als gelbes Oel. $R_f$ (C) = 0,36; $R_f$ (E) = 0,21 (Werte für die weniger polare Komponente).

g) 2(R,S)-(3-Benzyloxycarbonyl-4(S)-cyclohexylmethyl-2,2-dimethyl-1,3-oxazolidinyl-5(S)-methyl-3-methylbuttersäure:

16,5 g Kalium-tert-butylat werden in 250 ml Ether bei ca. 5° mit 1,77 ml Wasser versetzt. Die weisse Suspension wird noch 10 Min. im Eisbad gerührt und darauf mit 35,8 g der Verbindung Beispiel 1f) (Diastereomerengemisch) in 250 ml Ether versetzt, wobei die Temperatur unterhalb 10° gehalten wird. Das Reaktionsgemisch wird nun während 18 Std. bei Raumtemperatur gerührt und schliesslich auf 500 ml gesättigte, wässrige Ammoniumchloridlösung gegossen. Die wässrige Phase wird mit Essigester extrahiert und die organische Phase mit gesättigter, wässriger Natriumchloridlösung gewaschen, über Natriumsulfat getrocknet und eingedampft. Das ölige Rohprodukt wird durch Flash-Chromatographie aufgetrennt (2,5 kg Kieselgel 60, 40 - 63 µm, Laufmittel C). Z-Cha $\underline{cx}$ Val-OH, die weniger polare Komponente der Titelverbindung mit der gewünschten Konfiguration des an die Isopropylgruppe gebundenen C-Atoms (S-Konfiguration) wird als gelbes Oel erhalten. $R_f$ (I) = 0,20; $R_f$ (J) = 0,35.

h) HCl•H-Val-Tyr-OMe:

10,0 g Z-Val-Tyr-OMe werden in 200 ml Methanol und 24 ml 1 N HCl in Gegenwart von 1,0 g Palladium-Kohle (10 % Pd) bei Normaldruck und Raumtemperatur bis zur Sättigung hydriert. Das Reaktionsgemisch wird filtriert, und das Filtrat wird eingedampft und getrocknet. $R_f$ (N) = 0,64.

i) Z-Cha $\underline{cx}$ Val-Val-Tyr-OMe:

Eine Mischung aus 2,06 g HCl•H-Val-Tyr-OMe, 3,12 g Z-Cha $\underline{cx}$ Val-OH, 1,74 g DCCI, 1,22 g HOBt, 0,75 g N-Methylmorpholin und 50 ml DMF wird 16 Std. bei Raumtemperatur gerührt. Der DCH wird abfiltriert, das Filtrat eingeengt und am Hochvakuum getrocknet. Der Rückstand wird mittels Flash-Chromatographie (100 g Kieselgel 60, Laufmittel B) gereinigt. $R_f$ (A) = 0,36.

j) H-Cha $\underline{c}$ Val-Val-Tyr-OMe:

3,6 g Z-Cha $\underline{cx}$ Val-Val-Tyr-Ome werden in 80 m Methanol-Wasser 9:1 in Gegenwart von 360 mg Palladium-Kohle (10 % Pd) bei Normaldruck und Raumtemperatur bis zur Sättigung hydriert. Das Reaktionsgemisch wird filtriert, und das Filtrat wird mit 30 ml Wasser verdünnt und für 5 Std. bei Raumtemperatur gerührt. Nach Abdampfen des Lösungsmittels wird die Titelverbindung aus Dioxan/Wasser 9:1 lyophilisiert. $R_f$ (H) = 0,10.

k) BOC-Val-Cha $\overset{c}{-}$ Val-Val-Tyr-OMe:

wird ausgehend von 1,45 g H-Cha $\overset{c}{-}$ Val-Val-Tyr-OMe, 630 mg BOC-Val-OH, 530 mg HOBH und 660 mg DCCI analog Beispiel 1i) erhalten und durch Umfällung aus Methanol-$NaHCO_3$-Lösung gereinigt. $R_f$ (G) = 0,75.

l) HCl•H-Val-Cha $\overset{c}{-}$ Val-Val-Tyr-OMe:

2,16 g BOC-Val-Cha $\overset{c}{-}$ Val-Val-Tyr-OMe werden in 20 ml 4,3N HCl in Dioxan gelöst und für 30 Min. bei Raumtemperatur gerührt. Das Reaktionsgemisch wird eingedampft, und der Rückstand wird durch Flash-Chromatographie (60 g Kieselgel 60, Laufmittel G) gereinigt. $R_f$ (G) = 0,18.

m) Fmoc-Phe-Val-Cha $\overset{c}{-}$ Val-Val-Tyr-OMe:

Eine Mischung aus 240 mg HCl•H-Val-Cha $\overset{c}{-}$ Val-Val-Tyr-Ome, 170 mg Fmoc-Phe-OTcp, 0,15 ml N-Ethyldiisopropylamin und 10 ml DMF wird 20 Std. bei Raumtemperatur gerührt. Das Reaktionsgemisch wird eingedampft, und der Rückstand wird mit 100 ml Diisopropylether gefällt. Der Niederschlag wird abfiltriert und anschliessend aus Dioxan/Wasser 9:1 lyophilisiert.

n) H-Phe-Val-Cha $\overset{c}{-}$ Val-Val-Tyr-OMe:

Eine Lösung aus 305 mg Fmoc-Phe-Val-Cha $\overset{c}{-}$ Val-Val-Tyr-OMe, 5 ml Piperidin und 5 ml DMF wird für 60 Min. bei Raumtemperatur gerührt und danach eingedampft. Der Rückstand wird in wenig Methylenchlorid gelöst und mit Diisopropylether gefällt. Der Niederschlag wird bei 0° abfiltriert und anschliessend aus Dioxan/Wasser 9:1 lyophilisiert. $R_f$ (M) = 0,7.

Beispiel 2: BBSP-Val-Cha $\overset{c}{-}$ Val-Val-Tyr-OMe:

Analog Beispiel 1 wird die Titelverbindung ausgehend von 760 mg HCl•H-Val-Cha $\overset{c}{-}$ Val-Val-Tyr-OMe (Beispiel 1 l), 350 mg 2(S)-Benzyl-3-tert-butylsulfonylpropionsäure (BBSP-OH), 190 mg HOBt und 280 mg DCCI hergestellt und durch Flash-Chromatographie gereinigt. $R_f$ (G) = 0,50; FAB-MS: $(M+H)^+$ = 913; $t_{Ret}$-(A) = 42.1 Min.

Das Ausgangsmaterial wird wie folgt hergestellt.

a) 2(S)-Benzyl-3-tert-butylsulfonyl-propionsäure (BBSP-OH):

142 g 2(R,S)-Benzyl-3-tert-butylsulfonyl-propionsäure werden mit 85,7 g (+)-Dehydroabietylamin und 27,8 ml Triethylamin in 2 l Isopropanol umgesetzt. Nach viermaliger Kristallisation des Salzes aus heissem Isopropanol wird die Säure wieder durch Extraktion mit verdünnter Natriumcarbonatlösung und anschliessendem Ansäuren mit verdünnter Salzsäure freigesetzt. Durch Umkristallisation aus Essigester/Hexan wird die Titelverbindung in hoher optischer Reinheit erhalten (über 98 % ee): $[\alpha]_D^{20}$ = -10,9° (c = 0,91 in $CH_2Cl_2$); Smp. 99-101°; $R_f$ (A) = 0,16; $R_f$ (B) = 0,4.

b) 2-(R,S)-Benzyl-3-tert-butylsulfonyl-propionsäure:

91,9 g 2-Benzyl-3-tert-butylsulfonyl-propionsäureethylester werden in 500 ml 6 N Salzsäure und 100 ml Essigsäure 15 Std. am Rückfluss gekocht. Beim Abkühlen kristallisiert die Titelverbindung direkt aus dem Reaktionsgemisch. Smp. 147-8°; $R_f$ (B) = 0,4; $^1$H-NMR ($CDCl_3$): 1,35 (s, 9H); 2,97 (m, 1H); 3,05 (dd, 1H); 3,22 (dd, 1H); 3.45 (m, 2H); 7,25 (m, 5H); 8,5 (br s, 1H).

c) 2-Benzyl-3-tert-butylsulfonyl-propionsäure-ethylester:

60 g α-Benzylacrylsäure-ethylester werden in 600 ml Ethanol gelöst und mit 0,83 g Natriummethylat und 37 ml tert-Butylmercaptan umgesetzt. Die Mischung wird 24 Std. bei Raumtemperatur gerührt, mit 500 ml 0,04 N wässeriger Schwefelsäure verdünnt und unter Eiskühlung mit 260 g Oxone® (Kaliumperoxomonosulfat, 50 % $KHSO_5$, Ventron) versetzt. Das Reaktionsgemisch wird über Nacht bei Raumtemperatur gerührt, dann filtriert und eingeengt. Die wässrige Lösung wird mit Methylenchlorid extrahiert, die Extrakte über Natriumsulfat getrocknet und eingedampft. Smp. 47-48°; $^1$H-NMR ($CDCl_3$): 1,13 ppm (t, 3H); 1,38 (s, 9H); 2,95 (dd, 1H); 3,01 (dd, 1H); 3,10 (dd, 1H); 3,42 (dd, 1H); 3,46 (dd, 1H); 4,12 (q, 2H); 7,25 (m, 5H).

d) α-Benzylacrylsäure-ethylester::

20 g Benzylmalonsäurediethylester in 40 ml Ethanol werden bei Raumtemperatur mit 4,0 g KOH in 50 ml Ethanol versetzt, über Nacht bei Raumtemperatur gerührt, eingedampft, mit 7,1 ml Wasser versetzt und im Eisbad mit 6,3 ml konz. Salzsäure angesäuert. Es wird zwischen Wasser und Ether verteilt, die organische Phase getrocknet und der Ether abdestilliert. Der Rückstand wird mit 12,9 ml Pyridin, 0,61 g Piperidin und 1,78 g Paraformaldehyd versetzt. Das Gemisch wird im Oelbad (130°) während 90 Min. erhitzt, abgekühlt, mit 220 ml Wasser versetzt und dreimal mit 75 ml n-Hexan extrahiert. Die vereinigten organischen Phasen werden mit Wasser, 1 N HCl, Wasser, ges. $NaHCO_3$-Lösung und Sole gewaschen. Die Titelverbindung wird durch Destillation gewonnen. $^1$H-NMR (DMSO-$d_6$): 1,2 ppm (t, 3H); 3,6 (d, 2H); 4,1 (q, 2H); 5,6 (m, 1H); 6,15 (m, 1H); 7,25 (m, 5H).

Beispiel 3: BBSP-Val-Cha $\overset{c}{-}$ Val-Val-Tyr-OH

Eine Mischung aus 65 mg BBSP-Val-Cha $\overset{c}{-}$ Val-Val-Tyr-OMe (Beispiel 2), 2 ml 2 N NaOH und 3 ml Dioxan wird 20 Min. bei Raumtemperatur gerührt. Das Reaktionsgemisch wird anschliessend mit 2 ml 2 N HCl verdünnt und lyophilisiert. Der Rückstand wird mittels Flash-Chromatographie (8 g Kieselgel, Laufmittel H) gereinigt. $R_f$ (N) = 0,53.

Beispiel 4: BBSP-Val-Cha $\overset{c}{-}$ Val-Val-Tyr-$NH_2$

Analog Beispiel 2 wird die Titelverbindung ausgehend von 30 mg HCl•H-Val-Cha $\overset{c}{-}$ Val-Val-Tyr-$NH_2$, 15 mg 2(S)-Benzyl-3-tert-butylsulfonylpropionsäure (BBSP-OH), 8 mg HOBt und 12 mg DCCI hergestellt und durch Flash-Chromatographie an 7 g Kieselgel 60 (Laufmittel F) gereinigt. $R_f$ (G) = 0,32; FAB-MS: $(M+H)^+$ = 898; $t_{Ret}$(A) = 41,7 Min.

Die Ausgangsmaterialien werden folgendermassen hergestellt:

a) HCl•H-Val-Cha $\overset{c}{-}$ Val-Val-Tyr-$NH_2$

wird ausgehend von 360 mg BOC-Val-Cha $\overset{c}{-}$ Val-Val-Tyr-$NH_2$ und 5,0 ml 4,3 N HCl in Dioxan analog Beispiel 1 l) hergestellt. $R_f$ (G) = 0,33.

b) BOC-Val-Cha $\overset{c}{-}$ Val-Val-Tyr-$NH_2$

wird ausgehend von 300 mg H-Cha $\overset{c}{-}$ Val-Val-Tyr-$NH_2$, 147 mg BOC-Val-OH, 122 mg HOBH und 268 mg DCCI analog Beispiel 1 k) erhalten. $R_f$ (L) = 0,3.

c) H-Cha $\overset{c}{-}$ Val-Val-Tyr-$NH_2$

wird durch Hydrierung von 600 mg Z-Cha $\overset{cx}{-}$ Val-Val-Tyr-$NH_2$ in Gegenwart von 60 mg Palladium-Kohle (10 % Pd) analog Beispiel 1 j) erhalten. $R_f$ (L) = 0,06.

d) Z-Cha $\overset{cx}{-}$ Val-Val-Tyr-$NH_2$:

Eine Mischung aus 772 mg Z-Cha $\overset{cx}{-}$ Val-Val-Tyr-OH, 590 mg $NH_4Cl$, 315 mg DCCI, 217 mg HOBt

und 10 ml DMF wird mit $NaHCO_3$ auf pH 5,5-6,0 gestellt und anschliessend für 16 Std. gerührt. Der DCH wird abfiltriert und das Filtrat eingedampft. Der Rückstand wird durch Flash-Chromatographie an 40 g Kieselgel 60 (Laufmittel F) gereinigt. $R_f$ (G) = 0,40.

e) Z-Cha $\stackrel{cx}{-}$ Val-Val-Tyr-OH

wird ausgehend von 790 mg Z-Cha $\stackrel{cx}{-}$ Val-Val-Tyr-OMe (Beispiel 1 i), 4 ml 4 N NaOH und 16 ml Dioxan analog Beispiel 3 hergestellt. $R_f$ (G) = 0,07.

Beispiel 5: N-(3-Amino-3,3-dimethylpropanoyl)-Tyr(OMe)-Val-Cha $\stackrel{c}{-}$ Val-Val-Tyr-$NH_2$

Eine Lösung von 120 mg N-(3-tert-Butoxycarbonylamino-3,3-dimethylpropanoyl)-Tyr(OMe)-Val-Cha $\stackrel{c}{-}$ Val-Val-Tyr-$NH_2$ in 1,0 ml 95%iger wässriger Trifluoressigsäure wird für 20 Min. gerührt. Das Reaktionsgemisch wird eingedampft, und der Rückstand wird durch Flash-Chromatographie an 10 g Kieselgel 60 (Laufmittel G) gereinigt. $R_f$ (L) = 0,06, $R_f$ (K) = 0,46; FAB-MS: $(M+H)^+$ = 908; $t_{Ret}$(A) = 35,6 Min.

Die Ausgangsmaterialien werden folgendermassen hergestellt:

a) N-(3-tert-Butoxycarbonylamino-3,3-dimethylpropanoyl)-Tyr(OMe)-Val-Cha $\stackrel{c}{-}$ Val-Val-Tyr-$NH_2$

wird ausgehend von 90 mg H-Tyr(OMe)-Val-Cha $\stackrel{c}{-}$ Val-Val-Tyr-$NH_2$, 31 mg 3-tert-Butoxycarbonylamino-3,3-dimethylpropionsäure, 26 mg HOBt und 34 mg DCCI analog Beispiel 1 i) hergestellt und durch Flash-Chromatographie an 8 g Kieselgel 60 (Laufmittel G) gereinigt. $R_f$ (G) = 0,30.

b) H-Tyr(OMe)-Val-Cha $\stackrel{c}{-}$ Val-Val-Tyr-$NH_2$

wird ausgehend von 260 mg HCl•H-Val-Cha $\stackrel{c}{-}$ Val-Val-Tyr-$NH_2$ (Beispiel 4a), 320 mg Fmoc-Tyr(OMe)-OTcp und 0,15 ml N-Ethyldiisopropylamin analog Beispiel 1m) und 1n) hergestellt.

c) Fmoc-Tyr(OMe)-OTcp:

Zu einer Lösung von 1,45 g Fmoc-Tyr(OMe)-OH in 30 ml abs. THF werden bei 0° 0,76 g Trichlorphenol und 0,82 g DCCI gegeben, und das Reaktionsgemisch wird für 1/2 Std. bei 0° und anschliessend für 2 Std. bei Raumtemperatur gerührt. Der DCH wird bei 0° abfiltriert, das Filtrat wird eingedampft, und der Rückstand wird aus THF-Hexan kristallisiert.

Beispiel 6: Z-Arg-Arg-Pro-Tyr(OMe)-Val-Cha $\stackrel{c}{-}$ Val-Val-Tyr-$NH_2$

Analog Beispiel 1 wird die Titelverbindung ausgehend von 150 mg H-Tyr(OMe)-Val-Cha $\stackrel{c}{-}$ Val-Val-Tyr-$NH_2$, 150 mg Z-Arg-Arg-Pro-OH•HCl, 35 mg Triethylammoniumchlorid, 50 mg HOBt und 68 mg DCCI hergestellt. $R_f$ (M) = 0,24; $t_{Ret}$(A) = 36,3 Min.

Beispiel 7: N-(2(R,S)-Benzyl-3-pivaloyl-propionyl)-Nle-Cha $\stackrel{c}{-}$ Val-Val-Tyr-OMe

Eine Lösung von 20 mg HCl•H-Nle-Cha $\stackrel{c}{-}$ Val-Val-Tyr-OMe, 22 mg (R, S)-2-Benzyl-3-pivaloylpropionsäure (J. Med. Chem. 31, 1839 (1988)), 33 mg HBTU und 24 µl Triethylamin in 2 ml DMF wird 2 Std. bei Raumtemperatur gerührt. Die Mischung wird vollständig eingedampft, der Rückstand mit ges. Natriumbicarbonat-Lösung und Diisopropylether gewaschen und schliesslich in DMF gelöst. Das Produkt wird mit Diisopropylether gefällt und ergibt nach Lyophilisation aus Dioxan/tert-Butanol/Wasser die Titelverbindung als Diastereomerengemisch. FAB-MS: $(M+H)^+$ = 891; $R_f$ (P) = 0,55; HPLC: 2 Diastereomere im Verhältnis 1,8:1, $t_{Ret}$(B) = 30,0 und 30,4 Min.

Das Ausgangsmaterial wird folgendermassen hergestellt:

a) HCl•H-Nle-Cha $\stackrel{c}{-}$ Val-Val-Tyr-OMe

wird ausgehend von 50 mg BOC-Nle-Cha $\underline{c}$ Val-Val-Tyr-OMe und 5 ml 4N HCl in Dioxan analog Beispiel 1l) hergestellt. Das Produkt wird aus Dioxan lyophilisiert. $R_f$ (P) = 0,15; $t_{Ret}$(B) = 19,9 Min.

b) BOC-Nle-Cha $\underline{c}$ Val-Val-Tyr-OMe:

Eine Lösung von 60 mg H-Cha $\underline{c}$ Val-Val-Tyr-OMe (Beispiel 1j), 28 mg BOC-Nle-OH, 46 mg HBTU und 35 µl Triethylamin in 2 ml DMF wird 3 Std. bei Raumtemperatur gerührt. Die Mischung wird eingedampft, der Rückstand mit ges. Natriumbicarbonat-Lösung und Diisopropylether gewaschen und schliesslich in DMF gelöst Das Produkt wird mit Diisopropylether gefällt und aus Dioxan/tert-Butanol/Wasser lyophilisiert. FAB-MS: $(M+H)^+$ = 761; $R_f$ (P) = 0,50; $t_{Ret}$(B) = 27,9 Min.

Beispiel 8: BBSP-Nle-Cha $\underline{c}$ Val-Val-Tyr-OMe

Analog Beispiel 7 wird die Titelverbindung ausgehend von 20 mg HCl•H-Nle-Cha $\underline{c}$ Val-Val-Tyr-OMe (Beispiel 7a), 25 mg 2(S)-Benzyl-3-tert-butylsulfonylpropionsäure (BBSP-OH), 33 mg HBTU und 24 µl Triethylamin hergestellt, durch Flash-Chromatographie an 10 g Kieselgel (Laufmittel P) gereinigt und aus Dioxan/tert-Butanol/Wasser lyophilisiert. FAB-MS: $(M+H)^+$ = 927; $R_f$ (P) = 0,45; $t_{Ret}$(B) = 28,0 Min.

Beispiel 9: BBSP-Tyr-Cha $\underline{c}$ Val-Val-Tyr-OMe

Eine Lösung von 17 mg BBSP-Tyr(OtBu)-Cha $\underline{c}$ Val-Val-Tyr-OMe in 2 ml 95 %iger wässriger Trifluoressigsäure wird 2 Std. bei Raumtemperatur gerührt. Die Mischung wird eingedampft und der Rückstand durch Chromatographie an 10 g Kieselgel (Laufmittel P) gereinigt. Die produkthaltigen Fraktionen werden eingedampft, der Rückstand in DMF gelöst und mit Diisopropylether gefällt. Die Titelverbindung wird aus Dioxan/tert-Butanol/Wasser lyophilisiert. FAB-MS: $(M+H)^+$ = 977; $R_f$ (P) = 0,40; $t_{Ret}$(B) = 25,5 Min.

Das Ausgangsmaterial wird folgendermassen hergestellt:

a) BBSP-Tyr(OtBu)-Cha $\underline{c}$ Val-Val-Tyr-OMe

wird analog Beispiel 7 aus 20 mg H-Tyr(OtBu)-Cha $\underline{c}$ Val-Val-Tyr-OMe, 22 mg 2(S)-Benzyl-3-tert-butylsulfonylpropionsäure (BBSP-OH), 30 mg HBTU und 22 µl Triethylamin hergestellt. Das Rohprodukt wird durch Flash-Chromatographie an 10 g Kieselgel (Laufmittel P) gereinigt und aus Dioxan/tert-Butanol/Wasser lyophilisiert. $R_f$ (P) = 0,55.

b) H-Tyr(OtBu)-Cha $\underline{c}$ Val-Val-Tyr-OMe:

Eine Lösung von 70 mg Fmoc-Tyr(OtBu)-Cha $\underline{c}$ Val-Val-Tyr-OMe in 2 ml Dimethylacetamid/Piperidin 30:20 (v/v) wird 30 Min. bei Raumtemperatur gerührt. Die Mischung wird eingedampft, der Rückstand in DMF gelöst und mit Diisopropylether gefällt. Der ölige Rückstand wird mit Methylenchlorid gewaschen, in DMF gelöst und bei 0° erneut mit Diisopropylether gefällt und ergibt die Titelverbindung als amorphen Festkörper. $R_f$ (P) = 0,30; $t_{Ret}$(B) = 22,3 Min.

c) Fmoc-Tyr(OtBu)-Cha $\underline{c}$ Val-Val-Tyr-OMe

wird analog Beispiel 7 ausgehend von 60 mg H-Cha $\underline{c}$ Val-Val-Tyr-OMe, 55 mg Fmoc-Tyr(OtBu)-OH, 46 mg HBTU und 35 µl Triethylamin hergestellt. Das Rohprodukt wird durch Flash-Chromatographie an 20 g Kieselgel (Laufmittel P) gereinigt. Die produkthaltigen Fraktionen werden eingedampft und der Rückstand aus DMF/Diisopropylether gefällt. $R_f$ (P) = 0,60; $t_{Ret}$(B) = 32,4 Min.

Beispiel 10: N-(2(R,S)-Benzyl-3-pivaloyl-propionyl)-Ala-Cha $\underline{c}$ Val-Val-Tyr-OMe

Eine Mischung aus 185 mg rohem H-Ala-Cha $\underline{c}$ Val-Val-Tyr-OMe, 17,5 mg 2(R,S)-Benzyl-3-pivaloylpropionsäure, 12,8 mg HOBH, 15,6 mg DCCI und 0,64 ml DMF wird 16 Std. bei Raumtemperatur gerührt. Analog Beispiel 1 wird aufgearbeitet, wobei nach Chromatographie (Kieselgel 60, Laufmittel Essigester) ein

Diastereomerengemisch der Titelverbindung erhalten wird. FAB-MS: $(M+H)^+$ = 849,7; $R_f$ (R) = 0,7; $t_{Ret}(B)$ = 27,5 und 27,9 Min.

Das Ausgangsmaterial wird folgendermassen hergestellt:

a) H-Ala-Cha $\overset{c}{-}$ Val-Val-Tyr-OMe:

Eine Lösung von 90 mg BOC-Ala-Cha $\overset{c}{-}$ Val-Val-Tyr-OMe in 2,3 ml 95 %iger wässriger Trifluoressigsäure wird 1,5 Std. bei 0° gerührt. Das Reaktionsgemisch wird im Eisbad mit wenig Wasser versetzt und die Trifluoressigsäure mit festem $NaHCO_3$, neutralisiert. Der Rückstand wird mit Essigester mehrmals gewaschen, die organischen Phasen vereinigt und über Natriumsulfat getrocknet. Nach dem Eindampfen wird die rohe Titelverbindung erhalten. $R_f$ (R) = 0.

b) BOC-Ala-Cha $\overset{c}{-}$ Val-Val-Tyr-OMe:

Eine Mischung aus 109,5 mg H-Cha $\overset{c}{-}$ Val-Val-Tyr-OMe (Beispiel 1j), 41,6 mg BOC-Ala-OH, 49,6 mg DCCI, 39,8 mg HOBH und 2 ml DMF wird 5 Std. bei Raumtemperatur gerührt. Nach Kühlen auf 0° und Filtration wird am Hochvakuum bei max. 60° das DMF abdestilliert. Der Rückstand wird in 5 ml Methylenchlorid gelöst, mit 1 ml wässriger Natriumbicarbonatlösung die organische Phase gewaschen und über Natriumsulfat getrocknet. Nach dem Eindampfen wird die rohe Titelverbindung durch Chromatographie (38 g Kieselgel 60, Laufmittel Essigester) gereinigt. FAB-MS: $(M+H)^+$ = 719; $R_f$ (R) = 0,7.

Beispiel 11: N-(2(R,S)-Benzyl-3-pivaloyl-propionyl)-Ser-Cha $\overset{c}{-}$ Val-Val-Tyr-OMe

Analog Beispiel 10 wird ein Diastereomerengemisch der Titelverbindung ausgehend von 80 mg H-Ser-Cha $\overset{c}{-}$ Val-Val-Tyr-OMe (Rohprodukt), 17,5 mg 2(R,S)-Benzyl-3-pivaloylpropionsäure, 12,8 mg HOBH und 15,8 mg DCCI in 0,64 ml DMF hergestellt. FAB-MS: $(M+H)^+$ = 865; $R_f$ (R) = 0,7; $t_{Ret}(B)$ = 26,28 und 26,71 Min.

Das Ausgangsmaterial wird folgendermassen hergestellt:

a) H-Ser-Cha $\overset{c}{-}$ Val-Val-Tyr-OMe:

Analog Beispiel 10a) wird die Titelverbindung ausgehend von 95 mg BOC-Ser-Cha $\overset{c}{-}$ Val-Val-Tyr-OMe in 2,3 ml 95%iger Trifluoressigsäure hergestellt. $R_f$ (R) = 0.

b) BOC-Ser-Cha $\overset{c}{-}$ Val-Val-Tyr-OMe:

Analog Beispiel 10b) wird die Titelverbindung ausgehend von 109,5 mg H-Cha $\overset{c}{-}$ Val-Val-Tyr-OMe, 45,1 mg BOC-Ser-OH, 39,8 mg HOBH und 49,6 mg DCCI hergestellt und durch Chromatographie gereinigt. FAB-MS: $(M+H)^+$ = 735; $R_f$ (R) = 0,65.

Beispiel 12: N-(2(R,S)-Benzyl-3-pivaloyl-propionyl)-Phe-Cha $\overset{c}{-}$ Val-Val-Tyr-OMe

48,5 mg H-Phe-Cha $\overset{c}{-}$ Val-Val-Tyr-OMe und 20,8 mg 2(R,S)-Benzyl-3-pivaloylpropionsäure werden in 2 ml DMF gelöst und mit 31,7 g BOP und 14,6 µl Triethylamin versetzt. Die Lösung wird bei Raumtemperatur für 15 Std. gerührt und danach eingedampft. Der Rückstand wird in wenig Diisopropylether digeriert. FAB-MS: $(M+H)^+$ = 925; $R_f$ (S) = 0,5; $t_{Ret}(B)$ = 30,7 und 31,1 Min.

Das Ausgangsmaterial wird folgendermassen hergestellt:

a) H-Phe-Cha $\overset{c}{-}$ Val-Val-Tyr-OMe:

Eine Lösung aus 83 mg Fmoc-Phe-Cha $\overset{c}{-}$ Val-Val-Tyr-OMe, 2,4 ml Piperidin und 2,4 ml DMF wird für

90 Min. bei Raumtemperatur gerührt und danach eingedampft. Der Rückstand wird in wenig Methylenchlorid gelöst und mit Diisopropylether gefällt. Der Niederschlag wird bei 0° abfiltriert und anschliessend aus Dioxan/Wasser 9:1 lyophilisiert. FAB-MS: $(M+H)^+$ = 695; $R_f$ (Q) = 0,36.

b) Fmoc-Phe-Cha $\overset{c}{-}$ Val-Val-Tyr-OMe:

Eine Mischung aus 50 mg H-Cha $\overset{c}{-}$ Val-Val-Tyr-OMe, 67,3 mg Fmoc-Phe-OTcp, 17 µl N-Ethyldiisopropylamin und 1,2 ml DMF wird 75 Min. bei Raumtemperatur gerührt. Das Reaktionsgemisch wird eingedampft, und der Rückstand wird mit 5 ml Diisopropylether gefällt. Der Niederschlag wird abfiltriert und ein zweites Mal aus DMF/Diisopropylether gefällt. $R_f$ (Q) = 0,37.

Beispiel 13: N-(2(R,S)-Benzyl-3-pivaloyl-propionyl)-Leu-Cha $\overset{c}{-}$ Val-Val-Tyr-OMe

35,5 mg H-Leu-Cha $\overset{c}{-}$ Val-Val-Tyr-OMe und 16 mg 2(R,S)-Benzyl-3-pivaloylpropionsäure werden in 2 ml DMF gelöst und mit 28,5 mg BOP und 11,2 µl Triethylamin versetzt. Nach 150 Min. Rühren bei Raumtemperatur wird eingeengt und der Rückstand in 20 ml Diisopropylether digeriert. FAB-MS: $(M+H)^+$ = 891; $R_f$ (Q) = 0,57; $t_{Ret}$(B) = 30,1 und 30,4 Min.

Das Ausgangsmaterial wird folgendermassen hergestellt:

a) H-Leu-Cha $\overset{c}{-}$ Val-Val-Tyr-OMe:

Eine Lösung aus 108 mg Fmoc-Leu-Cha $\overset{c}{-}$ Val-Val-Tyr-OMe, 3,2 ml Piperidin und 3,2 ml DMF wird für 60 Min. bei Raumtemperatur gerührt und danach eingedampft. Der Rückstand wird in wenig DMF gelöst und mit 20 ml Diisopropylether gefällt. Der Niederschlag wird bei 0° abfiltriert und anschliessend aus Dioxan/Wasser 9:1 lyophilisiert. FAB-MS: $(M+H)^+$ = 661; $R_f$ (Q) = 0,64.

b) Fmoc-Leu-Cha $\overset{c}{-}$ Val-Val-Tyr-OMe:

Eine Mischung aus 80 mg H-Cha $\overset{c}{-}$ Val-Val-Tyr-OMe, 101 mg Fmoc-Leu-OTcp, 27,5 µl N-Ethyldiisopropylamin und 1,4 ml DMF wird 90 Min. bei Raumtemperatur gerührt. Das Reaktionsgemisch wird eingedampft, und der Rückstand wird mit 100 ml Diisopropylether gefällt. Der Niederschlag wird abfiltriert und anschliessend aus Dioxan/Wasser 9:1 lyophilisiert. $R_f$ (Q) = 0,375.

Beispiel 14: BBSP-Leu-Cha $\overset{c}{-}$ Val-Val-Tyr-OMe

35,5 mg H-Leu-Cha $\overset{c}{-}$ Val-Val-Tyr-OMe und 18,4 mg 2(S)-Benzyl-3-tert-butylsulfonylpropionsäure (BBSP-OH) werden in 1 ml DMF gelöst und mit 28,5 mg BOP und 11,2 µl Triethylamin versetzt. Nach 5 Std. Rühren bei Raumtemperatur wird eingeengt und der Rückstand in Diisopropylether digeriert. FAB-MS: $(M+H)^+$ = 927,5; $R_f$ (Q) = 0,54; $t_{Ret}$(B) = 28,0 Min.

Beispiel 15: BBSP-Leu-Cha $\overset{c}{-}$ Val-Val-p-biphenylylmethylamid

40 mg H-Leu-Cha $\overset{c}{-}$ Val-Val-p-biphenylylmethylamid und 18,4 mg BBSP-OH werden in 1 ml DMF gelöst und mit 28,5 mg BOP und 11 µl Triethylamin versetzt. Nach 5 Std. Rühren bei Raumtemperatur wird eingeengt und der Rückstand in Diisopropylether digeriert. FAB-MS: $(M+H)^+$ = 916; $R_f$ (Q) = 0,58.

Das Ausgangsmaterial wird folgendermassen hergestellt:

a) H-Leu-Cha $\overset{c}{-}$ Val-Val-p-biphenylylmethylamid:

Eine Lösung aus 108 mg Fmoc-Leu-Cha $\overset{c}{-}$ Val-Val-p-biphenylylmethylamid, 3 ml Piperidin und 3 ml DMF wird für 60 Min. bei Raumtemperatur gerührt und danach eingedampft. Der Rückstand wird in wenig

DMF gelöst und mit 20 ml Diisopropylether gefällt. Der Niederschlag wird bei 0° abfiltriert und anschliessend aus Dioxan/Wasser 9:1 lyophilisiert. FAB-MS: $(M+H)^+$ - 649; $R_f$ (Q) = 0,72.

b) Fmoc-Leu-Cha $\stackrel{c}{-}$ Val-Val-p-biphenylylmethylamid:

Eine Mischung aus 100 mg H-Cha $\stackrel{c}{-}$ Val-Val-p-biphenylylmethylamid, 120 mg Fmoc-Leu-OTcp, 35 µl N-Ethyldiisopropylamin und 2 ml DMF wird 90 Min. bei Raumtemperatur gerührt. Das Reaktionsgemisch wird eingedampft, und der Rückstand wird mit 100 ml Diisopropylether gefällt. Der Niederschlag wird abfiltriert und anschliessend aus Dioxan/Wasser 9:1 lyophilisiert. $R_f$ (Q) = 0,41.

c) H-Cha $\stackrel{c}{-}$ Val-Val-p-biphenylylmethylamid:

290 mg N-(5(S)-Azido-6-cyclohexyl-4(S)-hydroxy-2(S)-isopropylhexanoyl)-Val-p-biphenylylmethylamid werden in 40 ml Methanol gelöst, mit 70 mg Palladiumkohle (5 % Pd) versetzt und mit Wasserstoff reduziert. Nach 2 Std. bei Raumtemperatur wird vom Katalysator abfiltriert, die Lösung eingeengt und der Rückstand gefällt. FAB-MS: $(M+H)^+$ = 536; $R_f$ (T) = 0,23.

d) N-(5(S)-Azido-6-cyclohexyl-4(S)-hydroxy-2(S)-isopropylhexanoyl)-Val-p-biphenylylmethylamid:

386 mg N-(5(S)-Azido-6-cyclohexyl-4(S)-tert-butyldimethylsilyloxy-2(S)-isopropyl-hexanoyl)-Val-p-biphenylylmethylamid in 5 ml DMF werden mit 360 mg Tetrabutylammoniumfluorid versetzt. Nach 4 Std. wird die Lösung eingeengt und der Rückstand in ca. 100 ml Essig ester aufgenommen und mit Natriumbicarbonatlösung gewaschen. Nach Einengen der Lösung wird der Rückstand aus Methylenchlorid/Hexan kristallisiert. FAB-MS: $(M+H)^+$ = 562; $R_f$ (U) = 0,12.

e) N-(5(S)-Azido-6-cyclohexyl-4(S)-tert-butyldimethylsilyloxy-2(S)-isopropyl-hexanoyl)-Val-p-biphenylylmethylamid:

376 mg 5(S)-Azido-6-cyclohexyl-4(S)-tert-butyldimethylsilyloxy-2(S)-isopropyl-hexansäure (J. Org. Chem. 54, 1178 (1989)) und 250 mg Valin-p-biphenylylmethylamid werden in 10 ml DMF gelöst und mit 404 mg BOP und 0,16 ml Triethylamin versetzt. Nach 16 Std. bei Raumtemperatur wird eingeengt, und der Rückstand mit Diisopropylether digeriert. FAB-MS: $(M+H)^+$ = 676; $R_f$ (U) = 0,45.

f) Valin-p-biphenylylmethylamid:

980 mg BOC-Val-p-biphenylylmethylamid werden in 10 ml Dioxan/4N HCl während 30 Min. bei Raumtemperatur stehen gelassen. Anschliessend wird eingeengt und zwischen Essigester und 1N NaOH verteilt. Die organische Phase wird eingeengt und der Rückstand an Kieselgel chromatographiert. $R_f$ (F) = 0,4.

g) BOC-Val-p-biphenylylmethylamid:

3,9 g N-tert-Butoxycarbonyl-L-valinhydroxysuccinimidester und 2,7 g Biphenylylmethylamin werden in 150 ml Methylenchlorid bei Raumtemperatur während 1 Std. gerührt. Die Mischung wird durch Kieselgel filtriert und eingedampft, und der Rückstand aus Hexan kristallisiert. FAB-MS: $(M+H)^+$ = 383; $R_f$ (U) - 0,28.

Beispiel 16: Gelatine-Lösung

Eine sterilfiltrierte wässrige Lösung von Z-Arg-Arg-Pro-Phe-Val-Cha $\stackrel{c}{-}$ Val-Val-Tyr-OMe wird unter Erwärmen mit einer sterilen Gelatinelösung, welche als Konservierungsmittel Phenol enthält, unter aseptischen Bedingungen so vermischt, dass 1,0 ml Lösung folgende Zusammensetzung hat:

| | |
|---|---|
| Z-Arg-Arg-Pro-Phe-Val-Cha $\stackrel{c}{-}$ Val-Val-Tyr--OMe | 3 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

Beispiel 17: Sterile Trockensubstanz zur Injektion

Man löst 5 mg Z-Arg-Arg-Pro-Phe-Val-Cha $\stackrel{c}{-}$ Val-Val-Tyr-OMe in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor dem Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel 18: Nasenspray

In einer Mischung von 3,5 ml Myglyol 812® und 0,08 g Benzylalkohol werden 500 mg fein gemahlenes (<5,0 µm) BBSP-Val-Cha $\stackrel{c}{-}$ Val-Val-Tyr-OMe suspendiert. Diese Suspension wird in einen Behälter mit Dosierventil eingefüllt. Es werden 5,0 g Freon® 12 unter Druck durch das Ventil in den Behälter abgefüllt. Durch Schütteln wird das Freon® in der Myglyol-Benzylalkoholmischung gelöst. Dieser Spraybehälter enthält ca. 100 Einzeldosen, die einzeln appliziert werden können.

Beispiel 19: Lacktabletten

Für die Herstellung von 10 000 Tabletten enthaltend je 100 mg Wirkstoff werden folgende Bestandteile verarbeitet:

| | |
|---|---|
| BBSP-Val-Cha $\stackrel{c}{-}$ Val-Val-Tyr--OMe | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | q.s. |

Ein Gemisch des BBSP-Val-Cha $\stackrel{c}{-}$ Val-Val-Tyr-OMe, 50 g Maisstärke und der kolloidalen Kieselsäure wird mit Stärkekleister aus 250 g Maisstärke und 2,2 kg entmineralisiertem Wasser zu einer feuchten Masse verarbeitet.

Diese wird durch ein Sieb von 3 mm Maschenweite getrieben und bei 45° während 30 Min. im Wirbelschichttrockner getrocknet. Das trockene Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

Die Presslinge werden in einem Dragierkessel von 45 cm Durchmesser mit einer Lösung von 20 g Schellack und 40 g Hydroxypropylmethylcellulose (niedere Viskosität) in 110 g Methanol und 1350 g Methylenchlorid durch gleichmässiges Aufsprühen während 30 Min. überzogen; dabei wird durch gleichzeitiges Einblasen von Luft von 60° getrocknet.

Anstelle der in den Beispielen 16-19 genannten Wirkstoffe kann man in diesen Beispielen auch dieselbe

Menge eines anderen Wirkstoffes der vorangehenden Beispiele verwenden.

Beispiel 20: Hemmung der isolierten HIV-1 gag-Protease

10 µl einer Lösung von HIV-1 gag-Protease (Aceton-Extrakt einer in E. coli exprimierten gag-Protease gemäss J. Hansen et al., The EMBO Journal 7, 1785 (1988)) und 190 µl β-Morpholinoethansulfonsäure-Pufferlösung pH 6 enthaltend 0,01 % 2-Amino-4-nitrophenol als internem Standard werden bei 37° vorinkubiert. Dann werden gleichzeitig 10 µl einer 0,24 mM DMSO-Lösung des Substrats H-Arg-Arg-Ser-Asn-Gln-Val-Ser-Gln-Asn-Tyr-Pro-Ile-Val-Gln-Asn-Ile-Gln-Gly-Arg-Arg-OH (Icosapeptid gemäss J. Schneider et al., Cell 54, 363 (1988), hergestellt auf einem automatisierten Peptidsynthesegerät mit Fmoc-geschützten Aminosäurebausteinen) und 10 µl einer DMSO-Lösung der auf Hemmwirkung zu untersuchenden Verbindung in einer Konzentration von 22 x $10^{-4}$M oder 22 x $10^{-6}$M zugegeben. Nach einer Stunde werden 50 µl Reaktionslösung entnommen, mit 5 µl 0,3 M Perchlorsäure versetzt und zentrifugiert. Die Menge an unverbrauchtem Substrat und die Spaltprodukte in der überstehenden Lösung werden mit HPLC bestimmt und daraus die prozentuale Hemmung bei $10^{-4}$M und $10^{-6}$M berechnet.

Für die HPLC-Analyse wird eine 125 x 4,6 mm reversed phase $C_{18}$ Nucleosil® 5 µ Säule verwendet; Gradient 10 % Acetonitril/0,1 % Trifluoressigsäure in Wasser → 25 % Acetonitril/0,08 % Trifluoressigsäure in Wasser in 30 Min., Durchflussrate 1,5 ml/Min.

Die Verbindungen der vorstehenden Beispiele weisen bei Konzentrationen von $10^{-4}$M eine Hemmwirkung von über 80 % und bei $10^{-6}$M eine Hemmwirkung von über 20 %, im Regelfall von über 50 %, auf.

Beispiel 21: Schutz gegen HIV-Infektion im Zelltest

Die verwendete Zellinie MT-2 ist eine menschliche T-Zell-Leukämie, die mit HTLV-1 transformiert ist und HTLV-1 kontinuierlich produziert, was die Zellen extrem empfindlich für den zytopathogenen Effekt von HIV macht (Science 229, 563 (1985)). Die MT-2 Zellen werden in RPMI 1640 Medium enthaltend 12 % hitze-inaktiviertes fötales Kälberserum (Seromed Biochrom KG, Berlin, BRD), Glutamin und Standard-Antibiotika kultiviert. Die Zellen werden bei 37° in einer befeuchteten Atmosphäre von 5 % $CO_2$ in Luft gehalten und in der logarithmischen Wachstumsphase für den Zelltest verwendet.

HIV LAV-03 (AIDS Research and Reference Reagent Program, NIH, Bethesda, MD, USA) wird in A 3.01-Zellen gezüchtet. Der Titer wird im Reverse Transcriptase Assay bestimmt. Für die verwendete Charge beträgt er 2 x $10^7$ IU/ml.

40'000 exponentiell wachsende MT-2 Zellen in 50 µl Kulturmedium werden in jede der Vertiefungen einer 96 Rundboden-Titerplatte gegeben. Die zu untersuchenden Verbindungen werden in vorgegebener Konzentration in 50 µl Kulturmedium beigefügt, und unmittelbar danach HIV in 100 µl Kulturmedium zugegeben. Zu Vergleichsproben wird 100 µl Kulturmedium ohne HIV zugegeben. Die Titerplatten werden sechs Tage inkubiert. Danach wird die Lebensfähigkeit der HIV-infizierten und der Vergleichs-Zellen im MTT-Assay geprüft: Der MTT-Assay beruht auf der Reduktion von gelbgefärbtem 3-(4,5-dimethylthiazol-2-yl)-2,5-diphenyltetrazolium-bromid (MTT, Sigma, St.Louis, USA) durch mytochondrische Dehydrogenasen von metabolisch aktiven Zellen zu einem blauen Formazan, das spektrophotometrisch bei 540 nm gemessen werden kann (J. Virological Methods 20, 309 (1988)). Die Lebensfähigkeit von Vergleichszellen und HIV-infizierten Zellen wird ebenfalls mikroskopisch in einem Hämozytometer nach der Trypanblau-Ausschlussmethode bestimmt.

Die untersuchten Verbindungen der vorstehenden Beispiele weisen bei Konzentrationen von $10^{-5}$ Mol/l Schutzwirkung gegen HIV-Infektion auf.

## Ansprüche

1. Verbindungen der Formel

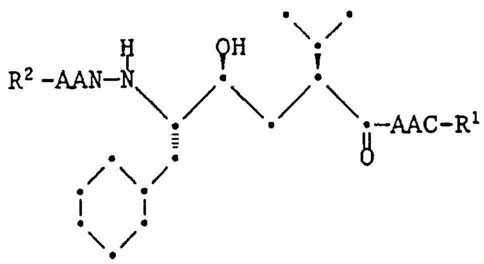

(I),

worin AAN ein bivalentes Radikal bestehend aus einem bis fünf bivalenten α-Aminosäureresten bedeutet, welches N-terminal mit dem Rest $R^2$ und C-terminal mit der Gruppe NH- verbunden ist, AAC ein bivalentes Radikal bestehend aus einem oder zwei bivalenten α-Aminosäureresten bedeutet, welches N-terminal mit der Gruppe -C=O und C-terminal mit dem Rest $R^1$ verbunden ist, $R^1$ für Hydroxy, verethertes Hydroxy, Amino oder substituiertes Amino mit Ausnahme eines von einer α-Aminosäure abgeleiteten Aminorestes steht, und $R^2$ Wasserstoff oder einen Acylrest mit Ausnahme eines gegebenenfalls N-substituierten Acylrestes einer natürlichen Aminosäure bedeutet, ferner Salze von solchen Verbindungen mit salzbildenden Gruppen.

2. Verbindungen gemäss Anspruch 1, dadurch gekennzeichnet, dass sie im Radikal AAN zwei oder mehr α-Aminosäurereste aufweisen oder dass das Radikal AAN aus nur einem α-Aminosäurerest besteht und gleichzeitig der Rest $R^2$ ein Analogon zu Phenylalanyl (H-Phe) ist und/oder dass sie im Radikal AAC zwei α-Aminsosäurereste aufweisen oder dass das Radikal AAC aus nur einem α-Aminosäurerest besteht und gleichzeitig der Rest $R^1$ ein Analogon zum über Stickstoff gebundenen Rest der Aminosäure Tyrosin (-Tyr-OH) ist, sowie Salze davon.

3. Verbindungen gemäss Anspruch 1 der Formel

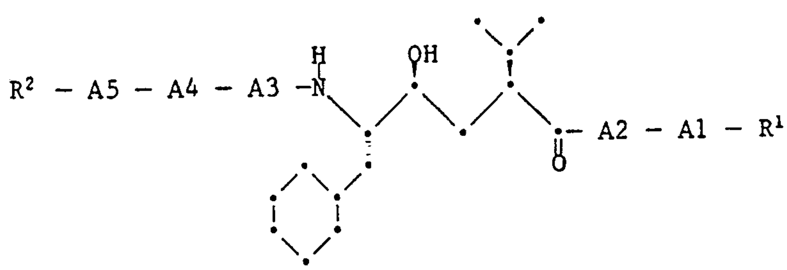

(II),

worin die Radikale A1, A2, A3 und A4 je einen bivalenten α-Aminosäurerest bedeuten, der N-terminal mit dem in Formel II jeweils links davon stehenden Radikal und C-terminal mit dem jeweils rechts davon stehenden Radikal bzw. mit dem Rest $R^1$ verbunden ist, A5 für eine einfache Bindung steht oder einen bivalenten Rest bestehend aus bis zu drei peptidisch verknüpften α-Aminosäuren bedeutet, welcher N-terminal mit $R^2$ und C-terminal mit A4 verbunden ist, und $R^1$ und $R^2$ die in Anspruch 1 für Formel I angegebenen Bedeutungen aufweisen, sowie Salze davon.

4. Verbindungen gemäss Anspruch 3 der Formel II, worin A1 ausgewählt ist unter den bivalenten Resten von Tyrosin, Phenylalanin, Naphthylalanin, Tryptophan, Lysin und Asparaginsäure, A2 ausgewählt ist unter den bivalenten Resten von Valin, Isoleucin, Leucin, Norleucin, Phenylalanin, Alanin und Glycin, A3 ausgewählt ist under den bivalenten Resten von Valin, Alanin, Leucin, Isoleucin, Asparagin, Glutamin, Norleucin, Phenylalanin, Serin und Histidin, A4 ausgewählt ist unter den bivalenten Resten von Phenylalanin, Tyrosin, mit Niederalkyl verethertem Tyrosin, Tryptophan, Cyclohexylalanin, Leucin, Naphthylalanin, Histidin, Asparaginsäure und Lysin, und A5, $R^1$ und $R^2$ die in Anspruch 3 angegebenen Bedeutungen aufweisen, sowie Salze davon.

5. Verbindungen gemäss Anspruch 4, worin A5 für eine einfache Bindung steht oder einen bivalenten Rest bestehend aus bis zu drei gleichen oder verschiedenen, peptidisch verknüpften α-Aminosäuren ausgewählt unter den bivalenten Resten von Arginin, Prolin, Histidin, Lysin, Ornithin oder Tryptophan bedeutet, $R^1$ für Hydroxy, Niederalkoxy, Amino oder Mono- oder Diniederalkylamino steht, und $R^2$ Wasserstoff, Niederalkanoyl, Niederalkoxycarbonyl, Arylniederalkoxycarbonyl oder einen Rest der Formel

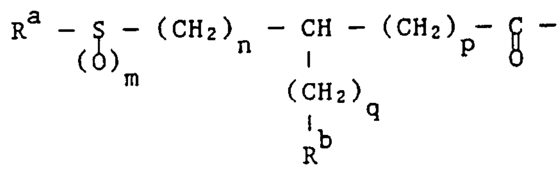 (Ia),

worin $R^a$ unsubstituiertes oder durch Hydroxy oder Niederalkoxy substituiertes Niederalkyl, Phenyl, Benzyl oder unsubstituiertes oder durch Oxido oder Niederalkyl substituiertes Heteroaryl mit 1 oder 2 Stickstoffatomen, $R^b$ Cyclohexyl oder Phenyl, m 0, 1 oder 2, n 1, p 0 und q 1 oder 2 darstellen, bedeutet, sowie Salze davon.

6. Verbindungen gemäss Anspruch 5, worin A5 für eine einfache Bindung oder den bivalenten Rest Arginin-Arginin-Prolin- steht, sowie Salze davon.

7. Verbindungen gemäss Anspruch 3 der Formel II, worin A1 ausgewählt ist unter den bivalenten Resten von Tyrosin, Phenylalanin, Tryptophan, Naphthylalanin, Lysin und Asparaginsäure, A2 und A3 ausgewählt sind unter den bivalenten Resten von Valin, Isoleucin, Leucin, Norleucin und Alanin, A4 den bivalenten Rest von Phenylalanin, Tyrosin oder Naphthylalanin bedeutet, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Niederalkoxy substituiertes Phenylniederalkoxycarbonyl, Diphenylniederalkoxycarbonyl oder Fluorenylniederalkoxycarbonyl bedeutet, sowie Salze davon.

8. Verbindungen gemäss Anspruch 3 der Formel II, worin A1 den bivalenten Rest von Tyrosin bedeutet, A2 ausgewählt ist unter den bivalenten Resten von Valin, Isoleucin, Norleucin, Leucin, Phenylalanin, Alanin und Glycin, A3 den bivalenten Rest von Valin bedeutet, A4 den bivalenten Rest von Phenylalanin, Tyrosin oder Naphthylalanin bedeutet, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Niederalkoxy substituiertes Phenylniederalkoxycarbonyl, Diphenylniederalkoxycarbonyl oder Fluorenylniederalkoxycarbonyl bedeutet, sowie Salze davon.

9. Verbindungen gemäss Anspruch 3 der Formel II, worin A1 den bivalenten Rest von Tyrosin bedeutet, A2 für den bivalenten Rest von Valin oder Leucin steht, A3 ausgewählt ist unter den bivalenten Resten von Valin, Leucin, Isoleucin, Norleucin, Alanin, Asparagin, Glutamin, Phenylalanin, Serin und Histidin, A4 den bivalenten Rest von Phenylalanin bedeutet, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Niederalkoxy substituiertes Phenylniederalkoxycarbonyl, Diphenylniederalkoxycarbonyl oder Fluorenylniederalkoxycarbonyl bedeutet, sowie Salze davon.

10. Verbindungen gemäss Anspruch 3 der Formel II, worin A1 den bivalenten Rest von Tyrosin bedeutet, A2 für den bivalenten Rest von Valin oder Leucin steht, A3 für den bivalenten Rest von Valin steht, A4 ausgewählt ist unter den bivalenten Resten von Phenylalanin, Tyrosin, mit Methyl verethertem Tyrosin, Tryptophan, Cyclohexylalanin, Naphthylalanin und Lysin, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Niederalkoxy substituiertes Phenylniederalkoxycarbonyl, Diphenylniederalkoxycarbonyl oder Fluorenylniederalkoxycarbonyl bedeutet, sowie Salze davon.

11. Verbindungen gemäss Anspruch 1 der Formel

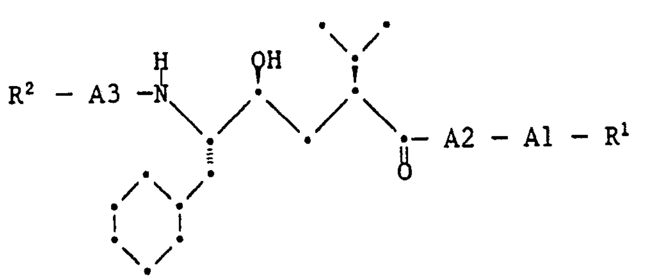 (III),

worin die Radikale A1, A2 und A3 je einen bivalenten α-Aminosäurerest bedeuten, der N-terminal mit dem in Formel III jeweils links davon stehenden Radikal bzw. mit dem Rest $R^2$ und C-terminal mit dem jeweils rechts davon stehenden Radikal bzw. mit dem Rest $R^1$ verbunden ist, und $R^1$ und $R^2$ die in Anspruch 1 für Formel I angegebenen Bedeutungen aufweisen, sowie Salze davon.

12. Verbindungen gemäss Anspruch 11 der Formel III, worin A1 ausgewählt ist unter den bivalenten Resten von Tyrosin, Phenylalanin, Tryptophan, α-Naphthylalanin, Lysin und Asparaginsäure, A2 ausgewählt ist under den bivalenten Resten von Valin, Isoleucin, Leucin, Norleucin, Phenylalanin, Alanin und Glycin, A3 ausgewählt ist unter den bivalenten Resten von Valin, Alanin, Leucin, Isoleucin, Asparagin, Glutamin, Norleucin, Phenylalanin, Serin und Histidin, und $R^1$ und $R^2$ die in Anspruch 11 angegebenen Bedeutungen aufweisen, sowie Salze davon.

13. Verbindungen gemäss Anspruch 11 der Formel III worin A1 ausgewählt ist unter den bivalenten Resten von Tyrosin, Phenylalanin, Tryptophan, α-Naphthylalanin, Lysin und Asparaginsäure, A2 ausgewählt ist unter den bivalenten Resten von Valin, Isoleucin, Leucin, Norleucin, Phenylalanin, Alanin und Glycin, A3 der bivalente Rest von Tyrosin oder von mit Niederalkyl veräthertem Tyrosin ist, und $R^1$ und $R^2$ die in Anspruch 11 angegebenen Bedeutungen aufweisten, sowie Salze davon.

14. Verbindungen gemäss Anspruch 12 oder 13, worin $R^1$ für Hydroxy, Niederalkoxy, Amino oder Mono- oder Diniederalkylamino steht, und $R^2$ einen Rest der Formel

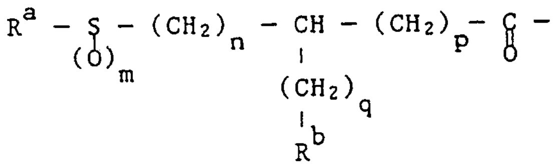

(Ia)

bedeutet, worin $R^a$ unsubstituiertes oder durch Hydroxy oder Niederalkoxy substituiertes Niederalkyl, Phenyl, Benzyl oder unsubstituiertes oder durch Oxido oder Niederalkyl substituiertes Heteroaryl mit 1 oder 2 Stickstoffatomen, $R^b$ Cyclohexyl oder Phenyl, m 0, 1 oder 2, n 1, p 0 und q 1 oder 2 darstellen, sowie Salze davon.

15. Verbindungen gemäss Anspruch 12 oder 13, worin $R^1$ für Hydroxy, Niederalkoxy, Amino oder Mono- oder Diniederalkylamino steht, und $R^2$ einen Rest mit dem Strukturelement der Formel

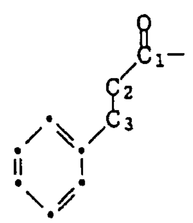

(Ib)

darstellt, worin der carbocyclische Ring auch ganz oder teilweise gesättigt sein kann, eines der Kohlenstoffatome des carbocyclischen Ringes mit $C_2$ oder $C_3$ zu einem fünf- oder sechsgliedrigen Ring verknüpft sein kann, eines der Kohlenstoffatome $C_2$ oder $C_3$ durch ein Heteroatom NH, O oder S ersetzt sein kann, die Carbonylgruppe eine Heterocarbonylgruppe P=O sein kann und freie Valenzen Wasserstoff oder einen Substituenten Niederalkyl, Niederalkylthioniederalkyl, Niederalkylsulfinylniederalkyl, Niederalkylsulfonylniederalkyl, Phenylniederalkyl, Hydroxy, Niederalkoxy, Phenylniederalkoxy, Aminoniederalkyl, Niederalkylaminoniederalkyl, Niederalkanoylaminoniederalkyl, Niederalkanoyl, Niederalkanoylniederalkyl oder Niederalkylcarbamoyl tragen, darstellt, sowie Salze davon.

16. Verbindungen gemäss Anspruch 14 der Formel III, worin A1 ausgewählt ist unter den bivalenten Resten von Tyrosin, Phenylalanin, Tryptophan, Naphthylalanin, Lysin und Asparaginsäure, A2 und A3 den bivalenten Rest von Valin oder Leucin bedeuten, $R^1$ für Hydroxy, Niederalkoxy, Niederalkylamino oder Amino steht, und $R^2$ einen Rest der Formel Ia bedeutet, worin $R^a$ für Niederalkyl steht, $R^b$ Phenyl bedeutet und m zwei, n eins, p null und q eins bedeuten, sowie Salze davon.

17. Verbindungen gemäss Anspruch 14 der Formel III, worin A1 den bivalenten Rest von Tyrosin bedeutet, A2 ausgewählt ist unter den bivalenten Resten von Valin, Isoleucin, Leucin, Norleucin, Phenylalanin, Alanin und Glycin, A3 den bivalenten Rest von Valin oder Leucin bedeutet, $R^1$ für Hydroxy, Niederalkoxy, Niederalkylamino oder Amino steht, und $R^2$ einen Rest der Formel Ia bedeutet, worin $R^a$ für Niederalkyl steht, $R^b$ Phenyl bedeutet und m zwei, n eins, p null und q eins bedeuten, sowie Salze davon.

18. Verbindungen gemäss Anspruch 14 der Formel III, worin A1 den bivalenten Rest von Tyrosin bedeutet, A2 für den bivalenten Rest von Valin oder Leucin steht, A3 ausgewählt ist unter den bivalenten Resten von Valin, Alanin, Leucin, Isoleucin, Asparagin, Glutamin, Norleucin, Phenylalanin, Serin und Histidin,

$R^1$ für Hydroxy, Niederalkoxy, Niederalkylamino oder Amino steht, und $R^2$ einen Rest der Formel Ia bedeutet, worin $R^a$ für Niederalkyl steht, $R^b$ Phenyl bedeutet und m zwei, n eins, p null und q eins bedeuten, sowie Salze davon.

19. Verbindungen gemäss Anspruch 15 der Formel III, worin A1 den bivalenten Rest von Tyrosin bedeutet, A2 den bivalenten Rest von Valin bedeutet, A3 ausgewählt ist unter den bivalenten Resten von Valin, Leucin, Norleucin, Phenylalanin, Alanin, Serin, Tyrosin und mit Niederalkyl verethertem Tyrosin, $R^1$ für Hydroxy, Niederalkoxy, Niederalkylamino oder Amino steht, und $R^2$ 3-Phenyl- oder 3-Cyclohexyl-propionyl bedeutet, das in 2-Stellung durch Niederalkylsulfonylniederalkyl oder durch Niederalkanoylniederalkyl substituiert ist, sowie Salze davon.

20. Verbindungen gemäss Anspruch 1 der Formel IV,

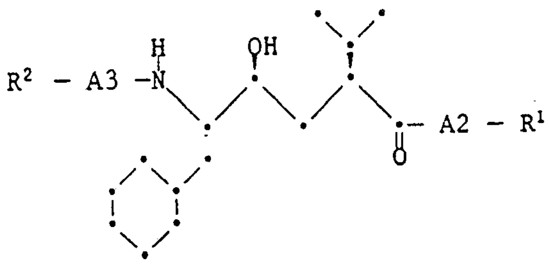

(IV)

worin A3 einen bivalenten α-Aminosäurerest bedeutet, der N-terminal mit dem Rest $R^2$ und C-terminal mit der Gruppe NH- verbunden ist, A2 einen bivalenten α-Aminosäurerest bedeutet, der N-terminal mit der Gruppe -C=O und C-terminal mit dem Rest $R^1$ verbunden ist, $R^1$ Niederalkylamino oder einen Rest mit dem Strukturelement der Formel

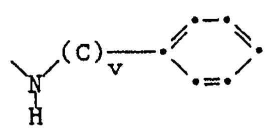

(Ic)

bedeutet, worin der carbocyclische Ring ein Stickstoffatom enthalten kann und auch ganz oder teilweise gesättigt und/oder durch Hydroxy oder durch Phenyl substituiert sein kann, der Index v für null bis fünf steht, eines der Kohlenstoffatome des carbocyclischen Ringes mit einem der Atome $-(C)_v-$ zu einem fünf- oder sechsgliedrigen Ring verbunden sein kann und freie Valenzen Wasserstoff oder einen Substituenten Niederalkyl oder Hydroxyniederalkyl tragen, und $R^2$ einen Rest mit dem Strukturelement der Formel

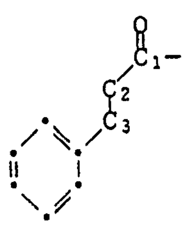

(Ib)

darstellt, worin der carbocyclische Ring auch ganz oder teilweise gesättigt sein kann, eines der Kohlenstoffatome des carbocyclischen Ringes mit $C_2$ oder $C_3$ zu einem fünf- oder sechsgliedrigen Ring verknüpft sein kann, eines der Kohlenstoffatome $C_2$ oder $C_3$ durch ein Heteroatom NH, O oder S ersetzt sein kann, die Carbonylgruppe eine Heterocarbonylgruppe P=O sein kann und freie Valenzen Wasserstoff oder einen Substituenten Niederalkyl, Niederalkylthioniederalkyl, Niederalkylsulfinylniederalkyl, Niederalkylsulfonylniederalkyl, Phenylniederalkyl, Hydroxy, Niederalkoxy, Phenylniederalkoxy, Aminoniederalkyl, Niederalkylaminoniederalkyl, Niederalkanoylaminoniederalkyl, Niederalkanoyl, Niederalkanoylniederalkyl oder Niederalkylcarbamoyl tragen, sowie Salze davon.

21. Verbindungen gemäss Anspruch 20, worin A2 ausgewählt ist unter den bivalenten Resten von Valin, Isoleucin, Norleucin, Leucin, Phenylalanin, Alanin und Glycin und A3 ausgewählt ist unter den bivalenten Resten von Valin, Alanin, Leucin, Asparagin, Glutamin, Norleucin, Phenylalanin, Serin und Histidin, sowie

Salze davon.

22. Verbindungen gemäss Anspruch 20, worin A2 und A3 unabhängig voneinander jeweils den bivalenten Rest von Valin oder Leucin bedeuten, sowie Salze davon.

23. Verbindungen gemäss Anspruch 20, worin A2 den bivalenten Rest von Valin bedeutet, A3 ausgewählt ist unter den bivalenten Resten von Valin, Leucin, Norleucin, Phenylalanin, Alanin, Serin, Tyrosin und mit Niederalkyl verethertem Tyrosin, $R^1$ Niederalkylamino oder ein Rest mit dem Strukturelement der Formel Ic ist, worin der carbocyclische Ring unsubstituiert oder durch Hydroxy oder durch Phenyl substituiert sein kann, der Index v für null bis fünf steht und freie Valenzen Wasserstoff tragen, und $R^2$ 3-Phenyl- oder 3-Cyclohexyl-propionyl bedeutet, das in 2-Stellung durch Niederalkylsulfonylniederalkyl oder durch Niederalkanoylniederalkyl substituiert ist, sowie Salze davon.

24. Die Verbindung gemäss Anspruch 11, worin A1 den bivalenten Rest von Tyrosin, A2 und A3 jeweils den bivalenten Rest von Valin, $R^1$ Methoxy und $R^2$ 2(S)-Benzyl-3-tert-butylsulfonylpropionyl bedeuten.

25. Pharmazeutische Präparate enthaltend Verbindungen der Ansprüche 1 bis 24 oder ein pharmazeutisch annehmbares Salz davon zusammen mit einem pharmazeutisch geeigneten Trägermaterial.

26. Verwendung der in den Ansprüchen 1 bis 24 genannten Verbindungen als retrovirale Proteasehemmer.

27. Verwendung der in den Ansprüchen 1 bis 24 genannten Verbindungen zur Herstellung von pharmazeutischen Präparaten zur Bekämpfung von durch Retroviren verursachten Krankheiten.

28. Verfahren zur Herstellung von den in Anspruch 1 genannten Verbindungen der Formel I, in der alle Symbole die in Anspruch 1 angegebenen Bedeutungen haben, und deren Salzen, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene funktionelle Gruppe mit Ausnahme der an der Reaktion teilnehmenden Gruppe gegebenenfalls in geschützter Form vorliegen, unter Bildung einer Amidbindung kondensiert, oder

b) die Ketogruppe in einer Verbindung der Formel

$$R^2 - AAN - \overset{H}{\overset{|}{N}} - \underset{\underset{CH_2 - C_6H_{11}}{|}}{CH} - \overset{O}{\overset{\|}{C}} - CH_2 - \overset{CH(CH_3)_2}{\overset{|}{CH}} - \overset{O}{\overset{\|}{C}} - AAC - R^1 \qquad (V),$$

worin die Symbole die genannten Bedeutungen haben und funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) eine Aldehyd-Verbindung der Formel

$$R^2 - AAN - \overset{H}{\overset{|}{N}} - \underset{\underset{CH_2 - C_6H_{11}}{|}}{CH} - \overset{O}{\overset{\|}{C}} - H \qquad (VI),$$

worin die Symbole die genannten Bedeutungen haben und funktionelle Gruppen mit Ausnahme der Aldehydgruppe gegebenenfalls in geschützter Form vorliegen, mit einer Organometallverbindung der Formel

$$M - CH_2 - \overset{CH(CH_3)_2}{\overset{|}{CH}} - \overset{O}{\overset{\|}{C}} - AAC - R^1 \qquad (VII),$$

worin die Symbole die genannten Bedeutungen haben und M ein Metallradikal bedeutet, umsetzt und das gebildete Additionsprodukt hydrolysiert, oder

d) in einer Verbindung der Formel

$$R^2-AAN-\overset{H}{N}-\underset{CH_2-C_6H_{11}}{CH}-\overset{X}{CH}-CH_2-\overset{CH(CH_3)_2}{CH}\text{———}\overset{O}{\overset{\|}{C}}-AAC-R^1 \quad (VIII),$$

worin X eine nukleofuge Abgangsgruppe ist, die übrigen Symbole die oben genannten Bedeutungen haben und funktionelle Gruppen gegebenenfalls in geschützter Form vorliegen, den Substituenten X durch eine Hydroxygruppe austauscht, oder

e) in einer Verbindung der Formel

$$R^2-AAN-\overset{H}{N}-\underset{CH_2-C_6H_{11}}{CH}-\overset{OH}{CH}-CH_2-\overset{CH(CH_3)_2}{CH}\text{———}\overset{O}{\overset{\|}{C}}-AAC'-CN \quad (IX),$$

worin die Symbole die genannten Bedeutungen haben und AAC′ die bedeutung von AAC abzüglich der terminalen Carbonylgruppe hat und vorhandene funktionelle Gruppen gegebenenfalls in geschützter Form vorliegen, die Gruppe AAC′-CN in eine Gruppe AAC-$R^1$ überführt, oder

f) in einer Verbindung der Formel

$$R^2-AAN-\overset{H}{N}-\underset{CH_2-C_6H_{11}}{CH}-\overset{O}{CH-CH}-\overset{CH(CH_3)_2}{CH}\text{———}\overset{O}{\overset{\|}{C}}-AAC-R^1 \quad (X),$$

worin der Symbole die genannten Bedeutungen haben und funktionelle Gruppen gegebenenfalls in geschützter Form vorliegen, mit einem regioselektiven Reduktionsmittel die Epoxygruppe zu einer Hydroxygruppe reduziert, oder

g) zur Herstellung einer Verbindung der Formel I, worin $R^2$ einen Rest der Formel

$$R^a-\underset{(O)_m}{S}-(CH_2)_n-\underset{\underset{R^b}{|}}{\underset{(CH_2)_q}{\underset{|}{CH}}}-(CH_2)_p-\underset{O}{\overset{}{\underset{\|}{C}}}- \quad (Ia)$$

und m 0 oder 2, n 1 und p 0 bedeuten, eine Verbindung der Formel $R^a$-S(O)$_m$H oder ein Salz davon an eine Verbindung der Formel

$$R^b-(CH_2)_q-\underset{CH_2}{\overset{}{\underset{\|}{C}}}-\overset{O}{\overset{\|}{C}}-AAN-\overset{H}{N}-\underset{CH_2-C_6H_{11}}{CH}-\overset{OH}{CH}-CH_2-\overset{CH(CH_3)_2}{CH}\text{———}\overset{O}{\overset{\|}{C}}-AAC-R^1 \quad (XI),$$

worin die Symbole die genannten Bedeutungen haben und funktionelle Gruppen gegebenenfalls in geschützter Form vorliegen, addiert, oder

h) zur Herstellung einer Verbindung der Formel I, worin $R^2$ einen Rest der Formel

$$R^a-\underset{(O)_m}{S}-(CH_2)_n-\underset{\underset{R^b}{|}}{\underset{(CH_2)_q}{\underset{|}{CH}}}-(CH_2)_p-\underset{O}{\overset{}{\underset{\|}{C}}}- \quad (Ia)$$

und p O bedeutet, eine Verbindung der Formel

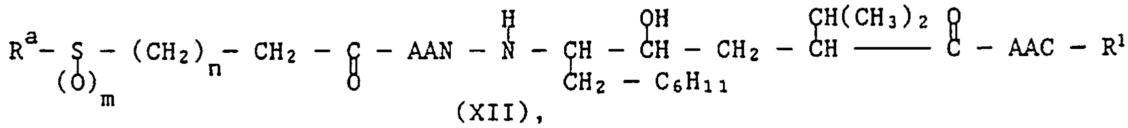

(XII),

worin die Symbole die genannten Bedeutungen haben und funktionelle Gruppen gegebenenfalls in geschützter Form vorliegen, mit einer den Rest $R^b$-$(CH_2)q$- einführenden Verbindung alkyliert, und gewünschtenfalls

i) in einer erhältlichen Verbindung vorhandene Schutzgruppen abspaltet und/oder gewünschtenfalls nach Ausführung eines der vorstehend genannten Verfahren a) - h) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

Patentansprüche für folgende Vertragsstaaten: ES, GR

1. Verfahren zur Herstellung von Verbindungen der Formel

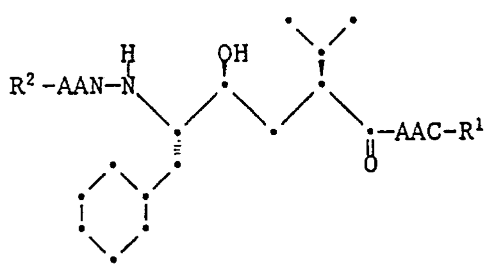

(I),

worin AAN ein bivalentes Radikal bestehend aus einem bis fünf bivalenten α-Aminosäureresten bedeutet, welches N-terminal mit dem Rest $R^2$ und C-terminal mit der Gruppe NH- verbunden ist, AAC ein bivalentes Radikal bestehend aus einem oder zwei bivalenten α-Aminosäureresten bedeutet, welches N-terminal mit der Gruppe -C=O und C-terminal mit dem Rest $R^1$ verbunden ist, $R^1$ für Hydroxy, verethertes Hydroxy, Amino oder substituiertes Amino mit Ausnahme eines von einer α-Aminosäure abgeleiteten Aminorestes steht, und $R^2$ Wasserstoff oder einen Acylrest mit Ausnahme eines gegebenenfalls N-substituierten Acylrestes einer natürlichen Aminosäure bedeutet, ferner von Salzen von solchen Verbindungen mit salzbildenden Gruppen, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene funktionelle Gruppen mit Ausnahme der an der Reaktion teilnehmenden Gruppen gegebenenfalls in geschützter Form vorliegen, unter Bildung einer Amidbindung kondensiert, oder

b) die Ketogruppe in einer Verbindung der Formel

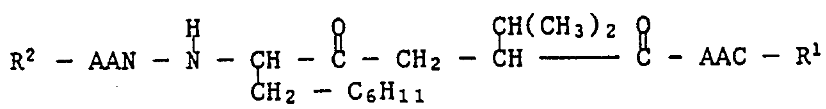

(V),

worin die Symbole die genannten Bedeutungen haben und funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) eine Aldehyd-Verbindung der Formel

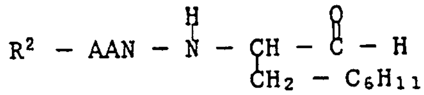

(VI),

worin die Symbole die genannten Bedeutungen haben und funktionelle Gruppen mit Ausnahme der Aldehydgruppe gegebenenfalls in geschützter Form vorliegen, mit einer Organometallverbindung der Formel

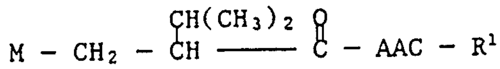

(VII),

worin die Symbole die genannten Bedeutungen haben und M ein Metallradikal bedeutet, umsetzt und das gebildete Additionsprodukt hydrolysiert, oder

d) in einer Verbindung der Formel

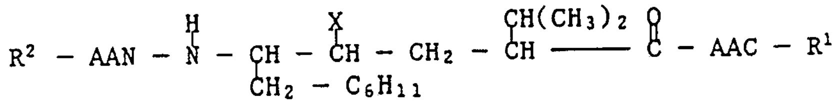

(VIII),

worin X eine nukleofuge Abgangsgruppe ist, die übrigen Symbole die oben genannten Bedeutungen haben und funktionelle Gruppen gegebenenfalls in geschützter Form vorliegen, den Substituenten X durch eine Hydroxygruppe austauscht, oder

e) in einer Verbindung der Formel

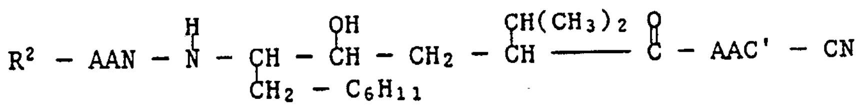

(IX),

worin die Symbole die genannten Bedeutungen haben und AAC′ die Bedeutung von AAC abzüglich der terminalen Carbonylgruppe hat und vorhandene funktionelle Gruppen gegebenenfalls in geschützter Form vorliegen, Gruppe AAC′-CN in eine Gruppe AAC-$R^1$ überführt, oder

f) in einer Verbindung der Formel

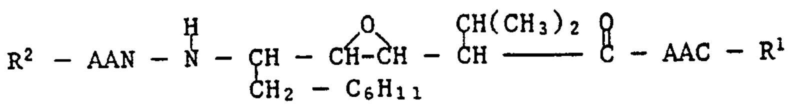

(X),

worin die Symbole die genannten Bedeutungen haben und funktionelle Gruppen gegebenenfalls in geschützter Form vorliegen, mit einem regioselektiven Reduktionsmittel die Epoxygruppe zu einer Hydroxygruppe reduziert, oder

g) zur Herstellung einer Verbindung der Formel I, worin $R^2$ einen Rest der Formel

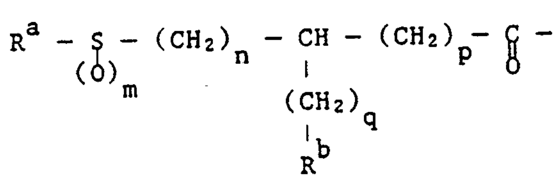

(Ia)

und m 0 oder 2, n 1 und p 0 bedeuten, eine Verbindung der Formel $R^a$-$S(O)_m$H oder ein Salz davon an eine Verbindung der Formel

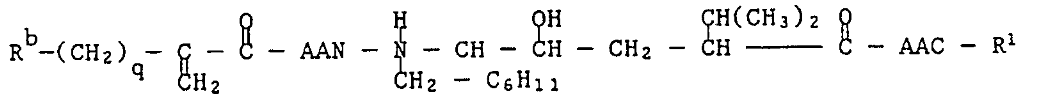 (XI),

worin die Symbole die genannten Bedeutungen haben und funktionelle Gruppen gegebenenfalls in geschützter Form vorliegen, addiert, oder

h) zur Herstellung einer Verbindung der Formel I, worin $R^2$ einen Rest der Formel

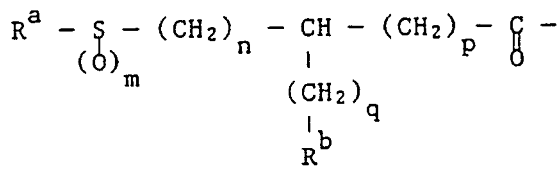 (Ia)

und p O bedeutet, eine Verbindung der Formel

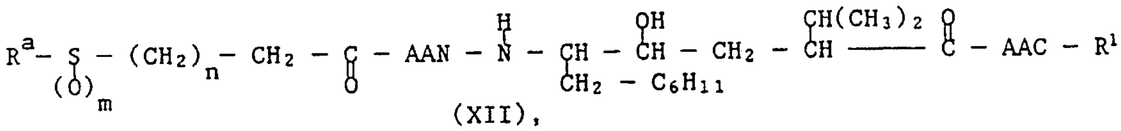

(XII),

worin die Symbole die genannten Bedeutungen haben und funktionelle Gruppen gegebenenfalls in geschützter Form vorliegen, mit einer den Rest $R^b$-$(CH_2)$q- einführenden Verbindung alkyliert, und gewünschtenfalls

i) in einer erhältlichen Verbindung vorhandene Schutzgruppen abspaltet und/oder gewünschtenfalls nach Ausführung eines der vorstehend genannten Verfahren a) - h) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

2. Verfahren gemäss Anspruch 1 zur Herstellung von Verbindungen der Formel I, die im Radikal AAN zwei oder mehr α-Aminosäurereste aufweisen oder worin das Radikal AAN aus nur einem α-Aminsäurerest besteht und gleichzeitig der Rest $R^2$ ein Analogen zu Phenylalanyl (H-Phe) ist und/oder die im Radikal AAC zwei α-Aminsosäurereste aufweisen oder worin das Radikal AAC aus nur einem α-Aminosäurerest besteht und gleichzeitig der Rest $R^1$ ein Analogon zum über Stickstoff gebundenen Rest der Aminosäure Tyrosin (-Tyr-OH) ist, sowie von Salzen davon.

3. Verfahren gemäss Anspruch 1 zur Herstellung von Verbindungen der Formel

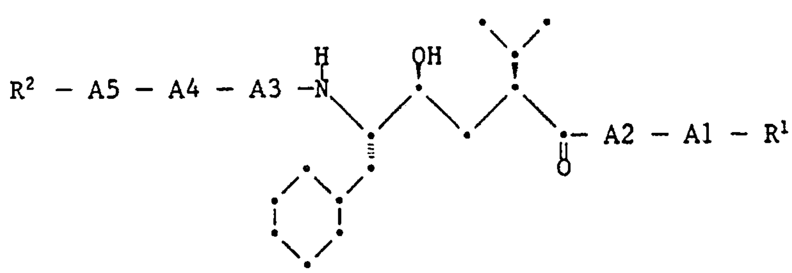 (II),

worin die Radikale A1, A2, A3 und A4 je einen bivalenten α-Aminosäurerest bedeuten, der N-terminal mit dem in Formel II jeweils links davon stehenden Radikal und C-terminal mit dem jeweils rechts davon stehenden Radikal bzw. mit dem Rest $R^1$ verbunden ist, A5 für eine einfache Bindung steht oder einen bivalenten Rest bestehend aus bis zu drei peptidisch verknüpften α-Aminosäuren bedeutet, welcher N-terminal mit $R^2$ und C-terminal mit A4 verbunden ist, und $R^1$ und $R^2$ die in Anspruch 1 für Formel I

angegebenen Bedeutungen aufweisen, sowie von Salzen davon.

4. Verfahren gemäss Anspruch 3 zur Herstellung von Verbindungen der Formel II, worin A1 ausgewählt ist unter den bivalenten Resten von Tyrosin, Phenylalanin, Naphthylalanin, Tryptophan, Lysin und Asparaginsäure, A2 ausgewählt ist unter den bivalenten Resten von Valin, Isoleucin, Leucin, Norleucin, Phenylalanin, Alanin und Glycin, A3 ausgewählt ist unter den bivalenten Resten von Valin, Alanin, Leucin, Isoleucin, Asparagin, Glutamin, Norleucin, Phenylalanin, Serin und Histidin, A4 ausgewählt ist unter den bivalenten Resten von Phenylalanin, Tyrosin, mit Niederalkyl verethertem Tyrosin, Tryptophan, Cyclohexylalanin, Leucin, Naphthylalanin, Histidin, Asparaginsäure und Lysin, und A5, $R^1$ und $R^2$ die in Anspruch 3 angegebenen Bedeutungen aufweisen, sowie von Salzen davon.

5. Verfahren gemäss Anspruch 4 zur Herstellung von Verbindungen der Formel II, worin A5 für eine einfache Bindung steht oder einen bivalenten Rest bestehend aus bis zu drei gleichen oder verschiedenen, peptidisch verknüpften α-Aminosäure ausgewählt unter den bivalenten Resten von Arginin, Prolin, Histidin, Lysin, Ornithin oder Tryptophan bedeutet, $R^1$ für Hydroxy, Niederalkoxy, Amino oder Mono- oder Diniederalkylamino steht, und $R^2$ Wasserstoff, Niederalkanoyl, Niederalkoxycarbonyl, Arylniederalkoxycarbonyl oder einen Rest der Formel

$$R^a - \underset{(O)_m}{S} - (CH_2)_n - \underset{\underset{R^b}{|}}{\underset{(CH_2)_q}{\underset{|}{CH}}} - (CH_2)_p - \underset{O}{\overset{}{\underset{\|}{C}}} - \qquad (Ia),$$

worin $R^a$ unsubstituiertes oder durch Hydroxy oder Niederalkoxy substituiertes Niederalkyl, Phenyl, Benzyl oder unsubstituiertes oder durch Oxido oder Niederalkyl substituiertes Heteroaryl mit 1 oder 2 Stickstoffatomen, $R^b$ Cyclohexyl oder Phenyl, m 0, 1 oder 2, n 1, p 0 und q 1 oder 2 darstellen, bedeutet, sowie von Salzen davon.

6. Verfahren gemäss Anspruch 5 zur Herstellung von Verbindungen der Formel II, worin A5 für eine einfache Bindung oder den bivalenten Rest -Arginin-Arginin-Prolin- steht, sowie von Salzen davon.

7. Verfahren gemäss Anspruch 3 zur Herstellung von Verbindungen der Formel II, worin A1 ausgewählt ist unter den bivalenten Resten von Tyrosin, Phenylalanin, Tryptophan, Naphthylalanin, Lysin und Asparaginsäure, A2 und A3 ausgewählt sind unter den bivalenten Resten von Valin, Isoleucin, Leucin, Norleucin und Alanin, A4 den bivalenten Rest von Phenylalanin, Tyrosin oder Naphthylalanin bedeutet, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Niederalkoxy substituiertes Phenylniederalkoxycarbonyl, Diphenylniederalkoxycarbonyl oder Fluorenylniederalkoxycarbonyl bedeutet, sowie von Salzen davon.

8. Verfahren gemäss Anspruch 3 zur Herstellung von Verbindungen der Formel II, worin A1 den bivalenten Rest von Tyrosin bedeutet, A2 ausgewählt ist unter den bivalenten Resten von Valin, Isoleucin, Norleucin, Leucin, Phenylalanin, Alanin und Glycin, A3 den bivalenten Rest von Valin bedeutet, A4 den bivalenten Rest von Phenylalanin, Tyrosin oder Naphthylalanin bedeutet, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Niederalkoxy substituiertes Phenylniederalkoxycarbonyl, Diphenylniederalkoxycarbonyl oder Fluorenylniederalkoxycarbonyl bedeutet, sowie von Salzen davon.

9. Verfahren gemäss Anspruch 3 zur Herstellung von Verbindungen der Formel II, worin A1 den bivalenten Rest von Tyrosin bedeutet, A2 für den bivalenten Rest von Valin oder Leucin steht, A3 ausgewählt ist unter den bivalenten Resten von Valin, Leucin, Isoleucin, Norleucin, Alanin, Asparagin, Glutamin, Phenylalanin, Serin und Histidin, A4 den bivalenten Rest von Phenylalanin bedeutet, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Nieder alkoxy substituiertes Phenylniederalkoxycarbonyl, Diphenylniederalkoxycarbonyl oder Fluorenylniederalkoxycarbonyl bedeutet, sowie von Salzen davon.

10. Verfahren gemäss Anspruch 3 zur Herstellung von Verbindungen der Formel II, worin A1 den bivalenten Rest von Tyrosin bedeutet, A2 für den bivalenten Rest von Valin oder Leucin steht, A3 für den bivalenten Rest von Valin steht, A4 ausgewählt ist unter den bivalenten Resten von Phenylalanin, Tyrosin, mit Methyl verethertem Tyrosin, Tryptophan, Cyclohexylalanin, Naphthylalanin und Lysin, A5 eine Bindung bedeutet, $R^1$ für Hydroxy, Niederalkoxy, Niederalkylamino oder Amino steht, und $R^2$ Wasserstoff, Niederalkanoyl, Niederalkoxycarbonyl, unsubstituiertes oder durch Niederalkoxy substituiertes Phenylniederalkoxycarbonyl, Diphenylniederalkoxycarbonyl oder Fluorenylniederalkoxycarbonyl bedeutet, sowie von Salzen davon.

11. Verfahren gemäss Anspruch 1 zur Herstellung von Verbindungen der Formel

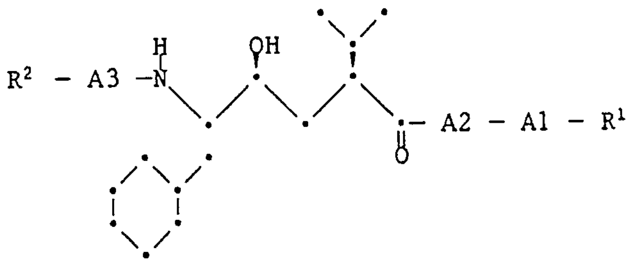

(III),

worin die Radikale A1, A2 und A3 je einen bivalenten α-Aminosäurerest bedeuten, der N-terminal mit dem in Formel III jeweils links davon stehenden Radikal bzw. mit dem Rest $R^2$ und C-terminal mit dem jeweils rechts davon stehenden Radikal bzw. mit dem Rest $R^1$ verbunden ist, und $R^1$ und $R^2$ die in Anspruch 1 für Formel I angegebenen Bedeutungen aufweisen, sowie von Salzen davon.

12. Verfahren gemäss Anspruch 11 zur Herstellung von Verbindungen der Formel III, worin A1 ausgewählt ist unter den bivalenten Resten von Tyrosin, Phenylalanin, Tryptophan, α-Naphthylalanin, Lysin und Asparaginsäure, A2 ausgewählt ist unter den bivalenten Resten von Valin, Isoleucin, Leucin, Norleucin, Phenylalanin, Alanin und Glycin, A3 ausgewählt ist unter den bivalenten Resten von Valin, Alanin, Leucin, Isoleucin, Asparagin, Glutamin, Norleucin, Phenylalanin, Serin und Histidin, und $R^1$ und $R^2$ die in Anspruch 11 angegebenen Bedeutungen aufweisen, sowie von Salzen davon.

13. Verfahren gemäss Anspruch 11 zur Herstellung von Verbindungen der Formel III worin A1 ausgewählt ist unter den bivalenten Resten von Tyrosin, Phenylalanin, Tryptophan, α-Naphthylalanin, Lysin und Asparaginsäure, A2 ausgewählt ist unter den bivalenten Resten von Valin, Isoleucin, Leucin, Norleucin, Phenylalanin, Alanin und Glycin, A3 der bivalente Rest von Tyrosin oder von mit Niederalkyl verethertem Tyrosin ist, und $R^1$ und $R^2$ die in Anspruch 11 angegebenen Bedeutungen aufweisen, sowie von Salzen davon.

14. Verfahren gemäss Anspruch 12 oder 13 zur Herstellung von Verbindungen der Formel III, worin $R^1$ für Hydroxy, Niederalkoxy, Amino oder Mono- oder Diniederalkylamino steht, und $R^2$ einen Rest der Formel

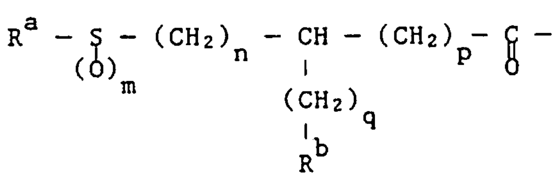

(Ia)

bedeutet, worin $R^a$ unsubstituiertes oder durch Hydroxy oder Niederalkoxy substituiertes Niederalkyl, Phenyl, Benzyl oder unsubstituiertes oder durch Oxido oder Niederalkyl substituiertes Heteroaryl mit 1 oder 2 Stickstoffatomen, $R^b$ Cyclohexyl oder Phenyl, m 0, 1 oder 2, n 1, p 0 und q 1 oder 2 darstellen, sowie von Salzen davon.

15. Verfahren gemäss Anspruch 12 oder 13 zur Herstellung von Verbindungen der Formel III, worin $R^1$ für Hydroxy, Niederalkoxy, Amino oder Mono- oder Diniederalkylamino steht, und $R_2$ einen Rest mit dem Strukturelement der Formel

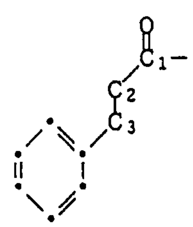

(Ib)

darstellt, worin der carbocyclische Ring auch ganz oder teilweise gesättigt sein kann, eines der Kohlenstoffatome des carbocyclischen Ringes mit $C_2$ oder $C_3$ zu einem fünf- oder sechsgliedrigen Ring verknüpft sein kann, eines der Kohlenstoffatome $C_2$ oder $C_3$ durch ein Heteroatom NH, O oder S ersetzt sein kann, die Carbonylgruppe eine Heterocarbonylgruppe P=O sein kann und freie Valenzen Wasserstoff oder einen Substituenten Niederalkyl, Niederalkylthioniederalkyl, Niederalkylsulfinylniederalkyl, Niederalkylsulfonylniederalkyl, Phenylniederalkyl, Hydroxy, Niederalkoxy, Phenylniederalkoxy, Aminoniederalkyl, Niederalkylaminoniederalkyl, Niederalkanoylaminoniederalkyl, Niederalkanoyl, Niederalkanoylniederalkyl oder Niederalkylcarbamoyl tragen, darstellt, sowie von Salzen davon.

16. Verfahren gemäss Anspruch 14 zur Herstellung von Verbindungen der Formel III, worin A1 ausgewählt ist unter den bivalenten Resten von Tyrosin, Phenylalanin, Tryptophan, Naphthylalanin, Lysin und Asparaginsäure, A2 und A3 den bivalenten Rest von Valin oder Leucin bedeuten, $R^1$ für Hydroxy, Niederalkoxy, Niederalkylamino oder Amino steht, und $R^2$ einen Rest der Formel Ia bedeutet, worin $R^a$ für Niederalkyl steht, $R^b$ Phenyl bedeutet und m zwei, n eins, p null und q eins bedeuten, sowie von Salzen davon.

17. Verfahren gemäss Anspruch 14 zur Herstellung von Verbindungen der Formel III, worin A1 den bivalenten Rest von Tyrosin bedeutet, A2 ausgewählt ist unter den bivalenten Resten von Valin, Isoleucin, Leucin, Norleucin, Phenylalanin, Alanin und Glycin, A3 den bivalenten Rest von Valin oder Leucin bedeutet, $R^1$ für Hydroxy, Niederalkoxy, Niederalkylamino oder Amino steht, und $R^2$ einen Rest der Formel Ia bedeutet, worin $R^a$ für Niederalkyl steht, $R^b$ Phenyl bedeutet und m zwei, n eins, p null und q eins bedeuten, sowie von Salzen davon.

18. Verfahren gemäss Anspruch 14 zur Herstellung von Verbindungen der Formel III, worin A1 den bivalenten Rest von Tyrosin bedeutet, A2 für den bivalenten Rest von Valin oder Leucin steht, A3 ausgewählt ist unter den bivalenten Resten von Valin, Leucin, Isoleucin, Asparagin, Glutamin, Norleucin, Phenylalanin, Serin und Histidin, $R^1$ für Hydroxy, Niederalkoxy, Niederalkylamino oder Amino steht, und $R^2$ einen Rest der Formel Ia bedeutet, worin $R^a$ für Niederalkyl steht, $R^b$ Phenyl bedeutet und m zwei, n eins, p null und q eins bedeuten, sowie von Salzen davon.

19. Verfahren gemäss Anspruch 15 zur Herstellung von Verbindungen der Formel III, worin A1 den bivalenten Rest von Tyrosin bedeutet, A2 den bivalenten Rest von Valin bedeutet, A3 ausgewählt ist unter den bivalenten Resten von Valin, Leucin, Norleucin, Phenylalanin, Alanin, Serin, Tyrosin und mit Niederalkyl verethertem Tyrosin, $R^1$ für Hydroxy, Niederalkoxy, Niederalkylamino oder Amino steht, und $R^2$ 3-Phenyl- oder 3-Cyclohexyl-propionyl bedeutet, das in 2-Stellung durch Niederalkylsulfonylniederalkyl oder durch Niederalkanoylniederalkyl substituiert ist, sowie von Salzen davon.

20. Verfahren gemäss Anspruch 1 zur Herstellung von Verbindungen der Formel IV,

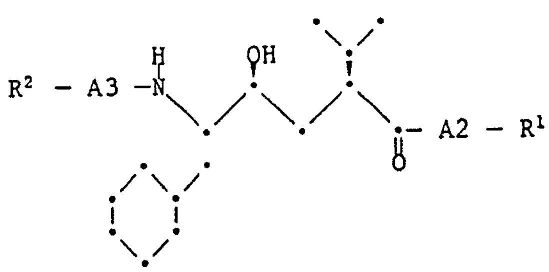

(IV)

worin A3 einen bivalenten α-Aminosäurerest bedeutet, der N-terminal mit dem Rest $R^2$ und C-terminal mit der Gruppe NH- verbunden ist, A2 einen bivalenten α-Aminosäurerest bedeutet, der N-terminal mit der Gruppe -C=O und C-terminal mit dem Rest $R^1$ verbunden ist, $R^1$ Niederalkylamino oder einen Rest mit dem Strukturelement der Formel

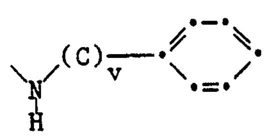

(Ic)

bedeutet, worin der carbocyclische Ring ein Stickstoffatom enthalten kann und auch ganz oder teilweise gesättigt und/oder durch Hydroxy oder durch Phenyl substituiert sein kann, der Index v für null bis fünf steht, eines der Kohlenstoffatome des carbocyclischen Ringes mit einem der Atome $-(C)_v-$ zu einem fünf- oder sechsgliedrigen Ring verbunden sein kann und freie Valenzen Wasserstoff oder einen Substituenten

Niederalkyl oder Hydroxyniederalkyl tragen, und $R^2$ einen Rest mit dem Strukturelement der Formel

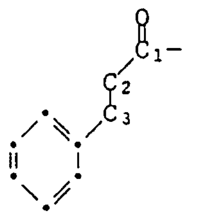

(Ib)

darstellt, worin der carbocyclische Ring auch ganz oder teilweise gesättigt sein kann, eines der Kohlenstoffatome des carbocyclischen Ringes mit $C_2$ oder $C_3$ zu einem fünf- oder sechsgliedrigen Ring verknüpft sein kann, eines der Kohlenstoffatome $C_2$ oder $C_3$ durch ein Heteroatom NH, O oder S ersetzt sein kann, die Carbonylgruppe eine Heterocarbonylgruppe P=O sein kann und freie Valenzen Wasserstoff oder einen Substituenten Niederalkyl, Niederalkylthioniederalkyl, Niederalkylsulfinylniederalkyl, Niederalkylsulfonylniederalkyl, Phenylniederalkyl, Hydroxy, Niederalkoxy, Phenylniederalkoxy, Aminoniederalkyl, Niederalkylaminoniederalkyl, Niederalkanoylaminoniederalkyl, Niederalkanoyl, Niederalkanoylniederalkyl oder Niederalkylcarbamoyl tragen, sowie von Salzen davon.

21. Verfahren gemäss Anspruch 20 zur Herstellung von Verbindungen der Formel IV, worin A2 ausgewählt ist unter den bivalenten Resten von Valin, Isoleucin, Norleucin, Leucin, Phenylalanin, Alanin und Glycin und A3 ausgewählt ist unter den bivalenten Resten von Valin, Alanin, Leucin, Asparagin, Glutamin, Norleucin, Phenylalanin, Serin und Histidin, sowie von Salzen davon.

22. Verfahren gemäss Anspruch 20 zur Herstellung von Verbindungen der Formel IV, worin A2 und A3 unabhängig voneinander jeweils den bivalenten Rest von Valin oder Leucin bedeuten, sowie von Salzen davon.

23. Verfahren gemäss Anspruch 20 zur Herstellung von Verbindungen der Formel IV, worin A2 den bivalenten Rest von Valin bedeutet, A3 ausgewählt ist unter den bivalenten Resten von Valin, Leucin, Norleucin, Phenylalanin, Alanin, Serin, Tyrosin und mit Niederalkyl verethertem Tyrosin, $R^1$ Niederalkylamino oder ein Rest mit dem Strukturelement der Formel Ic ist, worin der carbocyclische Ring unsubstituiert oder durch Hydroxy oder durch Phenyl substituiert sein kann, der Index v für null bis fünf steht und freie Valenzen Wasserstoff tragen, und $R^2$ 3-Phenyl- oder 3-Cyclohexyl-propionyl bedeutet, dass in 2-Stellung durch Niederalkylsulfonylniederalkyl oder durch Niederalkanoylniederalkyl substituiert ist, sowie von Salzen davon.

24. Verfahren gemäss Anspruch 11 zur Herstellung der Verbindung der Formel III, worin A1 den bivalenten Rest von Tyrosin, A2 und A3 jeweils den bivalenten Rest von Valin, $R^1$ Methoxy und $R^2$ 2(S)-Benzyl-3-tert-butylsulfonylpropionyl bedeuten.

25. Verfahren zur Herstellung von pharmazeutischen Präparaten enthaltend Verbindungen der Formel I gemäss Anspruch 1 oder ein pharmazeutisch annehmbares Salz davon zusammen mit einem pharmazeutisch geeigneten Trägermaterial.